(19) Europäisches Patentamt
European Patent Office
Office européen des brevets

(11) EP 2 928 965 B1

(12) **EUROPEAN PATENT SPECIFICATION**

(45) Date of publication and mention of the grant of the patent:
**04.07.2018 Bulletin 2018/27**

(21) Application number: **13812349.2**

(22) Date of filing: **06.12.2013**

(51) Int Cl.:
***C09B 23/00*** *(2006.01)* ***C12Q 1/68*** *(2018.01)*
***G01N 33/52*** *(2006.01)* ***G01N 33/58*** *(2006.01)*
***G01N 33/68*** *(2006.01)*

(86) International application number:
**PCT/US2013/073548**

(87) International publication number:
**WO 2014/089421 (12.06.2014 Gazette 2014/24)**

# (54) DETECTION OF RNA MOLECULES USING SUBSTITUTED UNSYMMETRICAL CYANINE DYES

NACHWEIS VON RNA-MOLEKÜLEN MITTELS SUBSTITUIERTER UNSYMMETRISCHER CYANINFARBSTOFFE

DÉTECTION DE MOLÉCULES D'ARN AU MOYEN DE COLORANTS DE CYANINE SUBSTITUÉS ET NON SYMÉTRIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.12.2012 US 201261734862 P**
**30.08.2013 US 201361872276 P**

(43) Date of publication of application:
**14.10.2015 Bulletin 2015/42**

(73) Proprietor: **Life Technologies Corporation**
**Carlsbad, CA 92008 (US)**

(72) Inventors:
- **DALLWIG, Jason**
**Eugene, OR 97405 (US)**
- **GEE, Kyle**
**Carlsbad, California 92008 (US)**
- **AHNERT, Nancy**
**Eugene, OR 97405 (US)**
- **KANG, Hee Chol**
**Eugene, OR 97405 (US)**
- **YANG, Jongtae**
**Carlsbad, California 92008 (US)**

(74) Representative: **Hoffmann Eitle**
**Patent- und Rechtsanwälte PartmbB**
**Arabellastraße 30**
**81925 München (DE)**

(56) References cited:
**WO-A1-00/66664** **WO-A1-02/26891**
**WO-A1-2005/033342** **WO-A1-2012/061403**
**WO-A2-96/13552** **WO-A2-2005/056689**
**US-A1- 2006 003 337**

- **SHOHEI YAMAMURA ET AL: "Detection of miRNA in Cell Cultures by Using Microchip Electrophoresis with a Fluorescence-Labeled Riboprobe", SENSORS, vol. 12, no. 12, 7 June 2012 (2012-06-07), pages 7576-7586, XP055098936, ISSN: 1424-8220, DOI: 10.3390/s120607576**
- **MATVEEVA E G ET AL: "Ratiometric FRET-based detection of DNA and micro-RNA in solution", JOURNAL OF LUMINESCENCE, ELSEVIER BV NORTH-HOLLAND, NL, vol. 129, no. 11, 1 November 2009 (2009-11-01), pages 1281-1285, XP026471685, ISSN: 0022-2313, DOI: 10.1016/J.JLUMIN.2009.06.009 [retrieved on 2009-06-21]**
- **Invitrogen: "Quibit 2.0 Fluorometer", , 1 January 2011 (2011-01-01), XP055098924, Retrieved from the Internet: URL:http://www.lifetechnologies.com/content/dam/LifeTech/migration/en/filelibrary/cell-tissue-analysis/qubit-all-file-types.par.19803.file.dat/co16190 qubit brochure.pdf [retrieved on 2014-01-28]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 928 965 B1

## Description

### TECHNICAL FIELD

Substituted unsymmetrical cyanine dyes are provided that are useful in the detection of RNA molecules (*e.g.,* microRNA); also provided are compositions, kits and assay systems that include such dyes, as well as methods for their use.

### BACKGROUND

MicroRNAs (miRNAs) are small, highly conserved RNA molecules that, like small interfering RNAs (siRNAs), modulate the expression of their cognate mRNA molecules by base-pairing with them and preventing expression through a variety of mechanisms. miRNAs have been shown to act as key regulators of development, cell proliferation, differentiation, and the cell cycle.

Although various nucleic acid binding dyes are known, (WO 00/66664, WO 96/13552, WO 2005/033342 and WO 2005/056689) there remains a need to develop materials and methods for detecting nucleic acid molecules, in particular, small single-stranded RNA molecules, such as microRNA.

### SUMMARY

Provided herein are substituted unsymmetrical cyanine dyes, kits including such dyes, as well as methods using such dyes for detecting RNA molecules. The invention is illustrated by the appended claims. The disclosed dyes and compositions can be used advantageously for the detection of short and/or single-stranded RNA molecules. It was surprisingly found that the dye compounds and the methods disclosed herein can be used to detect short, single-stranded RNA molecules that are usually not detectable by standard fluorescent methods. Furthermore, the methods provided herein can detect very low concentrations of short RNA molecules. It was also surprisingly found that the compositions and methods provided herein are more specific for short RNA molecules than for larger RNA molecules. The compositions and methods provided herein overcome many of the disadvantages associated with conventional double-stranded nucleic acid binding dyes and provide new opportunities for the use in the analysis and quantification of RNA.

In one aspect, a method of detecting the presence of single-stranded RNA (ssRNA) in a sample is provided, the method includes:

(a) combining a sample suspected of containing ssRNA, wherein the ssRNA comprises 25 or fewer nucleotides, with a substituted, unsymmetrical cyanine dye compound selected from the group consisting of Compound 4, Compound 5, Compound 6, Compound 7, Compound 8, Compound 9, Compound 10, Compound 11, Compound 12, Compound 13, Compound 14, Compound 15, Compound 16, Compound 17, Compound 18, Compound 19, Compound 20, Compound 21, Compound 23, Compound 25, Compound 26, Compound 27, Compound 30, Compound 31, Compound 32, Compound 33, Compound 34, Compound 35, Compound 36, Compound 37, Compound 38, Compound 39, Compound 40, Compound 41, Compound 42, Compound 43, Compound 44, Compound 45, Compound 46, Compound 47, Compound 48, Compound 49, Compound 50, Compound 51, Compound 52, Compound 53, Compound 54, Compound 55, Compound 56, Compound 57, Compound 58, Compound 59 and Compound 60;

(b) incubating the sample for a time sufficient for the dye compound to combine with the ssRNA in the sample, if present, to form a dye-ssRNA complex that emits a detectable fluorescent signal; and

(c) detecting the emission of the fluorescent signal, wherein the detectable fluorescent signal is indicative of the presence of a dye-ssRNA complex.

In certain embodiments, the ssRNA comprises 20-25 nucleotides. In certain embodiments, the ssRNA comprises 10-20 nucleotides. In certain embodiments, the ssRNA is naturally occurring. In certain embodiments, the ssRNA comprises a chemical modification to at least one sugar, phosphate or base group. In certain embodiments, the ssRNA comprises a chemical group other than a hydroxyl or phosphate at its 5' termini. In certain embodiments, the ssRNA is microRNA (miRNA), small nuclear RNA (snRNA), small nucleolar RNA (snoRNA), tiny non-coding RNA (tncRNA), small modulatory RNA (smRNA), or piwi-interacting RNA (piRNA). In certain embodiments, the ssRNA is miRNA.

The methods provided herein can detect as little as 0.5 ng ssRNA in a sample even in the presence of larger RNA, such as mRNA or rRNA. In certain embodiments, the method further comprises detecting ssRNA in a concentration of 0.5 ng/mL or greater. In certain embodiments, the method further comprises detecting ssRNA in a concentration of 5 ng/mL or greater. In certain embodiments, the method further comprises detecting ssRNA in a concentration of 10 ng/mL or greater. In certain embodiments, the method further comprises detecting ssRNA in a concentration of 25 ng/mL or greater. In certain embodiments, the method further comprises detecting ssRNA in a concentration of about 5 ng/mL

to about 1000 ng/mL. In certain embodiments, the method further comprises detecting ssRNA in a concentration of about 25 ng/mL to about 1000 ng/mL. In certain embodiments, the method further comprises detecting ssRNA in a concentration of about 50 ng/mL to about 1000 ng/mL. In certain embodiments, the sample can include ssRNA in a concentration of about 5 ng/mL to about 500 ng/mL. In certain embodiments, the sample can include ssRNA in a concentration of about 25 ng/mL to about 500 ng/mL. In certain embodiments, the sample can include ssRNA in a concentration of about 50 ng/mL to about 100 ng/mL.

In certain embodiments, the fluorescence signal is stable for at least 30 minutes at room temperature. In certain embodiments, the fluorescence signal is stable for at least 3 hours at room temperature.

In yet another aspect, a method of detecting single-stranded RNA (ssRNA) in a sample is provided, the method includes:

(a) combining a sample suspected of containing ssRNA, wherein the ssRNA comprises 25 or fewer nucleotides, with a substituted, unsymmetrical cyanine dye compound of the Formula **II,**

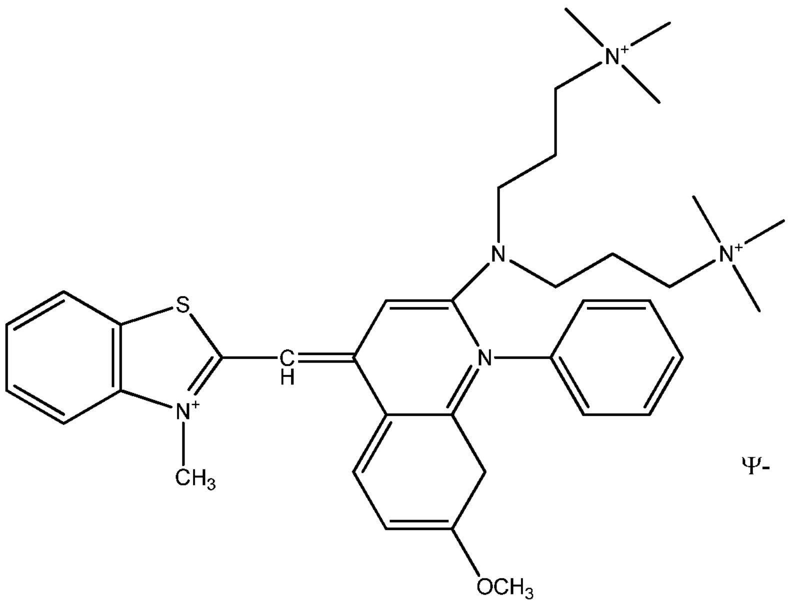

$\Psi^-$

(**II**);

(b) incubating the sample for a time sufficient for the dye compound to combine with the ssRNA in the sample, if present, to form a dye-ssRNA complex that emits a detectable fluorescent signal; and

(c) detecting the emission of the fluorescent signal, wherein the detectable fluorescent signal is indicative of the presence of a dye-ssRNA complex.

Examples of $\Psi^-$ include, among others, chloride, bromide, iodide, sulfate, alkanesulfonate, arylsulfonate, phosphate, perchlorate, tetrafluoroboronate, tetraarylboride, nitrate, and anions of aromatic or aliphatic carboxylic acids. Preferred $\psi$-counterions are bromide, chloride, iodide, perchlorate, and various sulfonates.

In certain embodiments, the ssRNA comprises 20-25 nucleotides. In certain embodiments, the ssRNA comprises 10-20 nucleotides. In certain embodiments, the ssRNA is naturally occurring. In certain embodiments, the ssRNA comprises a chemical modification to at least one sugar, phosphate or base group. In certain embodiments, the ssRNA comprises a chemical group other than a hydroxyl or phosphate at its 5' termini. In certain embodiments, the ssRNA is microRNA (miRNA), small nuclear RNA (snRNA), small nucleolar RNA (snoRNA), tiny non-coding RNA (tncRNA), small modulatory RNA (smRNA), or piwi-interacting RNA (piRNA). In certain embodiments, the ssRNA is miRNA.

The methods provided herein can detect as little as 0.5 ng ssRNA in a sample even in the presence of larger RNA, such as mRNA or rRNA. In certain embodiments, the method further comprises detecting ssRNA in a concentration of 0.5 ng/mL or greater. In certain embodiments, the method further comprises detecting ssRNA in a concentration of 5 ng/mL or greater. In certain embodiments, the method further comprises detecting ssRNA in a concentration of 10 ng/mL or greater. In certain embodiments, the method further comprises detecting ssRNA in a concentration of 25 ng/mL or greater. In certain embodiments, the method further comprises detecting ssRNA in a concentration of about 5 ng/mL to about 1000 ng/mL. In certain embodiments, the method further comprises detecting ssRNA in a concentration of about 25 ng/mL to about 1000 ng/mL. In certain embodiments, the method further comprises detecting ssRNA in a concentration of about 50 ng/mL to about 1000 ng/mL. In certain embodiments, the sample can include ssRNA in a

concentration of about 5 ng/mL to about 500 ng/mL. In certain embodiments, the sample can include ssRNA in a concentration of about 25 ng/mL to about 500 ng/mL. In certain embodiments, the sample can include ssRNA in a concentration of about 50 ng/mL to about 100 ng/mL.

In certain embodiments, the fluorescence signal is stable for at least 30 minutes at room temperature. In certain embodiments, the fluorescence signal is stable for at least 3 hours at room temperature.

The methods provided herein can be used to detect any type of short RNA molecule. The short RNA molecule can be a single-stranded RNA (ssRNA) molecule. The ssRNA can be naturally occurring or can include a chemical modification to at least one sugar, phosphate or base group. For example, the ssRNA can include a chemical group other than a hydroxy or phosphate at its 5' terminus. In certain embodiments, the ssRNA molecule can include 20-25 nucleotides. In other embodiments, the ssRNA molecule can include 10-20 nucleotides. In certain methods, the ssRNA is microRNA (miRNA). The ssRNA can be a small nuclear RNA (snRNA), small nucleolar RNA (snoRNA), tiny non-coding RNA (tncRNA), small modulatory RNA (smRNA) or piwi-interacting RNA (piRNA).

The methods provided herein can be used to detect extremely small quantities of RNA (*e.g.,* ssRNA) in a sample. For example, the methods provided herein can detect as little as 0.5 ng ssRNA in a sample even in the presence of larger RNA such as rRNA or mRNA. In certain embodiments, the method further comprises detecting ssRNA in a concentration of 0.5 ng/mL or greater. In certain embodiments, the method further comprises detecting ssRNA in a concentration of 5 ng/mL or greater. In certain embodiments, the method further comprises detecting ssRNA in a concentration of 10 ng/mL or greater. In certain embodiments, the method further comprises detecting ssRNA in a concentration of 25 ng/mL or greater. In certain embodiments, the method further comprises detecting ssRNA in a concentration of about 5 ng/mL to about 1000 ng/mL. In certain embodiments, the method further comprises detecting ssRNA in a concentration of about 25 ng/mL to about 1000 ng/mL. In certain embodiments, the method further comprises detecting ssRNA in a concentration of about 50 ng/mL to about 1000 ng/mL. In certain embodiments, the sample can include ssRNA in a concentration of about 5 ng/mL to about 500 ng/mL. In certain embodiments, the sample can include ssRNA in a concentration of about 25 ng/mL to about 500 ng/mL. In certain embodiments, the sample can include ssRNA in a concentration of about 50 ng/mL to about 100 ng/mL.

In certain embodiments, the fluorescence signal is stable for at least 30 minutes at room temperature. In certain embodiments, the fluorescence signal is stable for at least 3 hours at room temperature.

A fluorescent complex may be provided that includes ssRNA, wherein the ssRNA comprises 25 or fewer nucleotides, non-covalently bound to a dye of the Formula **II:**

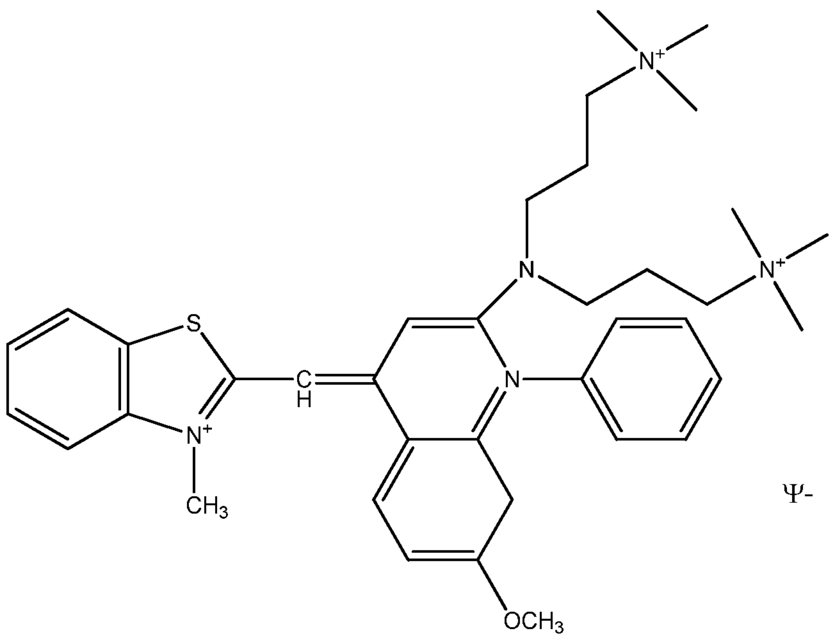

**(II)**

wherein the complex emits a detectable fluorescent signal.

Examples of $\Psi^-$ include, among others, chloride, bromide, iodide, sulfate, alkanesulfonate, arylsulfonate, phosphate, perchlorate, tetrafluoroboronate, tetraarylboride, nitrate, and anions of aromatic or aliphatic carboxylic acids. Preferred $\psi$-counterions are bromide, chloride, iodide, perchlorate, and various sulfonates.

The short RNA molecule can be a single-stranded RNA (ssRNA) molecule. The ssRNA can be naturally occurring or can include a chemical modification to at least one sugar, phosphate or base group. For example, the ssRNA can

include a chemical group other than a hydroxy or phosphate at its 5' terminus. In certain embodiments, the ssRNA molecule can include 20-25 nucleotides. In other embodiments, the ssRNA molecule can include 10-20 nucleotides. In certain methods, the ssRNA is microRNA (miRNA). The ssRNA can be a small nuclear RNA (snRNA), small nucleolar RNA (snoRNA), tiny non-coding RNA (tncRNA), small modulatory RNA (smRNA) or piwi-interacting RNA (piRNA).

A fluorescent complex may be produced that includes ssRNA, wherein the ssRNA comprises 25 or fewer nucleotides, non-covalently bound to a dye compound selected from the group consisting of Compound 4, Compound 5, Compound 6, Compound 7, Compound 8, Compound 9, Compound 10, Compound 11, Compound 12, Compound 13, Compound 14, Compound 15, Compound 16, Compound 17, Compound 18, Compound 19, Compound 20, Compound 21, Compound 23, Compound 25, Compound 26, Compound 27, Compound 30, Compound 31, Compound 32, Compound 33, Compound 34, Compound 35, Compound 36, Compound 37, Compound 38, Compound 39, Compound 40, Compound 41, Compound 42, Compound 43, Compound 44, Compound 45, Compound 46, Compound 47, Compound 48, Compound 49, Compound 50, Compound 51, Compound 52, Compound 53, Compound 54, Compound 55, Compound 56, Compound 57, Compound 58, Compound 59, and Compound 60, wherein the complex emits a detectable signal.

In certain embodiments, the short RNA molecule can be a single-stranded RNA (ssRNA) molecule. The ssRNA can be naturally occurring or can include a chemical modification to at least one sugar, phosphate or base group. For example, the ssRNA can include a chemical group other than a hydroxy or phosphate at its 5' terminus. In certain embodiments, the ssRNA molecule can include 20-25 nucleotides. In other embodiments, the ssRNA molecule can include 10-20 nucleotides. In certain methods, the ssRNA is microRNA (miRNA). The ssRNA can be a small nuclear RNA (snRNA), small nucleolar RNA (snoRNA), tiny non-coding RNA (tncRNA), small modulatory RNA (smRNA) or piwi-interacting RNA (piRNA).

The fluorescent signal generated by the fluorescent complex is extremely stable. For example, the signal can be stable for at least 30 minutes at room temperature. In some embodiments, the fluorescence signal is stable for at least 3 hours at room temperature.

In yet another aspect, a kit for detecting the presence of single-stranded RNA (ssRNA) in a sample is provided that includes:

a dye compound selected from the group consisting of Compound 4, Compound 5, Compound 6, Compound 7, Compound 8, Compound 9, Compound 10, Compound 11, Compound 12, Compound 13, Compound 14, Compound 15, Compound 16, Compound 17, Compound 18, Compound 19, Compound 20, Compound 23, Compound 25, Compound 26, Compound 27, Compound 30, Compound 31, Compound 32, Compound 33, Compound 34, Compound 35, Compound 36, Compound 37, Compound 38, Compound 39, Compound 40, Compound 43, Compound 44, Compound 45, Compound 46, Compound 47, Compound 48, Compound 49, Compound 50, Compound 51, Compound 52, Compound 53, Compound 54, Compound 55, Compound 56, Compound 57, Compound 58, Compound 59, and Compound 60; and

instructions for using the dye in a method as disclosed herein.

In yet another aspect, dye compounds are provided, the dye compound is selected from the group consisting of Compound 4, Compound 5, Compound 6, Compound 7, Compound 8, Compound 9, Compound 10, Compound 11, Compound 12, Compound 13, Compound 14, Compound 15, Compound 16. Compound 17, Compound 18, Compound 19, Compound 20, Compound 23, Compound 25, Compound 26, Compound 27, Compound 30, Compound 31, Compound 32, Compound 33, Compound 34, Compound 35, Compound 36, Compound 37, Compound 38, Compound 39, Compound 40, Compound 43, Compound 44, Compound 45, Compound 46, Compound 47, Compound 48, Compound 49, Compound 50, Compound 51, Compound 52, Compound 53, Compound 54, Compound 55, Compound 56, Compound 57, Compound 58, Compound 59, and Compound 60.

These and other features, aspects, and embodiments are described herein.

## BRIEF DESCRIPTION OF THE DRAWINGS

**FIGURE 1A** is a plot showing the line corresponding to the curve-fitting algorithm (a Modified Hill plot) used to calculate concentration in the MyQubit miRNA Assay. For reference, the positions of the standards and a set of data points from an actual experiment are shown superimposed onto the line, demonstrating that the curve-fitting algorithm gives accurate values for quantitation. **FIGURE 1B** is a computer screen shot of the QUBIT assay.

**FIGURE 2** is a screen shot of the upload of the MyQubit miRNA Assay.

**FIGURE 3** is a graphic representation of RNA assays according to certain embodiments of the methods disclosed herein wherein the selectivity for siRNA (small double stranded RNA) over rRNA (ribosomal RNA).

**FIGURE 4** is a graphic representation of RNA assays according to certain embodiments of the methods disclosed herein wherein the assay detects small ssRNA and dsRNA.

**FIGURE 5** is a graphic representation of RNA assays according to certain embodiments of the methods disclosed herein wherein the miRNA assay detects only a portion of the total RNA in the sample (extracted from U2OS cells).

**FIGURES 6A-6D** are graphic representations of RNA assays according to certain embodiments of the methods disclosed herein wherein the MyQubit miRNA Assay detected about 15% of each mRNA **(FIGURES 6A** and **6B),** and the QUBIT RNA Assay detected 90-150% of each mRNA **(FIGURES 6C** and **6D).**

**FIGURES 7A-7H** are graphic representations of RNA assays according to certain embodiments of the methods disclosed herein wherein various dye compounds of the present teachings are used.

## DETAILED DESCRIPTION

Definitions:

Unless defined otherwise, all technical and scientific terms used herein have the same meaning as is commonly understood by one of ordinary skill in the art.

As used herein, "a" or "an" means "at least one" or "one or more".

As used herein, "about" means that the numerical value is approximate and small variations would not significantly affect the use and practice of the compositions and methods provided herein. Where a numerical limitation is used, unless indicated otherwise by the context, "about" means the numerical value can vary by $\pm$10% and remain within the scope of the disclosed embodiments.

The term "or combinations thereof' as used herein refers to all permutations and combinations of the listed terms preceding the term. For example, "A, B, C, or combinations thereof" is intended to include at least one of: A, B, C, AB, AC, BC, or ABC, and if order is important in a particular context, also BA, CA, CB, ACB, CBA, BCA, BAC, or CAB. Continuing with this example, expressly included are combinations that contain repeats of one or more item or term, such as BB, AAA, AAB, BBC, AAABCCCC, CBBAAA, CABABB, and so forth. The skilled artisan will understand that typically there is no limit on the number of items or terms in any combination, unless otherwise apparent from the context.

Certain compounds of the present disclosure can exist in unsolvated forms as well as solvated forms, including hydrated forms. In general, the solvated forms are equivalent to unsolvated forms and are encompassed within the scope of the present disclosure. Certain compounds disclosed herein may exist in multiple crystalline or amorphous forms. In general, all physical forms are equivalent for the uses contemplated by the present disclosure and are intended to be within the scope of the present teachings.

Certain compounds disclosed herein possess asymmetric carbon atoms (optical centers) or double bonds; the racemates, diastereomers, geometric isomers and individual isomers are encompassed within the scope of the present disclosure.

The compounds described herein may be prepared as a single isomer (*e.g.,* enantiomer, *cis-trans,* positional, diastereomer) or as a mixture of isomers. In a preferred embodiment, the compounds are prepared as substantially a single isomer. Methods of preparing substantially isomerically pure compounds are known in the art. For example, enantiomerically enriched mixtures and pure enantiomeric compounds can be prepared by using synthetic intermediates that are enantiomerically pure in combination with reactions that either leave the stereochemistry at a chiral center unchanged or result in its complete inversion. Alternatively, the final product or intermediates along the synthetic route can be resolved into a single stereoisomer. Techniques for inverting or leaving unchanged a particular stereocenter, and those for resolving mixtures of stereoisomers are well known in the art and it is well within the ability of one of skill in the art to choose and appropriate method for a particular situation. *See,* generally, Furniss et al. (eds.), VOGEL'S ENCYCLOPEDIA OF PRACTICAL ORGANIC CHEMISTRY 5TH ED., Longman Scientific and Technical Ltd., Essex, 1991, pp. 809-816; and Heller, Acc. Chem. Res. 23: 128 (1990).

The compounds disclosed herein may also contain unnatural proportions of atomic isotopes at one or more of the atoms that constitute such compounds. For example, the compounds may be radiolabeled with radioactive isotopes, such as for example tritium ($^3H$), iodine-125 ($^{125}I$) or carbon-14 ($^{14}C$). All isotopic variations of the compounds disclosed herein, whether radioactive or not, are intended to be encompassed within the scope of the present disclosure.

Where a disclosed compound includes a conjugated ring system, resonance stabilization may permit a formal

electronic charge to be distributed over the entire molecule. While a particular charge may be depicted as localized on a particular ring system, or a particular heteroatom, it is commonly understood that a comparable resonance structure can be drawn in which the charge may be formally localized on an alternative portion of the compound.

Selected compounds having a formal electronic charge may be shown without an appropriate biologically compatible counterion. Such a counterion serves to balance the positive or negative charge present on the compound. As used herein, a substance that is biologically compatible is not toxic as used, and does not have a substantially deleterious effect on biomolecules. Examples of negatively charged counterions include, among others, chloride, bromide, iodide, sulfate, alkanesulfonate, arylsulfonate, phosphate, perchlorate, tetrafluoroborate, tetraarylboride, nitrate and anions of aromatic or aliphatic carboxylic acids. Preferred counterions may include chloride, iodide, perchlorate and various sulfonates. Examples of positively charged counterions include, among others, alkali metal, or alkaline earth metal ions, ammonium, or alkylammonium ions.

Where substituent groups are specified by their conventional chemical formulae, written from left to right, they equally encompass the chemically identical substituents, which would result from writing the structure from right to left, *e.g.,* $-CH_2O-$ is intended to also recite $-OCH_2-$.

A dashed line projecting from a substituent, such as:

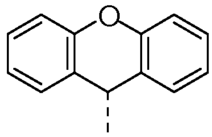

indicates the point of attachment to the base molecule. For a fused ring, dashed lines indicate portions of the base molecule where the fused ring is attached, such as:

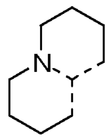

wherein the full molecule could have the structure:

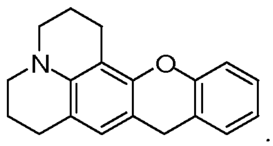

The term "alkyl," by itself or as part of another substituent means, unless otherwise stated, a straight or branched chain, or cyclic hydrocarbon radical, or combination thereof, which may be fully saturated, mono- or polyunsaturated and can include divalent ("alkylene") and multivalent radicals, having the number of carbon atoms designated (*e.g.* $C_1$-$C_{10}$ means one to ten carbons). Examples of saturated hydrocarbon radicals include, but are not limited to, groups such as methyl, ethyl, n-propyl, isopropyl, n-butyl, t-butyl, isobutyl, sec-butyl, cyclohexyl, (cyclohexyl)methyl, cyclopropylmethyl, homologs and isomers of, for example, n-pentyl, n-hexyl, n-heptyl, n-octyl, and the like. An unsaturated alkyl group is one having one or more double bonds or triple bonds. Examples of unsaturated alkyl groups include, but are not limited to, vinyl, 2-propenyl, crotyl, 2-isopentenyl, 2-(butadienyl), 2,4-pentadienyl, 3-(1,4-pentadienyl), ethynyl, 1- and 3-propynyl, 3-butynyl, and the higher homologs and isomers. The term "alkyl," unless otherwise noted, is also meant to include those derivatives of alkyl defined in more detail below, such as "heteroalkyl." Alkyl groups that are limited to hydrocarbon groups are termed "homoalkyl".

Exemplary alkyl groups of use in the present disclosure contain between about one and about twenty five carbon atoms (*e.g.* methyl, ethyl and the like). Straight, branched or cyclic hydrocarbon chains having eight or fewer carbon atoms will also be referred to herein as "lower alkyl". In addition, the term "alkyl" as used herein further includes one or more substitutions at one or more carbon atoms of the hydrocarbon chain fragment.

As used herein, "aryl" refers to an aromatic moiety having a single ring or multiple condensed rings each of which is optionally and independently substituted with H, halogen, cyano, azido, sulfonic acid, alkali or ammonium salt of sulfonic acid, carboxylic acid, biologically compatible salt of carboxylic acid, nitro, alkyl, perfluoroalkyl, alkoxy, alkylthio, amino, monoalkylamino, dialkylamino or alkylamido.

The terms "alkoxy," "alkylamino" and "alkylthio" (or thioalkoxy) are used in their conventional sense, and refer to those alkyl groups attached to the remainder of the molecule via an oxygen atom, an amino group, or a sulfur atom, respectively.

The term "heteroalkyl," by itself or in combination with another term, means, unless otherwise stated, a straight or branched chain, or cyclic carbon-containing radical, or combinations thereof, consisting of the stated number of carbon atoms and at least one heteroatom selected from the group consisting of O, N, Si, P, S, and Se and wherein the nitrogen, phosphorous, sulfur, and selenium atoms are optionally oxidized, and the nitrogen heteroatom is optionally be quaternized. The heteroatom(s) O, N, P, S, Si, and Se may be placed at any interior position of the heteroalkyl group or at the position at which the alkyl group is attached to the remainder of the molecule. Examples include, but are not limited to, $-CH_2-CH_2-O-CH_3$, $-CH_2-CH_2-NH-CH_3$, $-CH_2-CH_2-N(CH_3)-CH_3$, $-CH_2-S-CH_2-CH_3$, $-CH_2-CH_2$, $-S(O)-CH_3$, $-CH_2-CH_2-S(O)_2-CH_3$, $-CH=CH-O-CH_3$, $-Si(CH_3)_3$, $-CH_2-CH=N-OCH_3$, and $-CH=CH-N(CH_3)-CH_3$. Up to two heteroatoms may be consecutive, such as, for example, $-CH_2-NH-OCH_3$ and $-CH_2-O-Si(CH_3)_3$. Similarly, the term "heteroalkylene" by itself or as part of another substituent means a divalent radical derived from heteroalkyl, as exemplified, but not limited by, $-CH_2-CH_2-S-CH_2-CH_2-$ and $-CH_2-S-CH_2-CH_2-NH-CH_2-$. For heteroalkylene groups, heteroatoms can also occupy either or both of the chain termini (*e.g.,* alkyleneoxy, alkylenedioxy, alkyleneamino, alkylenediamino, and the like). Still further, for alkylene and heteroalkylene linking groups, no orientation of the linking group is implied by the direction in which the formula of the linking group is written. For example, the formula $-C(O)_2R'-$ represents both $-C(O)_2R'-$ and $-R'C(O)_2-$.

As used herein, "heteroaryl" refers to a 5- or 6-membered aromatic heterocycle that is optionally fused to an additional six-membered aromatic ring or to one 5- or 6-membered heteroaromatic ring, said heteroaromatic ring containing 1-3 heteroatoms that are selected from the group consisting of O, N and S in any combination. Any heteroaryl substituent is attached by a single bond, and is optionally and independently substituted one or more times with H, halogen, alkyl having 1-6 carbons, or alkoxy having 1-6 carbons. Selected examples of heteroaryl substituents are pyrrole, thiophene, or furan (single ring, single hetero atom), oxazole, isoxazole, oxadiazole, or imidazole (single ring, multiple hetero atoms). Examples of multi-ring heteroaryl groups include benzoxazole, benzothiazole, benzimidazole (multi-ring, multiple hetero atoms), benzofuran or indole (multi-ring, single hetero atom).

The terms "cycloalkyl" and "heterocycloalkyl", by themselves or in combination with other terms, represent, unless otherwise stated, cyclic versions of "alkyl" and "heteroalkyl", respectively. Additionally, for heterocycloalkyl, a heteroatom can occupy the position at which the heterocycle is attached to the remainder of the molecule. Examples of cycloalkyl include, but are not limited to, cyclopentyl, cyclohexyl, 1-cyclohexenyl, 3-cyclohexenyl, cycloheptyl, and the like. Examples of heterocycloalkyl include, but are not limited to, 1-(1,2,5,6-tetrahydropyridyl), 1-piperidinyl, 2-piperidinyl, 3-piperidinyl, 4-morpholinyl, 3-morpholinyl, tetrahydrofuran-2-yl, tetrahydrofuran-3-yl, tetrahydrothien-2-yl, tetrahydrothien-3-yl, 1-piperazinyl, 2-piperazinyl, and the like.

As used herein, "alkoxy" refers to the group -O-alkyl wherein alkyl is defined herein. Alkoxy includes, by way of example, methoxy, ethoxy, *n*-propoxy, isopropoxy, *n*-butoxy, *t*-butoxy, *sec*-butoxy, and *n*-pentoxy.

As used herein, "alkenyl" refers to alkenyl groups having from 2 to 22 carbon atoms, preferably 2 to 4 carbon atoms, and having at least 1 and preferably from 1 to 2 sites of alkenyl unsaturation. Such groups are exemplified, for example, by vinyl, allyl, and but-3-en-1-yl.

As used herein, "alkenoxy" refers to the group -O-alkenyl wherein alkenyl is defined herein. Alkenoxy includes, by way of example, vinyloxy, allyloxy, 1-butenoxy, 2-butenoxy, 2-pentenoxy, 3-pentenoxy, 4-pentenoxy.

As used herein, "alkylaryl" refers to an alkyl group substituted with an aryl group (*e.g.,* an aromatic group or an heteroaromatic group).

As used herein, "heterocycle" or "heterocyclic" or "heterocycloalkyl" or "heterocyclyl" refers to a saturated or unsaturated group having a single ring or multiple condensed rings, including fused bridged and spiro ring systems, from 1 to 10 carbon atoms and from 1 to 4 hetero atoms selected from the group consisting of nitrogen, sulfur or oxygen within the ring wherein, in fused ring systems, one or more the rings may be cycloalkyl, aryl or heteroaryl provided that the point of attachment is through the nonaromatic ring. In one embodiment, the nitrogen and/or sulfur atom(s) of the heterocyclic group are optionally oxidized to provide for the N-oxide, sulfinyl, sulfonyl moieties.

As used herein, "sulfo" refers to sulfonic acid or sulfonate.

As used herein, "halogen" refers to fluorine, chlorine, bromine, or iodine.

Each of the above terms (*e.g.,* "alkyl," "heteroalkyl," "aryl" and "heteroaryl") includes both substituted and unsubstituted forms of the indicated radical.

As used herein, the term "heteroatom" includes oxygen (O), nitrogen (N), sulfur (S), phosphorous (P) and selenium (Se).

The term "amino" or "amine group" refers to the group -NR'R" (or $-N^+RR'R"$) where R, R' and R" are independently selected from the group consisting of hydrogen, alkyl, substituted alkyl, aryl, substituted aryl, aryl alkyl, substituted aryl alkyl, heteroaryl, and substituted heteroaryl. A substituted amine being an amine group wherein R' or R" is other than hydrogen. In a primary amino group, both R' and R" are hydrogen, whereas in a secondary amino group, either,

but not both, R' or R" is hydrogen. In addition, the terms "amine" and "amino" can include protonated and quaternized versions of nitrogen, comprising the group $-N^+RR'R"$ and its biologically compatible anionic counterions.

The term "sample" as used herein refers to any material that may contain nucleic acid. The sample may also include diluents, buffers, detergents, and contaminating species, debris and the like that are found mixed with the target. Illustrative examples include urine, sera, blood plasma, total blood, saliva, tear fluid, cerebrospinal fluid, secretory fluids and the like. Also included are solid, gel or substances such as mucus, body tissues, cells and the like suspended or dissolved in liquid materials such as buffers, extractants, solvents and the like. Typically, the sample is a live cell, a biological fluid that comprises endogenous host cell proteins, nucleic acid polymers, nucleotides, nucleosides, oligonucleotides, peptides and buffer solutions. The sample may be in an aqueous solution, a viable cell culture or immobilized on a solid or semi solid surface such as a polyacrylamide gel, membrane blot or on a microarray.

As used herein, "microRNA" or "miRNA" as used herein refers to a small ribonucleic acid (RNA) molecule consisting of about 25 or fewer nucleotides. Typical miRNA molecules have a length of between about 21 to 25 nucleotides. MicroRNAs are highly conserved RNA molecules that modulate the expression of their cognate mRNA molecules and prevent gene expression.

As used herein, "dye" refers to a compound that emits light to produce an observable detectable signal.

The term "detectable signal" as used herein refers to a change in or an occurrence of, a signal that is directly or indirectly detectable either by observation or by instrumentation. Typically, the detectable signal is an optical response resulting in a change in the wavelength distribution patterns or intensity of absorbance or fluorescence or a change in light scatter, fluorescence lifetime, fluorescence polarization, or a combination of the above parameters.

As used herein, the term "kit" refers to a packaged set of related components, typically one or more compounds or compositions. The kits disclosed herein comprise one or more of the cyanine dye compounds described herein, one or more carriers suitable for *in vitro* applications, and one or more containers in which to store the one or more dyes and/or one or more carriers, such as solvents, buffers, stabilizers, pH adjusting agents, *etc.* The kit optionally contains instructions for how to use and detect the dye or composition containing the dye in an assay for detection of ssRNA in a sample. The kit can further contain one or more pieces of equipment to administer the dye, or composition containing the dye including, but not limited to, syringes, pipettes, pipette bulbs, spatulas, vials, syringe needles, and various combinations thereof.

## The Compounds:

Disclosed herein are substituted unsymmetrical cyanine dyes and methods for their use in the detection of RNA, such as short and/or single-stranded RNA, as well as compositions, kits, including substituted unsymmetrical cyanine dyes. The compounds, compositions, methods, and kits can be used to detect RNA, for example, by conventional fluorescence microscopy, fluorescence spectroscopy, flow cytometry, and/or high content imaging.

The dye compounds disclosed herein, and compositions containing those dyes, can be used for a variety of *in vitro* or *ex vivo* assays designed for the detection of RNA molecules, *e.g.,* short and/or single-stranded RNA molecules. For example, the dyes can be used for single cell imaging or to assay a cell suspension, during which the dye(s) are loaded into cells by incubation with the cells for a sufficient period of time. Specific assays include, but are not limited to, those with live organ cultures as well as cell culture assays.

The disclosed dyes and compositions can be used advantageously for the detection of short and/or single-stranded RNA molecules. It was surprisingly found that the dye compounds and the methods disclosed herein can be used to detect short, single-stranded RNA molecules that are usually not detectable by standard fluorescent methods. Furthermore, the methods provided herein can detect very low concentrations of short RNA molecules even in the presence of larger RNA molecules such as rRNA and mRNA. It was also surprisingly found that the compositions and methods provided herein are more specific for short RNA molecules than for larger RNA molecules. The compositions and methods provided herein overcome many of the disadvantages associated with conventional double-stranded nucleic acid binding dyes and provide new opportunities for the use in the analysis and quantification of RNA.

The dye compounds disclosed herein can be generally described to comprise: 1) a substituted benzazolium ring, 2) a bridging methine, and 3) a quinolinium ring system, one or more positions of which may be substituted by a TAIL that contains at least one heteroatom.

## TAIL

TAIL is a heteroatom-containing side chain that is described by the formula LINK-SPACER-CAP. LINK is the linking moiety by which TAIL is attached to the core structure of the dye compounds disclosed herein. SPACER is a covalent linkage that connects LINK to CAP. CAP is the portion of TAIL that possesses a heteroatom component.

LINK is a single covalent bond, an ether linkage (-O-), a thioether linkage (-S-), or an amine linkage ($-NR_2-$). In each embodiment, LINK forms the attachment between the dye core structure and SPACER. When LINK is an amine,

the amine substituent ($R_{20}$) is optionally H, such that LINK-NH-. Alternatively, $R_{20}$ is a linear or branched alkyl having 1-8 carbons. In another embodiment, $R_{20}$ is -SPACER'-CAP', yielding a TAIL having the formula:

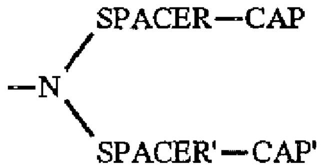

where SPACER' and CAP', respectively, may be the same as or different from SPACER and CAP, and are selected from the same alternatives defined for SPACER and CAP, respectively. For the sake of simplifying the description, SPACER and CAP are defined with the understanding that a description of SPACER includes SPACER' and a description of CAP includes CAP'.

SPACER is a covalent linkage that joins LINK and CAP. SPACER is a linear, branched, cyclic, heterocyclic, saturated or unsaturated arrangement of 1-16 carbon (C), nitrogen (N), phosphorous (P), oxygen (O) or sulfur (S) atoms. Alternatively, SPACER is a single covalent bond, such that both LINK and SPACER are not simultaneously single covalent bonds. Preferably, the SPACER linkage must begin and end with a carbon atom. Typically, if SPACER consists of a single atom, it is required to be a carbon atom, so that the first and last atom in SPACER (in this specific instance, they are the same atom) is a carbon. The 1-16 atoms making up SPACER are combined using any appropriate combination of ether, thioether, amine, ester, or amide bond; or single, double, triple or aromatic carbon-carbon bonds; or phosphorous-oxygen bonds; or phosphorus-sulfur bonds; or nitrogen-nitrogen bonds; or nitrogen-oxygen bonds; or aromatic or heteroaromatic bonds. SPACER is further substituted by hydrogen to accommodate the valence state of each atom in SPACER.

Generally, the atoms of SPACER are arranged such that all heteroatoms in the linear backbone of SPACER are separated by at least one carbon atom, and preferably separated by at least two carbon atoms. Typically, SPACER is 1-6 carbon atoms in a linear or branched saturated chain. SPACER incorporates a 6-membered heteroaromatic ring (phenylene linkage) or SPACER incorporates a 5- or 6-membered heteroaromatic ring, wherein the heteroatoms are O, N, or S. Alternatively, SPACER incorporates amide linkages, ester linkages, simple ethers and thioethers, and amines in a linear arrangement, such as $-CH_2-CH_2-(C{=}O)-NH-CH_2-CH_2-CH_2-$. Preferably, SPACER is an alkylene ($-(CH_2)_k-$, where k=1-8).

LINK and SPACER, in combination, serve to attach a heteroatom-containing group, CAP, to the dye core structure. CAP may contain oxygen, sulfur, or nitrogen, according to the formulae $-OR_{21}-$, $-SR_{21}$, $-NR_{21}R_{22}$, or $-N^+R_{21}R_{22}R_{23}\ \Psi^-$. The substituents $R_{21}$, $R_{22}$ and $R_{23}$ are independently H, or a linear or branched alkyl or cycloalkyl having 1-8 carbons. Where any of $R_{21}$, $R_{22}$ and $R_{23}$ are alkyl or cycloalkyl, they are optionally further substituted by halogen, hydroxyl, alkoxy having 1-8 carbons, amino, carboxy, or phenyl, where phenyl is optionally further substituted by halogen, hydroxyl, alkoxy having 1-8 carbons, amino, aminoalkyl having 1-8 carbons, or carboxyalkyl having 1-8 carbons. One or more of $R_{21}$, $R_{22}$ and $R_{23}$, taken in combination with SPACER forms a 5- or 6-membered ring that is aromatic, heteroaromatic, alicyclic or heteroalicyclic ring. When the 5- or 6-membered ring is heteroaromatic or heteroalicyclic, the ring contains 1-3 heteroatoms that are O, N or S. Alternatively, one or more of $R_{21}$, $R_{22}$, and $R_{23}$, taken in combination with $R_{20}$ and SPACER, forms a 5- or 6-membered ring that is aromatic, heteroaromatic, alicyclic or heteroalicyclic, as described above. Preferably, $R_{21}$ and $R_{22}$ are hydrogen, or alkyl having 1-8 carbons. $R_{23}$ is typically H or alkyl having 1-8 carbons.

When CAP is $-N^+R_{21}R_{22}R_{23}\ \Psi^-$, the biologically compatible counter ion $\Psi^-$ balances the positive charge present on the CAP nitrogen, which is a quaternary ammonium salt. As used herein, a substance that is biologically compatible is not toxic as used, and does not have a substantially deleterious effect on biomolecules. Examples of $\Psi^-$ include, among others, chloride, bromide, iodide, sulfate, alkanesulfonate, arylsulfonate, phosphate, perchlorate, tetrafluoroboronate, tetraarylboride, nitrate, and anions of aromatic or aliphatic carboxylic acids. Preferred $\psi$- counterions are bromide, chloride, iodide, perchlorate, and various sulfonates.

Additionally, CAP may incorporate a cyclic structure. CAP typically incorporates a 5- or 6-membered nitrogen-containing ring, optionally including an additional heteroatom (typically oxygen), where the ring nitrogen is optionally substituted by $R_{23}$ to give an ammonium salt. Specific versions of CAP include, but are not limited to,

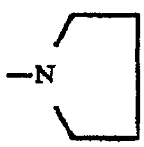, 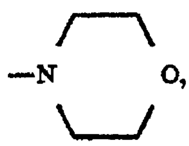, 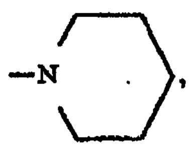,

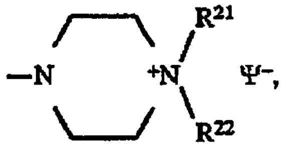, 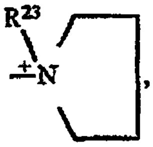, 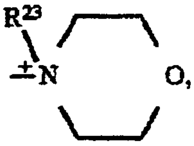,

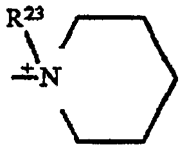, 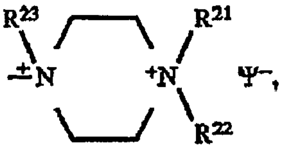,

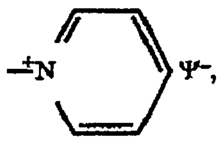, or 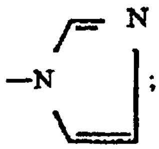;

CAP is preferably $-NR_{21}R_{22}$ or $-N{+}R_{21}R_{22}R_{23}\Psi^-$, where $R_{21}$, $R_{22}$, and $R_{23}$ are alkyls having 1-6 carbons. More preferably, CAP is $-N(CH_3)_2$ or $-N^+(CH_3)_3\Psi^-$.

Preferably, TAIL contains 6-10 non-hydrogen atoms, including LINK and CAP. Selected examples of TAIL are listed in Table 1. For each TAIL, the identities of LINK, SPACER and CAP are specified. Where $R_{21}$, $R_{22}$, or $R_{23}$ combined with either $R_{20}$ or SPACER, the combination is indicated in this table.

**Table 1:** Specific Examples of TAIL Moieties

| TAIL | LINK | SPACER/ SPACER' | CAP/ CAP' |
|---|---|---|---|
| 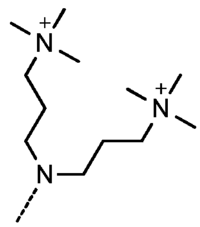 | -N-SPACER-CAP<br>-N-SPACER'-CAP' | $-CH_2CH_2CH_2-$<br>$-CH_2CH_2CH_2-$ | $-N{+}(CH_3)_3$<br>$-N{+}(CH_3)_3$ |
| 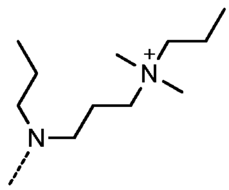 | $-N(CH_2CH_2CH_3)-$ | $-CH_2CH_2CH_2-$ | $-N^+(CH_3)_2[(CH_2)_2CH_3]$ |
| 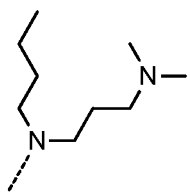 | $-N(CH_2CH_2CH_2CH_3)-$ | $-CH_2CH_2CH_2-$ | $-N(CH_3)_2$ |
| 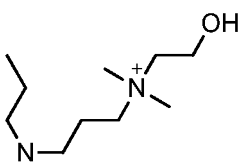 | $-N(CH_2CH_2CH_3)-$ | $-CH_2CH_2CH_2-$ | $-N^+(CH_3)_2[(CH_2)_2OH]$ |

(continued)

| TAIL | LINK | SPACER/ SPACER' | CAP/ CAP' |
|---|---|---|---|
| 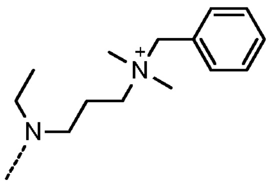 | $-N(CH_2CH_3)-$ | $-CH_2CH_2CH_2-$ | $-N^+(CH_3)_2[(CH_2)$-phenyl] |
| 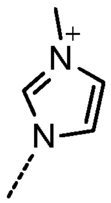 | $-NR^{23}-$ | -CH=N-CH=CH- | 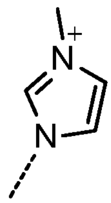 |

In the methods provided herein, the sample suspected of containing a short RNA molecule (*e.g.,* microRNA) is combined with a substituted unsymmetrical cyanine dye compound. An exemplary substituted, unsymmetrical cyanine dye compound is represented by the structure shown in Formula **I:**

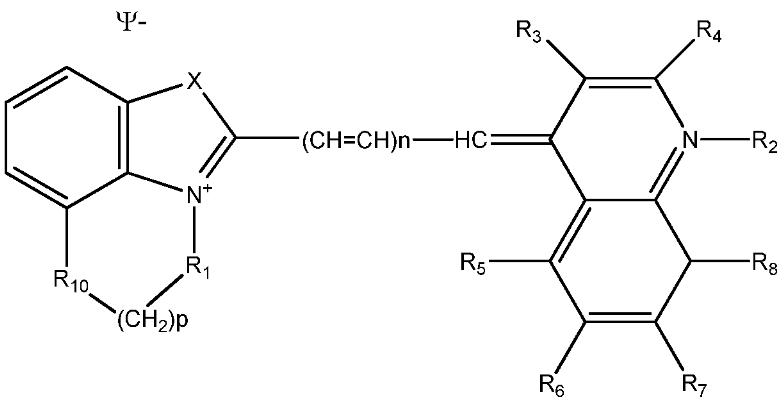
(I)

wherein

X is O or S;

n is 0, 1 or 2;

p is 0, 1 or 2;

Ψ is a biologically compatible counterion;

$R_1$ and $R_{10}$, which can be the same or different, are independently H; or a substituted or unsubstituted alkyl group (*e.g.,* an alkyl group having 1-6 carbons), wherein the substitution on the alkyl group, if present, is a carboxyl or (alkyl)ammonium group;

$R_2$ is a substituted or unsubstituted alkyl, naphthyl, phenyl, thienyl, or cycloalkyl having 3-8 carbons; wherein the substitution on $R_2$, if present, is a carboxyl or carboxamide group;

$R_3$ is H; or an alkyl, alkenyl, polyalkenyl, alkynyl or polyalkynyl group (*e.g.,* having 1-6 carbons); or a halogen; or a substituted or unsubstituted aryl or heteroaryl; or a substituted or unsubstituted cycloalkyl having 3-10 carbons; or $-OR_{11}$, $-SR_{11}$, $-(NR_9R_{11})$; or $-OSO_2R_{19}$ ; or a TAIL;

wherein $R_9$ and $R_{11}$, which can be the same or different, are independently H; or substituted or unsubstituted alkyl groups (*e.g.,* alkyl groups having 1-6 carbons); or alkyl groups substituted with one or more alkylammonium groups; or 1-2 alicyclic or aromatic rings; or $R_9$ and $R_{11}$ taken in combination are $-(CH_2)_4-$ or $-(CH_2)_5-$ to give a 5 or 6 membered ring; and where $R_{19}$ is alkyl (*e.g.,* having 1-6 carbons), or perfluoroalkyl (*e.g.,* having 1-6 carbons), or aryl;

$R_4$ is a substituted or unsubstituted alkyl, a substituted or unsubstituted alkenyl, a substituted or unsubstituted polyalkenyl, a substituted or unsubstituted alkynyl or a substituted or *unsubstituted* polyalkynyl group (*e.g.,* having 1-6 carbons); or a halogen; or a substituted or unsubstituted aryl or heteroaryl; or a substituted or unsubstituted cycloalkyl having 3-10 carbons; or $-OR_{11}$, $-SR_{11}$, $-(NR_9R_{11})$; or $-OSO_2R_{19}$; or a TAIL;

$R_5$, $R_6$, $R_7$ and $R_8$, which can be the same or different, are independently H; or an alkyl, alkenyl, polyalkenyl, alkynyl or polyalkynyl group (*e.g.,* having 1-6 carbons); or a halogen; or a substituted or unsubstituted aryl or heteroaryl; or a substituted or unsubstituted cycloalkyl having 3-8 carbons; or a TAIL; or $-OR_{11}$, $-SR_{11}$, or $-(NR_9R_{11})$;

TAIL is a heteroatom-containing moiety having the formula LINK-SPACER-CAP;

wherein

LINK is a single covalent bond, -O-, -S-, or $-NR_{20}-$; where $R_{20}$ is H, a linear or branched alkyl having 1-8 carbons, or $R_{20}$ is -SPACER'-CAP';

SPACER and SPACER', which can be the same or different, are covalent linkages, linear or branched, cyclic or heterocyclic, saturated or unsaturated, each having 1-16 nonhydrogen atoms selected from the group consisting of C, N, O and S, such that the linkage begins and ends with a carbon atom, and contains any combination of ether, thioether, amine, ester, amide, or aliphatic, oleic or aromatic carbon-carbon bonds, or aromatic carbon-nitrogen or nitrogen-nitrogen bonds; wherein all heteroatoms in the linear backbone of SPACER are separated by at least two carbon atoms;

CAP and CAP', which can be the same or different, are $-OR_{21}$, $-SR_{21}$, $-NR_{21}R_{22}$, or $-N^+R_{21}R_{22}R_{23}\Psi^-$;

wherein

$R_{21}$, $R_{22}$, and $R_{23}$ are independently H, or a linear or branched alkyl or cycloalkyl having 1-8 carbons, optionally further substituted by halogen, hydroxy, alkoxy having 1-8 carbons, carboxyalkyl having 1-8 carbons, or phenyl, where phenyl is optionally further substituted by halogen, hydroxy, alkoxy having 1-8 carbons, aminoalkyl having 1-8 carbons, or carboxyalkyl having 1-8 carbons; or, one or more of $R_{21}$, $R_{22}$, and $R_{23}$, taken in combination with SPACER or SPACER' or $R_{20}$ forms a 5- or 6-membered aromatic, heteroaromatic, alicyclic or heteroalicyclic ring, the heteroatoms selected from O, N or S; where $\Psi^-$ is a biologically compatible counterion; or

CAP and CAP' are independently

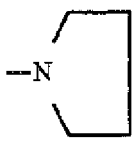, 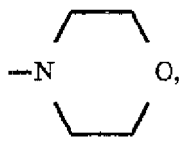, 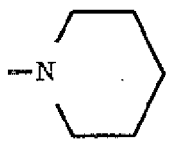,

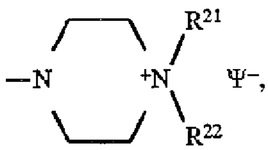, 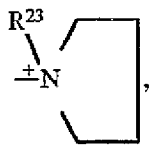, 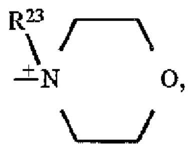,

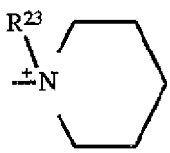, 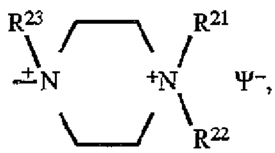,

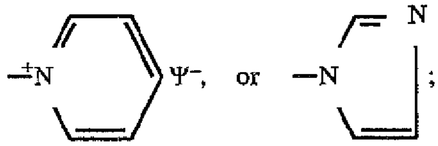

where $R_{21}$, $R_{22}$, $R_{23}$ and $\Psi^-$ are as defined previously; such that at least one of $R_3$, $R_4$, $R_5$, $R_6$, $R_7$, $R_8$ or $R_{10}$ is a TAIL; and, where more than one substituent is a TAIL, each TAIL is optionally the same or different;

or wherein at least one of $R_1$, $R_5$, $R_6$, $R_7$, $R_8$, $R_9$, $R_{10}$, $R_{11}$ or $R_{20}$, $R_{21}$, $R_{22}$ or $R_{23}$ is a water-soluble group.

When CAP is $-N^+R_{21}R_{22}R_{23}\ \Psi^-$, the biologically compatible counter ion $\Psi^-$ balances the positive charge present on the CAP nitrogen, which is a quaternary ammonium salt. As used herein, a substance that is biologically compatible is not toxic as used, and does not have a substantially deleterious effect on biomolecules. Examples of $\Psi^-$ include, among others, chloride, bromide, iodide, sulfate, alkanesulfonate, arylsulfonate, phosphate, perchlorate, tetrafluoroboronate, tetraarylboride, nitrate, and anions of aromatic or aliphatic carboxylic acids. Preferred $\psi$- counterions are bromide, chloride, iodide, perchlorate, and various sulfonates.

If p is 0, $R_1$ and $R_{10}$, which can be the same or different, are independently H; or a substituted or unsubstituted alkyl group (*e.g.,* an alkyl group having 1-6 carbons), wherein the substitution on the alkyl group, if present, is a carboxyl or (alkyl)ammonium group.

If p is not 0, $R_1$ and $R_{10}$, which can be the same or different, are independently a substituted or unsubstituted alkyl group (*e.g.,* an alkyl group having 1-6 carbons), wherein the substitution on the alkyl group, if present, is a carboxyl or (alkyl)ammonium group.

The water-soluble group can include a polymer (*e.g.*, a polyalkylene oxide, carbohydrate and polypeptide). The water-soluble group may comprise a polyethylene oxide. $R_4$ may be a TAIL; $R_2$ is a substituted or unsubstituted phenyl; and/or at least one of $R_5$, $R_6$, $R_7$ and $R_8$ is $-OR_{11}$, $-SR_{11}$, or - $NR_9R_{11}$). n may be 0. At least one of $R_5$, $R_6$, $R_7$ and $R_8$ may be $OR_{11}$. $OR_{11}$ may be $-OCH_3$. $R_7$ may be $-OCH_3$. $R_1$ may be an unsubstituted alkyl and $R_{10}$ is H. $R_1$ may be methyl and $R_{10}$ may be H.

The dye compound of Formula I is selected from the group consisting of Compound 4, Compound 5, Compound 6, Compound 7, Compound 8, Compound 9, Compound 10, Compound 11, Compound 12, Compound 13, Compound 14, Compound 15, Compound 16, Compound 17, Compound 18, Compound 19, Compound 20, Compound 21, Compound 23, Compound 25, Compound 26, Compound 27, Compound 30, Compound 31, Compound 32, Compound 33, Compound 34, Compound 35, Compound 36, Compound 37, Compound 38, Compound 39, Compound 40, Compound 41, Compound 42, Compound 43, Compound 44, Compound 45, Compound 46, Compound 47, Compound 48, Compound 49, Compound 50, Compound 51, Compound 52, Compound 53, Compound 54, Compound 55, Compound 56, Compound 57, Compound 58, Compound 59, and Compound 60.

The cyanine compounds disclosed herein optionally can include one or more groups that can enhance the solubility of the dye in an aqueous medium. Such groups are referred to herein as "water soluble groups" (WSG). In certain embodiments, such groups can include a water-soluble polymer. Various types of water-soluble polymers can be used as a WSG on the dyes disclosed herein. Typically, the water-soluble polymer group has a molecular weight of about 300 Da to 3000 Da and can be a charged, zwitterionic, or neutral compound. Representative examples of suitable water-soluble polymer groups include, without limitation, polyalkylene oxides, carbohydrates, polypeptide and a polyethylene oxide.

Certain dye embodiments described by Formula I are particularly sensitive to the presence of relatively short ssRNA sequences (*e.g.,* less than 25 nucleotides in length) when used in the methods provided herein. Representative examples of such dyes include those in which $R_4$ is a TAIL or $-(NR_9R_{11})$; $R_2$ is a substituted or unsubstituted phenyl; and/or at least one of $R_5$, $R_6$, $R_7$ and $R_8$ is $-OR_{11}$, $-SR_{11}$, or $-(NR_9R_{11})$.

A specific substituted unsymmetrical cyanine dye compound that can be particularly useful for detecting relatively short sequences of RNA (e.g., miRNA) in the disclosed methods is represented by the structure shown in Formula **II**:

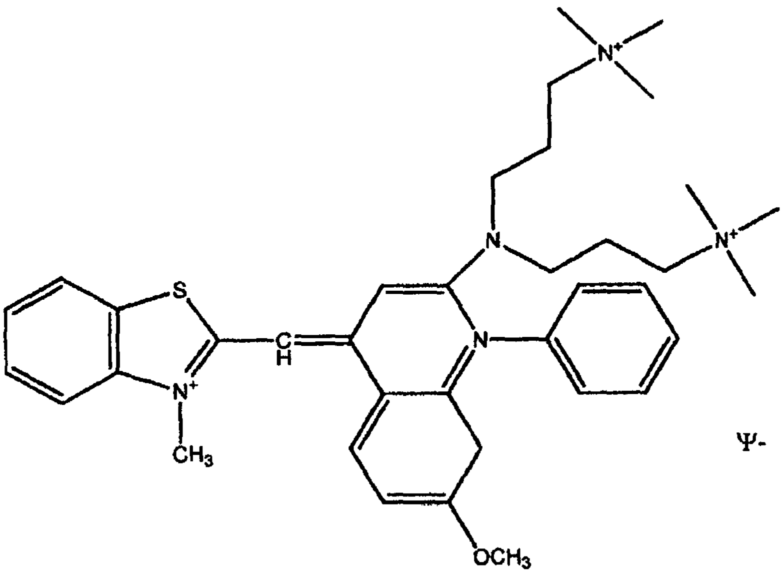

(II)

Further examples of substituted unsymmetrical cyanine dye compound for use in the disclosed methods are described in U.S. Patent No. 5,658,751.

It was surprisingly found that dyes of the present teachings that comprise a quaternary ammonium salt derived from either a benzyl or a 3-methylbenzyl group have increased fluorescent enhancement, as compared to the dye of Formula II, upon or after interacting with RNA. Without wishing to be bound by theory, the dye compounds of the present teachings with increased fluorescent enhancement comprise two positive charges: one from the benzothiazolium and the other from the tetraalkylammonium salt. Furthermore, the benzyl or 3-methylbenzyl group that forms the quaternary ammonium salt appears to have an effect on the hydrophobicity and/or hydrophilicity of the dye compounds disclosed herein.

The methods provided herein typically involve formation of a fluorescent complex if the sample contains a certain quantity of RNA. The association between the RNA and the dye compounds provided herein can involve a covalent or non-covalent (*e.g.,* ionic) interaction. In certain embodiments, the cyanine dyes disclosed herein bind to RNA non-covalently.

A fluorescent complex may be provided that includes ssRNA, wherein the ssRNA comprises 25 or fewer nucleotides, non-covalently bound to a dye of the Formula II:

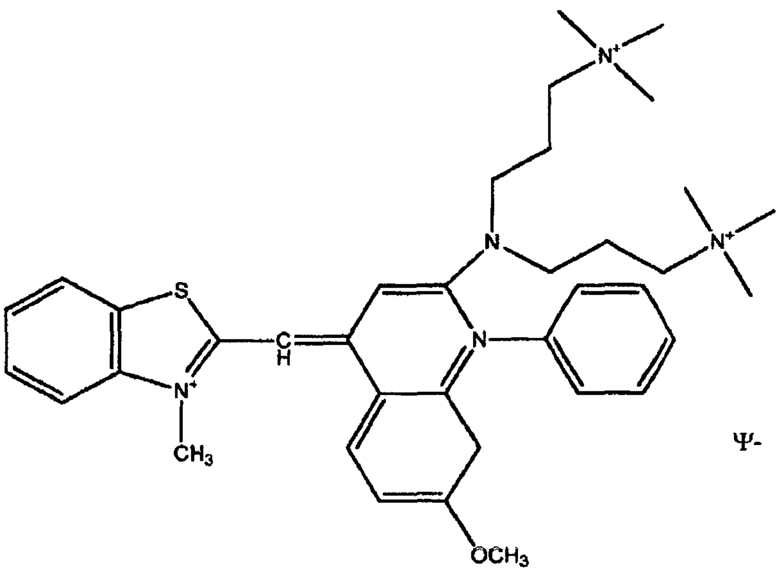

(II)

wherein the complex emits a detectable fluorescent signal.

A fluorescent complex may be produced that includes ssRNA, wherein the ssRNA comprises 25 or fewer

nucleotides, non-covalently bound to a dye compound selected from the group consisting of Compound 4, Compound 5, Compound 6, Compound 7, Compound 8, Compound 9, Compound 10, Compound 11, Compound 12, Compound 13, Compound 14, Compound 15, Compound 16, Compound 17, Compound 18, Compound 19, Compound 20, Compound 21, Compound 23, Compound 25, Compound 26, Compound 27, Compound 30, Compound 31, Compound 32, Compound 33, Compound 34, Compound 35, Compound 36, Compound 37, Compound 38, Compound 39, Compound 40, Compound 41, Compound 42, Compound 43, Compound 44, Compound 45, Compound 46, Compound 47, Compound 48, Compound 49, Compound 50, Compound 51, Compound 52, Compound 53, Compound 54, Compound 55, Compound 56, Compound 57, Compound 58, Compound 59, and Compound 60, wherein the complex emits a detectable signal.

The fluorescent signal generated by the fluorescent complex is extremely stable. For example, the signal can be stable for at least 30 minutes at room temperature. In some embodiments, the fluorescence signal is stable for at least 3 hours at room temperature.

Also provided herein is a dye compound selected from the group consisting of Compound 4, Compound 5, Compound 6, Compound 7, Compound 8, Compound 9, Compound 10, Compound 11, Compound 12, Compound 13, Compound 14, Compound 15, Compound 16, Compound 17, Compound 18, Compound 19, Compound 20, Compound 23, Compound 25, Compound 26, Compound 27, Compound 30, Compound 31, Compound 32, Compound 33, Compound 34, Compound 35, Compound 36, Compound 37, Compound 38, Compound 39, Compound 40, Compound 43, Compound 44, Compound 45, Compound 46, Compound 47, Compound 48, Compound 49, Compound 50, Compound 51, Compound 52, Compound 53, Compound 54, Compound 55, Compound 56, Compound 57, Compound 58, Compound 59, and Compound 60.

The dye compounds disclosed herein are typically dissolved or suspended in a carrier for use as a composition. The exact concentration of dye to be used is dependent upon the experimental conditions and the desired results, and optimization of experimental conditions is typically required to determine the best concentration of cyanine dye to be used in a given application. Typically for *in vitro* use, the concentration of the dye is the minimum amount required to yield a detectable signal in the sample within a reasonable time, with minimal background fluorescence.

Methods of Use:

A general procedure for using the cyanine dyes as disclosed herein for detecting for the presence of RNA molecules in a sample is described below:

A sample suspected of containing RNA (*e.g.,* ssRNA) is combined with a substituted, unsymmetrical cyanine dye compounds provided herein. The sample is incubated for a time sufficient for the dye compound to combine with the RNA in the sample, if present, to form a dye-RNA complex that emits a detectable fluorescent signal. The dye-RNA complex then is detected for the emission of the fluorescent signal, wherein the detectable fluorescent signal is indicative of the presence of a dye-RNA complex.

The sample can be a biological sample, such as cells, cell cultures, tissues, tissue and cell culture medium, organs, serum, whole blood, plasma, bodily fluids, buffer solutions, biological fluids, and the like. The cyanine dye compounds can be formulated with one or more carriers depending on the assay. The dye compounds then are incubated with the sample for a period of time sufficient for the dye compound to associate with the RNA species present in the sample. After such time, the sample is analyzed for fluorescence intensity. The fluorescence intensity after incubation is compared to the fluorescence intensity of the dye in the absence of sample. An increase in the fluorescence intensity of the dye in the biological sample indicates association of the dye with RNA (*i.e.,* the presence of RNA in the sample). The increased fluorescence can be measured or detected using any suitable instrument. Additionally, the fluorescence intensity after incubation can be compared to the fluorescence intensity of the dye in the presence of samples of known RNA concentrations for quantitative measurements.

In one method, a sample suspected of containing ssRNA (*e.g.,* miRNA) is combined with a substituted, unsymmetrical cyanine dye compound provided herein and then incubated for a time sufficient for the dye compound to combine with the ssRNA in the sample, if present, to form a dye-ssRNA complex. Once formed the dye-ssRNA complex can emit a detectable and stable fluorescent signal. After incubation, the emission of the fluorescent signal is detected, where a detectable fluorescent signal is indicative of the presence of a dye-ssRNA complex.

Typically, the fluorescence signal produced by the dye-RNA complex produced by the methods provided herein can be stable for at least 30 minutes at room temperature, and can be stable for much longer, *e.g.,* at least 3 hours at room temperature.

The detection of emitted light from fluorescent RNA-dye complexes can be achieved with any suitable optical detection system that provides spectral information for the complex, *e.g.*, grating spectrometer, prism spectrometer, imaging spectrometer, fluorometer, such as the QUBIT 2.0 Fluorometer (available from Life Technologies Corporation; Carlsbad, CA), fluorescence microplate reader, camera, flow cytometers, including bead-based and acoustic focusing flow cytometers, such as the ATTUNE Acoustic Focusing Flow Cytometer (available from Life Technologies Corporation),

or the like.

The present methods can be implemented to detect any type of RNA molecule, but are particularly useful in the detection of short RNA molecules. The disclosed methods are well-suited for the detection of short, single-stranded RNA molecules, such as miRNA. Short RNA molecules typically contain a sequence of about 10-100 nucleotides. For example, short RNA molecules can include less than about 50; or less than about 40; or less than about 30 nucleotides; or less than 20 nucleotides. In certain embodiments, the detectable RNA contains 25 or fewer nucleotides. In certain embodiments, methods provided herein can be used to detect RNA formed of a sequence of about 10-20 nucleotides; whereas in other methods, the detectable RNA is formed of a sequence of about 20-25 nucleotides. In certain embodiments, the detectable RNA is single-stranded and contains 25 or fewer nucleotides.

Methods provided herein can be used in the detection of any type of naturally occurring or chemically modified RNA molecule, including but not limited to relatively short ssRNA molecules, such as microRNA (miRNA), small nuclear RNA (snRNA), small nucleolar RNA (snoRNA), tiny non-coding RNA (tncRNA), small modulatory RNA (smRNA), and piwi-interacting RNA (piRNA). In certain embodiments, the RNA molecule can include a chemical modification to at least one sugar, phosphate or base group. For example, one common type of modification utilizes a chemical group other than a hydroxy or phosphate at the 5' termini of the RNA molecule.

The compositions and methods provided herein can be used to detect the presence of RNA and quantify the amount of RNA in a sample covering a wide range of concentrations. The dye compounds disclosed herein are extremely sensitive to the presence of RNA in a sample. For example, certain assays allow for the detection of RNA (*e.g.*, ssRNA) at concentrations as low as about 25 ng/mL of sample, although higher concentrations of RNA also can be detected using the methods provided herein.

The disclosed assays can be implemented with samples spanning a broad range of ssRNA concentrations. In certain embodiments, the sample can include ssRNA in a concentration of about 2.5 ng/mL to about 1000 ng/mL of sample. In certain embodiments, the sample can include ssRNA in a concentration of about 25 ng/mL to about 1000 ng/mL. In certain embodiments, the sample can include ssRNA in a concentration of about 50 ng/mL to 1000 ng/mL.

In one aspect, a method of detecting the presence of single-stranded RNA (ssRNA) in a sample is provided, the method includes:

(a) combining a sample suspected of containing ssRNA, wherein the ssRNA comprises 25 or fewer nucleotides, with a substituted, unsymmetrical cyanine dye compound selected from the group consisting of Compound 4, Compound 5, Compound 6, Compound 7, Compound 8, Compound 9, Compound 10, Compound 11, Compound 12, Compound 13, Compound 14, Compound 15, Compound 16, Compound 17, Compound 18, Compound 19, Compound 20, Compound 21, Compound 23, Compound 25, Compound 26, Compound 27, Compound 30, Compound 31, Compound 32, Compound 33, Compound 34, Compound 35, Compound 36, Compound 37, Compound 38, Compound 39, Compound 40, Compound 41, Compound 42, Compound 43, Compound 44, Compound 45, Compound 46, Compound 47, Compound 48, Compound 49, Compound 50, Compound 51, Compound 52, Compound 53, Compound 54, Compound 55, Compound 56, Compound 57, Compound 58, Compound 59 and Compound 60;
(b) incubating the sample for a time sufficient for the dye compound to combine with the ssRNA in the sample, if present, to form a dye-ssRNA complex that emits a detectable fluorescent signal; and
(c) detecting the emission of the fluorescent signal, wherein the detectable fluorescent signal is indicative of the presence of a dye-ssRNA complex.

In certain embodiments, the ssRNA comprises 20-25 nucleotides. In certain embodiments, the ssRNA comprises 10-20 nucleotides. In certain embodiments, the ssRNA is naturally occurring. In certain embodiments, the ssRNA comprises a chemical modification to at least one sugar, phosphate or base group. In certain embodiments, the ssRNA comprises a chemical group other than a hydroxyl or phosphate at its 5' termini. In certain embodiments, the ssRNA is microRNA (miRNA), small nuclear RNA (snRNA), small nucleolar RNA (snoRNA), tiny non-coding RNA (tncRNA), small modulatory RNA (smRNA), or piwi-interacting RNA (piRNA). In certain embodiments, the ssRNA is miRNA.

The methods provided herein can detect as little as 0.5 ng ssRNA in a sample even in the presence of larger RNA, such as mRNA or rRNA. In certain embodiments, the method further comprises detecting ssRNA in a concentration of 0.5 ng/mL or greater. In certain embodiments, the method further comprises detecting ssRNA in a concentration of 5 ng/mL or greater. In certain embodiments, the method further comprises detecting ssRNA in a concentration of 10 ng/mL or greater. In certain embodiments, the method further comprises detecting ssRNA in a concentration of 25 ng/mL or greater. In certain embodiments, the method further comprises detecting ssRNA in a concentration of about 5 ng/mL to about 1000 ng/mL. In certain embodiments, the method further comprises detecting ssRNA in a concentration of about 25 ng/mL to about 1000 ng/mL. In certain embodiments, the method further comprises detecting ssRNA in a concentration of about 50 ng/mL to about 1000 ng/mL. In certain embodiments, the sample can include ssRNA in a concentration of about 5 ng/mL to about 500 ng/mL. In certain embodiments, the sample can include ssRNA in a concentration of about 25 ng/mL to about 500 ng/mL. In certain embodiments, the sample can include ssRNA in a

concentration of about 50 ng/mL to about 100 ng/mL.

In certain embodiments, the fluorescence signal is stable for at least 30 minutes at room temperature. In certain embodiments, the fluorescence signal is stable for at least 3 hours at room temperature.

In yet another aspect, a method of detecting single-stranded RNA (ssRNA) in a sample is provided, the method includes:

(a) combining a sample suspected of containing ssRNA, wherein the ssRNA comprises 25 or fewer nucleotides, with a substituted, unsymmetrical cyanine dye compound of the Formula **II**:

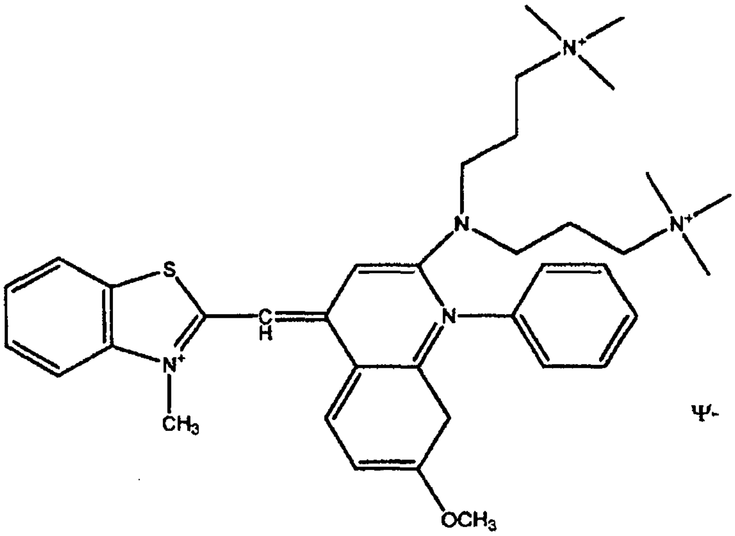

(II);

(b) incubating the sample for a time sufficient for the dye compound to combine with the ssRNA in the sample, if present, to form a dye-ssRNA complex that emits a detectable fluorescent signal; and

(c) detecting the emission of the fluorescent signal, wherein the detectable fluorescent signal is indicative of the presence of a dye-ssRNA complex.

In certain embodiments, the ssRNA comprises 20-25 nucleotides. In certain embodiments, the ssRNA comprises 10-20 nucleotides. In certain embodiments, the ssRNA is naturally occurring. In certain embodiments, the ssRNA comprises a chemical modification to at least one sugar, phosphate or base group. In certain embodiments, the ssRNA comprises a chemical group other than a hydroxyl or phosphate at its 5' termini. In certain embodiments, the ssRNA is microRNA (miRNA), small nuclear RNA (snRNA), small nucleolar RNA (snoRNA), tiny non-coding RNA (tncRNA), small modulatory RNA (smRNA), or piwi-interacting RNA (piRNA). In certain embodiments, the ssRNA is miRNA.

The methods provided herein can detect as little as 0.5 ng ssRNA in a sample even in the presence of larger RNA, such as mRNA or rRNA. In certain embodiments, the method further comprises detecting ssRNA in a concentration of 0.5 ng/mL or greater. In certain embodiments, the method further comprises detecting ssRNA in a concentration of 5 ng/mL or greater. In certain embodiments, the method further comprises detecting ssRNA in a concentration of 10 ng/mL or greater. In certain embodiments, the method further comprises detecting ssRNA in a concentration of 25 ng/mL or greater. In certain embodiments, the method further comprises detecting ssRNA in a concentration of about 5 ng/mL to about 1000 ng/mL. In certain embodiments, the method further comprises detecting ssRNA in a concentration of about 25 ng/mL to about 1000 ng/mL. In certain embodiments, the method further comprises detecting ssRNA in a concentration of about 50 ng/mL to about 1000 ng/mL. In certain embodiments, the sample can include ssRNA in a concentration of about 5 ng/mL to about 500 ng/mL. In certain embodiments, the sample can include ssRNA in a concentration of about 25 ng/mL to about 500 ng/mL. In certain embodiments, the sample can include ssRNA in a concentration of about 50 ng/mL to about 100 ng/mL.

In certain embodiments, the fluorescence signal is stable for at least 30 minutes at room temperature. In certain embodiments, the fluorescence signal is stable for at least 3 hours at room temperature.

The methods provided herein can be used to detect any type of short RNA molecule. The short RNA molecule can be a single-stranded RNA (ssRNA) molecule. The ssRNA can be naturally occurring or can include a chemical modification to at least one sugar, phosphate or base group. For example, the ssRNA can include a chemical group other than a hydroxy or phosphate at its 5' terminus. In certain embodiments, the ssRNA molecule can include 20-25 nucleotides. In other embodiments, the ssRNA molecule can include 10-20 nucleotides. In certain methods, the ssRNA

is microRNA (miRNA). The ssRNA can be a small nuclear RNA (snRNA), small nucleolar RNA (snoRNA), tiny non-coding RNA (tncRNA), small modulatory RNA (smRNA) or piwi-interacting RNA (piRNA).

The methods provided herein can be used to detect extremely small quantities of RNA (*e.g.,* ssRNA) in a sample. The methods provided herein can detect as little as 0.5 ng ssRNA in a sample even in the presence of larger RNA, such as mRNA or rRNA. In certain embodiments, the method further comprises detecting ssRNA in a concentration of 0.5 ng/mL or greater. In certain embodiments, the method further comprises detecting ssRNA in a concentration of 5 ng/mL or greater. In certain embodiments, the method further comprises detecting ssRNA in a concentration of 10 ng/mL or greater. In certain embodiments, the method further comprises detecting ssRNA in a concentration of 25 ng/mL or greater. In certain embodiments, the method further comprises detecting ssRNA in a concentration of about 5 ng/mL to about 1000 ng/mL. In certain embodiments, the method further comprises detecting ssRNA in a concentration of about 25 ng/mL to about 1000 ng/mL. In certain embodiments, the method further comprises detecting ssRNA in a concentration of about 50 ng/mL to about 1000 ng/mL. In certain embodiments, the sample can include ssRNA in a concentration of about 5 ng/mL to about 500 ng/mL. In certain embodiments, the sample can include ssRNA in a concentration of about 25 ng/mL to about 500 ng/mL. In certain embodiments, the sample can include ssRNA in a concentration of about 50 ng/mL to about 100 ng/mL.

In certain embodiments, the fluorescence signal is stable for at least 30 minutes at room temperature. In certain embodiments, the fluorescence signal is stable for at least 3 hours at room temperature.

Sample Preparation:

The sample may be prepared using methods well known in the art for isolating RNA for *in vitro* and solution based assay detection or well known methods for live cell or fixed cells for intracellular and/or detection of RNA. The sample includes, without limitation, any biological derived material that is thought to contain a nucleic acid polymer, such as RNA. Alternatively, samples also include material that nucleic acid polymers, such as RNA, have been added to such as a PCR reaction mixture, a polymer gel such as agarose or polyacrylamide gels or a microfluidic assay system. In another aspect of the present disclosure, the sample can also include a buffer solution that contains RNA to determine the cyanine dye compounds of the present disclosure that are ideal under different assay conditions.

The sample can be a biological fluid such as whole blood, plasma, serum, nasal secretions, sputum, saliva, urine, sweat, transdermal exudates, cerebrospinal fluid, or the like. Biological fluids also include tissue and cell culture medium wherein an analyte of interest has been secreted into the medium. Alternatively, the sample may be whole organs, tissue or cells from the animal. Examples of sources of such samples include muscle, eye, skin, gonads, lymph nodes, heart, brain, lung, liver, kidney, spleen, thymus, pancreas, solid tumors, macrophages, mammary glands, mesothelium, and the like. Cells include without limitation prokaryotic cells such as bacteria, yeast, fungi, mycobacteria and mycoplasma, and eukaryotic cells such as nucleated plant and animal cells that include primary cultures and immortalized cell lines. Typically prokaryotic cells include *E. coli* and *S. aureus.* Eukaryotic cells include without limitation ovary cells, epithelial cells, circulating immune cells, $\beta$ cells, hepatocytes, and neurons.

In an exemplary embodiment, the sample comprises biological fluids, buffer solutions, live cells, fixed cells, eukaryotic cells, prokaryotic cells, nucleic acid polymers, nucleosides, nucleotides, a polymeric gel or tissue sections. In a further aspect, the sample comprises nucleic acid polymers in an aqueous buffer.

The RNA may be either natural (biological in origin) or synthetic (prepared artificially). The presence of the RNA in the sample may be due to a successful or unsuccessful experimental methodology, undesirable contamination, or a disease state. RNA may be present in all, or only part, of a sample, and the presence of RNA may be used to distinguish between individual samples, or to differentiate a portion or region within a single sample.

The RNA may be enclosed in a biological structure, for example contained within a viral particle, an organelle, or within a cell. The RNA enclosed in biological structures may be obtained from a wide variety of environments, including cultured cells, organisms or tissues, unfiltered or separated biological fluids such as urine, cerebrospinal fluid, blood, lymph fluids, tissue homogenate, mucous, saliva, stool, or physiological secretions or environmental samples such as soil, water and air. The RNA may be endogenous or introduced as foreign material, such as by infection or by transfection.

Alternatively, the RNA is not enclosed within a biological structure, but is present as a sample solution. The sample solution can vary from one of purified RNA to crude mixtures such as cell extracts, biological fluids and environmental samples. In some cases it is desirable to separate the RNA from a mixture of biomolecules or fluids in the solution prior to combination with the present cyanine dye compounds. Numerous, well known, techniques exist for separation and purification of RNA from generally crude mixtures with other proteins or other biological molecules.

The sample may be incubated in the presence of the cyanine dye compounds disclosed herein for a time sufficient to form an RNA (*e.g.*, ssRNA)-reporter molecule complex. While permeation and complexation may be more or less rapid for the dye compounds disclosed herein, largely depending on the nature of the substituents present on the dye compound. It should be apparent to one skilled in the art that the time necessary for sufficient permeation of the dye, or sufficient formation of the resulting ssRNA-dye complex, is dependent upon the physical and chemical nature

of the individual sample and the sample medium (see for example U.S. Patent No. 5,658,751).

Illumination:

The sample containing an RNA-dye complex may be illuminated with a wavelength of light selected to give a detectable optical response, and observed with a means for detecting the optical response. By optical response is meant any detectable colorimetric or luminescent property of the complex. Typically, the optical response is related to the excitation or emission properties of the complex.

For example, the sample may be excited by a light source capable of producing light at or near the wavelength of maximum absorption of the fluorescent complex, such as an ultraviolet or visible lamp, an arc lamp, a laser, or even sunlight. The optical response is optionally detected by visual inspection, or by use of any of the following devices: CCD camera, video camera, photographic film, laser-scanning devices, fluorometers, photodiodes, quantum counters, epifluorescence microscopes, scanning microscopes, flow cytometers, fluorescence microplate readers, or by means for amplifying the signal such as photomultiplier tubes. Where the sample is examined using a flow cytometer, examination of the sample optionally includes sorting portions of the sample according to their fluorescence response.

The detection of emitted light from fluorescent RNA-dye complexes can be achieved with any suitable optical detection system that provides spectral information for the complex, *e.g.,* grating spectrometer, prism spectrometer, imaging spectrometer, fluorometer, such as the QUBIT 2.0 Fluorometer (available from Life Technologies Corporation; Carlsbad, CA), fluorescence microplate reader, camera, flow cytometers, including bead-based and acoustic focusing flow cytometers, such as the ATTUNE Acoustic Focusing Flow Cytometer (available from Life Technologies Corporation), or the like.

The wavelengths of the excitation and emission bands of the cyanine dye compounds disclosed herein vary with reporter molecule composition to encompass a wide range of illumination and detection bands. This allows the selection of individual reporter molecules for use with a specific excitation source or detection filter. In particular, present reporter molecules can be selected that possess excellent correspondence of their excitation band with the 488 nm band of the commonly used argon laser or emission bands which are coincident with preexisting filters.

The presence, location, and distribution of RNA, particularly single-stranded RNA, may be detected using the spectral properties of the RNA-dye complex. Once the RNA-dye complex is formed, its presence may be detected and used as an indicator of the presence, location, or type of nucleic acids in the sample, or as a basis for sorting cells, or as a key to characterizing the sample or cells in the sample. Such characterization may be enhanced by the use of additional reagents, including fluorescent reagents. The RNA (*e.g.,* ssRNA) concentration in a sample can also be quantified by comparison with known relationships between the fluorescence of the RNA-dye complex and concentration of nucleic acids in the sample. In particular, fluorescence intensity may be compared to a standard curve prepared from samples containing known nucleic acid concentrations, particularly RNA concentrations.

Kits:

The substituted unsymmetrical cyanine dye compounds for use in the disclosed methods and compositions described herein also can form part of a kit. Exemplary kits provided herein can include one or more substituted unsymmetrical cyanine dye compound, as disclosed herein (*e.g.,* a dye of Formula **I** or **II**) and instructions for using the dye in a method for detecting for the presence of ssRNA in a sample.

In yet another aspect, a kit for detecting the presence of single-stranded RNA (ssRNA) in a sample is provided that includes:

a dye compound selected from the group consisting of Compound 4, Compound 5, Compound 6, Compound 7, Compound 8, Compound 9, Compound 10, Compound 11, Compound 12, Compound 13, Compound 14, Compound 15, Compound 16, Compound 17, Compound 18, Compound 19, Compound 20, Compound 23, Compound 25, Compound 26, Compound 27, Compound 30, Compound 31, Compound 32, Compound 33, Compound 34, Compound 35, Compound 36, Compound 37, Compound 38, Compound 39, Compound 40, Compound 43, Compound 44, Compound 45, Compound 46, Compound 47, Compound 48, Compound 49, Compound 50, Compound 51, Compound 52, Compound 53, Compound 54, Compound 55, Compound 56, Compound 57, Compound 58, Compound 59, and Compound 60; and

instructions for using the dye in a method as disclosed herein.

Such kits can be prepared from readily available materials and reagents and can come in a variety of embodiments. The contents of the kit will depend on the design of the assay protocol or reagent for detection or measurement. All kits will contain instructions, appropriate reagents, and one or more of the presently disclosed cyanine dye compounds.

Typically, instructions include a tangible expression describing the reagent concentration or at least one assay method parameter such as the relative amounts of reagent and sample to be added together, maintenance time periods for reagent/sample admixtures, temperature, buffer conditions and the like to allow the user to carry out any one of the methods or preparations described above. In one aspect, the kit is formulated to facilitate the high-throughput screening of multiple samples, such as may be accomplished using automated methods.

Thus, a kit for detecting nucleic acid in a sample may comprise a dye compound as described above. The kit may further include instructions for performing one or more of the above disclosed methods, including the detection and/or quantitation of ssRNA.

The kit may optionally further include one or more of the following; sample preparation reagents, a buffering agent, nucleic acid standards, an aqueous nucleic acid reporter molecule dilution buffer, an organic solvent or an additional detection reagent, particularly where the additional detection reagent is an additional distinct nucleic acid reporter molecule.

In an exemplified embodiment, the dilution buffer (for the nucleic acid reporter molecule) contains a detergent in a final concentration of about 0.01% to about 0.5% (w/v). The detergents are as disclosed about and include CHAPS, TRITON-X, SDS and TWEEN 20.

The foregoing kits having been described it is understood that the many and varied compounds disclosed herein can be utilized with the many kits. The compounds not being limited to just those that are specifically disclosed.

The following examples are offered to illustrate but not to limit the embodiments described herein.

## EXAMPLES

### EXAMPLE 1: MyQubit microRNA Assay

The MyQubit miRNA Assay can be performed using a QUBIT 2.0 Fluorometer (available from Life Technologies Corporation; Cat. no. Q32866) equipped with V3.10 firmware or later to measure the concentration of microRNA in a sample.

The assay utilizes the dye compound of Formula **II** (referred to in this example as the "binding dye"), which exhibits a large increase in fluorescence upon binding to single-stranded DNA molecules. The assay described herein uses a chemically modified siRNA 21-mer (SILENCER Select GAPDH from Life Technologies Corporation).

The binding dye has been used to reproducibly quantify siRNA in pure samples at levels as low as 0.5 ng in the assay tube following the below protocol. The assay also can be applied to quantify miRNA in samples to similarly low levels.

The MyQubit miRNA Assay has a core dynamic range of 5-500 ng/mL miRNA in the assay tube (Figure 1). Because the assay tolerates 1-20 μL of sample in an assay volume of 200 μL, accurate results can be obtained for initial sample concentrations from about 50 ng/mL-100 μg/mL.

The following materials from Life Technologies Corporation are used in the assay: 20× TE Buffer (pH 7.5) (10 mM Tris, 1 mM EDTA (pH 7.5)); miRNA Std. 1 (Cat. no. T11493); 20× TE Buffer (pH 7.5) + 0.01% CHAPS (w/v) (*e.g.,* ZOOM CHAPS, Cat. no. ZC10003); SILENCER Select GAPDH Positive Control siRNA (Hs, Mm, Rn); and miRNA Std. 2 (Cat. no. 4390849); QUBIT Assay Tubes (Cat. no. Q32856) or AXYGEN PCR-05-C tubes (VWR part no. 10011-830); and plastic tubes for preparing buffers and dilutions of standards/samples. The amount of binding dye indicated above provides enough material for 1000 miRNA assays using the QUBIT 2.0 Fluorometer and following the protocol described below.

Material Storage and Handling: The binding dye should be stored at room temperature and protected from light. Buffers can be stored at room temperature for short periods of time (weeks); for long-term storage, these components should be stored at 4°C. SILENCER Select siRNA should be stored at < -20°C until use. Once reconstituted, the siRNA (and subsequent dilutions) should be stored at 4°C. The product information provided with the materials provides additional storage and handling information, including disposal.

Instrument Set-Up: The MyQubit miRNA Assay file (available at www.lifetechnologies.com/qubit) is uploaded to the fluorometer following the manufacturer's instruction (Figure 2). The QUBIT 2.0 Fluorometer will recognize the miRNA assay.qbt file and guide the user through the upload process.

The assay can be run using the following parameters:

Assay Temperature: The MyQubit miRNA Assay is designed to be performed at room temperature and delivers optimal performance when all solutions are at room temperature (22-28°C). Temperature fluctuations can influence the accuracy of the assay. To minimize temperature fluctuations, store the binding dye and assay buffer at room temperature and insert all assay tubes into the fluorometer only for as much time as it takes for the instrument to measure the fluorescence; the fluorometer can raise the temperature of the assay solution, even over a period of a few minutes. The assay tubes should not be held in the hand before reading, as this warms the solution and can result in a low reading.

Incubation Time: To allow the MyQubit miRNA Assay to reach optimal fluorescence, incubate the tubes for 2

minutes after mixing the sample or standard with the working solution. After this incubation period, the fluorescence signal is stable for at least 3 hours at room temperature.

Calibrating the QUBIT 2.0 Fluorometer: For each assay, one has the choice to run a new calibration or to use the values from the previous calibration. When first using the instrument for the described assay, it is recommended to conduct a new calibration be run each time. Once familiar with the assays, the instrument, pipetting accuracy, and temperature fluctuations within the laboratory, it can be acceptable to use the calibration data stored from the last time the assay was calibrated. The fluorescence signal in the tubes containing standards and the samples is stable for at least 3 hours when stored at room temperature. Therefore, it is recommended that a new calibration be performed each time a new working solution is prepared.

Calculating the Sample Concentration: The QUBIT 2.0 Fluorometer gives values for the MyQubit miRNA Assay in ng/mL. This value corresponds to the concentration after the sample is diluted into the assay tube. The following equation can be used to calculate the concentration of the starting sample:

$$\text{Concentration of sample} = \text{QF value} \times (200/x),$$

where QF value is the value given by the fluorometer, and x is the volume of sample in microliters added to the assay tube.

Dilution Calculator: The Dilution Calculator feature of the QUBIT 2.0 Fluorometer calculates the concentration of the original sample based on the volume of sample in the assay tube. To have the fluorometer perform this calculation, press **Calculate Stock Concentration** after a sample measurement is completed. Using the volume scroll wheel, select the volume of the original sample that is added to the assay tube. The fluorometer calculates the original sample concentration based on the measured assay concentration.

To save the data from the calculation to the fluorometer, press **Save** on the Dilution Calculator screen. The last calculated value of the measurement is saved to the instrument with a date and time stamp.

The Dilution Calculator generates a concentration for the original sample with the same units as the value in the assay tube given by the fluorometer. The units displayed in this calculation cannot be changed on the instrument for assays accessed using MyQubit.

Experimental Procedure:

Buffers and reagents are prepared as follows:

**2.1** Allow all reagents to come to room temperature.

**2.2** The binding dye comes as a 200× concentrate in DMSO. The stock solution is ready-to-use. No intermediate dilutions are required.

**2.3** The buffer used to prepare standard dilutions is 1× TE Buffer (10 mM Tris, 1 mM EDTA, pH 7.5), which can be prepared by diluting 20× TE Buffer (Cat. no. T11493) in E-pure water. This buffer solution also acts as Standard #1 in the calibration of the miRNA assay. 1 mL solution of 1× TE Buffer provides enough material for 100 assay calibrations.

**2.4** Prepare 1× TE Buffer + 0.01% CHAPS (w/v), which serves as the assay buffer. All dye working solutions should be prepared in TE Buffer + 0.01% CHAPS (w/v). For convenience, one can prepare an aqueous stock solution of 1% CHAPS (w/v) and dilute to 0.01% (w/v) accordingly. A solution of 200 mL buffer (20 ng of CHAPS in 200 mL of 1× TE buffer) is sufficient for 1000 assays, following the standard MyQubit miRNA Assay protocol described below. In the presence of CHAPS, dye working solution is stable for at least 3 hours at room temperature.

**2.5** Prepare a 100 ng/μL SILENCER Select siRNA solution by adding 650 μL of 1× TE Buffer only (no CHAPS) to the SILENCER Select siRNA (5 nmol, or 65 μg).

**2.6** Prepare a 10 ng/μL SILENCER Select siRNA stock solution by diluting the 100 ng/μL siRNA solution 10-fold in 1× TE Buffer only (no CHAPS) (add 100 μL of 100 ng/μL siRNA solution to 900 μL of 1× TE Buffer). This solution is Standard #2 in the calibration of the QUBIT miRNA Assay. 1 mL of the siRNA stock solution provides enough material for 100 assay calibrations.

Performing the MyQubit miRNA Assay

The protocol below describes the MyQubit miRNA Assay in a total volume of 200 μL per assay tube (see Table 2). The final concentrations of assay standards and samples are adjusted for the additional volume based on the dilution scheme outlined in the protocol below.

**3.1** Set up two assay tubes for the standards and one for each user sample. Use only thin-walled, clear 0.5 mL PCR tubes. Acceptable tubes include QUBIT assay tubes (set of 500, Cat. no. Q32856) or AXYGEN PCR-05-C tubes (VWR, part no. 10011-830).

**3.2** Prepare 200 μL of dye working solution for each standard and sample by diluting the ssDNA reagent 1:200 in IX TE Buffer + 0.01% CHAPS (w/v). For example, if assaying 5 samples, prepare 1 mL of dye working solution for the samples and 400 μL for the two standards, for a total of 1.4 mL of dye working solution.

**3.3** Prepare calibration standards by combining 10 μL of Standard #1 (1× TE Buffer only) with 190 μL of dye working solution, and 10 μL Standard #2 (10 ng/μL of SILENCER Select siRNA, from Step 2.6 above) with 190 μL of dye working solution.

**3.4** Prepare samples by combining 1-20 μL of sample with 180-199 μL of dye working solution.

**Table 2:** Volume of reagents used in the assay

| | **Standard Assay Tube** | **User Sample Assay Tube** |
|---|---|---|
| Volume of Dye **Working Solution** (from Step 32) | 190 μL | 180-199 μL |
| Volume of **Standard** | 10 μL | - |
| Volume of **User Sample** | - | 1-20 μL |
| **Total volume** in each Assay Tube | 200 μL | 200 μL |

**3.5** Vortex all of the tubes for 2-3 seconds and incubate at room temperature for 2 minutes.

**3.6** Calibrate the assay and read samples by selecting "miRNA" from the Home Screen and following the on-screen instructions.

***3.7*** *Optional:* Using the Dilution Calculator function of the QUBIT 2.0 Fluorometer, determine the stock concentration of the original sample.

EXAMPLE 2: miRNA and RNA Assays - Selectivity

rRNA (Life Technologies cat. #Q32852 component D, diluted in 10 mM Tris pH 7.5, 1 mM EDTA) at the concentrations listed in Figure 3, was spiked into 2 ng/μL siRNA (Life Technologies cat. #4390849, diluted in 10 mM Tris pH 7.5, 1 mM EDTA) and then read in the MyQubit miRNA and QUBIT RNA assays, as described in Example 1 (Life Technologies Corporation), as well as on the NANODROP Spectrophotometer (ThermoScientific, Waltham, MA) at an absorbance of 260 nm. Optical density values from the NANODROP instrument were converted to ng/μL using the following conversion factors: ssRNA: 1 A260 = 40 ng/μL; dsRNA: 1 A260 = 45 ng/μL. Eight replicates were used for all three methods.

The MyQubit miRNA assay read 102-196% of the siRNA even in the presence of 5-fold (500%) more rRNA than siRNA. The QUBIT RNA assay read 103-146% of the rRNA concentrations, even in the presence of 4-fold (400%) more siRNA than rRNA. The siRNA and rRNA concentrations read in the NANODROP Spectrophotometer were at the low-end of its detection limit. (see Figure 3).

EXAMPLE 3: miRNA Assays - Detection of small ssRNA and dsRNA

Single-stranded RNA's (ssRNA) were read at their initial concentration on the NANODROP Spectrophotometer and then diluted 1:50 for the MyQubit miRNA Assay. Double-stranded RNA's (dsRNA) were diluted 1:10, read on the NANODROP Spectrophotometer and then were further diluted 1:50 for the MyQubit miRNA Assay. The MyQubit miRNA assays were carried out as described in Example 1. The conversion formulae used for converting the absorbance at 260 nm to nucleic acid concentration using the NANODROP Spectrophotometer were as follows: ssRNA: 1 $A_{260}$ = 40 ng/μL; dsRNA: 1 $A_{260}$ = 45 ng/μL. All miRNA information can be found at www.mirbase.org. The miRNAs used in this assay were hsa-let-7a which has a length of 22 nucleotides, and hsa-miR-23a, which has a length of 21 nucleotides. The estimated concentrations of the ssRNA and dsRNA are shown in Figure 4, comparing the concentrations derived from the MyQubit miRNA Assay (black bars), the NANODROP Spectrophotometer (light gray bars), and the value that was written on each tube (dark gray bars). Tubes of RNA were obtained from Sasha Vlassov at AMBION (division of

Life Technologies) in Austin, TX.

EXAMPLE 4: Concentration of RNA Extracted from U2OS Cells using the MyQubit miRNA Assay and QUBIT RNA Assay

RNA was extracted from three different aliquots of U2OS cells, 2,400,000 cells in each aliquot, and processed according to the manufacturer's protocols in the AMBION MIRVANA kit (Life Technologies Corp.), and the QIAGEN MIRNEASY kit (QIAGEN, Netherlands). Stock concentrations were determined in triplicate readings on the NANODROP Spectrophotometer at 260 nm, using 1 $A_{260}$ = 40 ng/μL. Stock RNAs were diluted 1:100 and the concentrations were determined in triplicate in the MyQubit miRNA and QUBIT RNA Assays. Figure 5 shows the yield of RNA as determined by the MyQubit miRNA Assay (black bars), QUBIT RNA Assay (light gray bars), and the NANODROP Spectrophotometer (dark gray bars).

The concentrations determined by the MyQubit miRNA Assay were about 1/3 of the concentrations determined by the NANODROP Spectrophotometer, suggesting that the MyQubit miRNA Assay is less specific for larger RNA, such as rRNA (see Figure 3).

EXAMPLE 5: Detection of mRNA in the MyQubit miRNA Assay

Two mRNAs (EGFP mRNA, TriLink Biotechnologies cat. #L-6101, 996 nt long, and Fluc mRNA, TriLink Biotechnologies cat. #L-6107, 1929 nt long) were tested in the MyQubit miRNA and QUBIT RNA Assays. The mRNAs were diluted in 1× TE buffer, pH 7.5 to a final concentration of 15 ng/μL, and the concentrations were measured by absorbance at 258 nm on a NANODROP Spectrophotometer using 1 $A_{258}$ = 40 ng/μL. The 15 ng/μL solutions were further diluted to 0.1, 0.5, 2, 4, 8 and 10 ng/μL. For each assay, 50 μL of each dilution was aliquotted and 950 μL of working solution was added. 200 μL of each of the resulting samples was aliquotted to each of five MyQubit assay tubes and read in the respective assay (5 replicates).

The results of each assay are shown as follows: Figure 6A and 6B: The MyQubit miRNA Assay detected about 15% of each mRNA; Figures 6C and 6D: The QUBIT RNA Assay detected 90-150% of each mRNA.

EXAMPLE 6: Detection of Nucleotides in the MyQubit miRNA Assay

Pure single NTPs (ATP, CTP, GTP and UTP) were diluted 1× in TE buffer, pH 7.5 to a final concentration of 10 ng/μL. The concentration of each dilution was confirmed by $A_{260}$. 10 μL of each diluted NTP was aliquotted into QUBIT Assay tubes in triplicate for analysis in MyQubit miRNA and QUBIT RNA Assays. The four nucleotide solutions were also mixed in equal proportions and 10 μL of the NTP mixture was aliquotted in triplicate into QUBIT Assay tubes for analysis in MyQubit miRNA and QUBIT RNA Assays. 190 μL of miRNA or RNA Assay Working Solution was added to each sample and then read in the respective QUBIT Assay.

The signal for all of the single NTP and mixed NTP samples was very close to background, indicating that nucleotides were not detected by either assay (see Tables 3 and 4).

**Table 3:** MyQubit miRNA Assay

| Name | Expected conc. (in assay tube) | Assay Conc. | Units |
|---|---|---|---|
| std 2 | 500 ng/mL | 500 | ng/mL |
| std 2* | 500 ng/mL | 497 | ng/mL |
| std 1 | 0 | < 2.5 | ng/mL |
| std 1* | 0 | < 2.5 | ng/mL |
| mixed NT's | 500 ng/mL | < 2.5 | ng/mL |
| mixed NT's | 500 ng/mL | < 2.5 | ng/mL |
| mixed NT's | 500 ng/mL | < 2.5 | ng/mL |
| UTP | 500 ng/mL | < 2.5 | ng/mL |
| UTP | 500 ng/mL | < 2.5 | ng/mL |
| UTP | 500 ng/mL | < 2.5 | ng/mL |
| GTP | 500 ng/mL | < 2.5 | ng/mL |
| GTP | 500 ng/mL | < 2.5 | ng/mL |

(continued)

| Name | Expected conc. (in assay tube) | Assay Conc. | Units |
|---|---|---|---|
| GTP | 500 ng/mL | < 2.5 | ng/mL |
| CTP | 500 ng/mL | < 2.5 | ng/mL |
| CTP | 500 ng/mL | < 2.5 | ng/mL |
| CTP | 500 ng/mL | < 2.5 | ng/mL |
| ATP | 500 ng/mL | < 2.5 | ng/mL |
| ATP | 500 ng/mL | < 2.5 | ng/mL |
| ATP | 500 ng/mL | < 2.5 | ng/mL |

**Table 4:** QUBIT RNA Assay

| Name | Expected conc. (in assay tube) | Assay Conc. | Units |
|---|---|---|---|
| std 2 | 500 ng/mL | 500 | ng/ml |
| std 2* | 500 ng/mL | 494 | ng/ml |
| std 1 | 0 | <20 | ng/ml |
| std 1* | 0 | <20 | ng/ml |
| mixed NT's | 500 ng/mL | <20 | ng/ml |
| mixed NT's | 500 ng/mL | <20 | ng/ml |
| mixed NT's | 500 ng/mL | <20 | ng/ml |
| UTP | 500 ng/mL | <20 | ng/ml |
| UTP | 500 ng/mL | <20 | ng/ml |
| UTP | 500 ng/mL | <20 | ng/ml |
| GTP | 500 ng/mL | <20 | ng/ml |
| GTP | 500 ng/mL | <20 | ng/ml |
| GTP | 500 ng/mL | <20 | ng/ml |
| CTP | 500 ng/mL | <20 | ng/ml |
| CTP | 500 ng/mL | <20 | ng/ml |
| CTP | 500 ng/mL | <20 | ng/ml |
| ATP | 500 ng/mL | <20 | ng/ml |
| ATP | 500 ng/mL | <20 | ng/ml |
| ATP | 500 ng/mL | <20 | ng/ml |

EXAMPLE 7: Analysis of Dye Compounds of the Present Teachings in the MyQubit miRNA Assay

Several of the dye compounds disclosed herein were analyzed for fluorescence enhancement when bound to pure siRNA in the MyQubit miRNA Assay described herein. 58 dye compounds (see Table 5) plus the dye compound of Formula **II** and a control dye were tested in triplicate. 190 μL of 1 μM dye compound in 1× Tris-EDTA, 0.01 % CHAPS was added to 10 μL of 4 different concentrations of siRNA to GAPDH (Ambion cat. #4390849, lot ASO0THWA) in wells of a 96-well Costar black, clear bottom microplate. Plates were read on the SpectraMax plate reader in J2 west at 495 nm excitation and 525 nm emission. Figures 7A-H show the fluorescence signal for each of the dyes tested (Figure 7A = dye compounds 4-10, Figure 7B = dye compounds 11-17, Figure 7C = dye compounds 18-27, Figure 7D = dye compounds 30-37, Figure 7E = dye compounds 38-44, Figure 7F = dye compounds 45-52, Figure 7G = dye compounds 53-60, Figure 7H = dye compounds 61-65). All of the dye compounds tested had some fluorescence enhancement in

the presence of siRNA, except dye compound 62. Dye compounds that had a fluorescence enhancement signal that was >30% greater than that of the dye compound of Formula **II** at 500 ng/mL siRNA were dye compounds 7, 13, 14, 19, 20, 39, 40, 41, 46, 47, 53, 54, and 55. Dye compounds 61-65 had a low fluorescence signal.

**Table 5:** Dye compounds of Formula **III** tested in MyQubit miRNA Assay

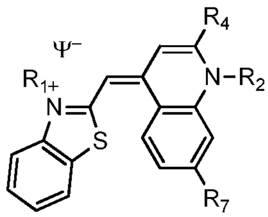

(Formula **III**)

| Compound | $R_1$ | $R_2$ | $R_4$ | $R_7$ |
|---|---|---|---|---|
| 4 | $-CH_3$ | phenyl | 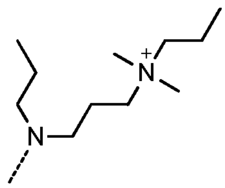 | H |
| 5 | $-CH_3$ | phenyl | 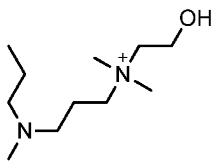 | H |
| 6 | $-CH_3$ | phenyl | 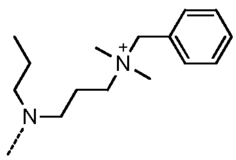 | H |
| 7 | $-CH_3$ | phenyl | 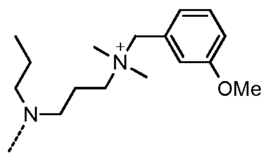 | H |
| 8 | $-CH_3$ | phenyl | 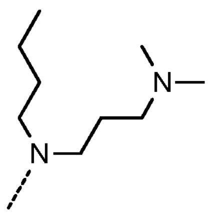 | H |
| 9 | $-CH_3$ | phenyl | 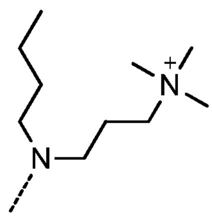 | H |

(continued)

| Compound | $R_1$ | $R_2$ | $R_4$ | $R_7$ |
|---|---|---|---|---|
| 10 | $-CH_3$ | phenyl | 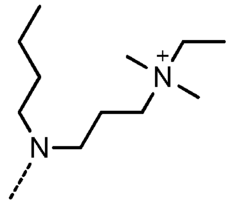 | H |
| 11 | $-CH_3$ | phenyl | 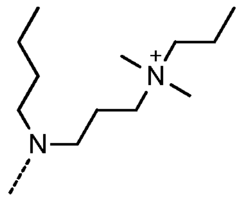 | H |
| 12 | $-CH_3$ | phenyl | 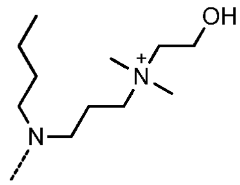 | H |
| 13 | $-CH_3$ | phenyl | 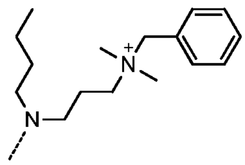 | H |
| 14 | $-CH_3$ | phenyl | 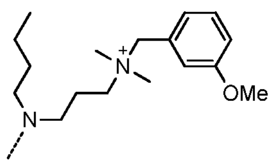 | H |
| 15 | $-CH_3$ | phenyl | 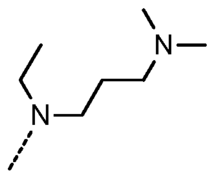 | H |
| 16 | $-CH_3$ | phenyl | 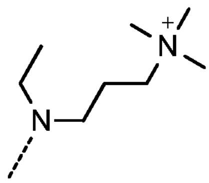 | H |
| 17 | $-CH_3$ | phenyl | 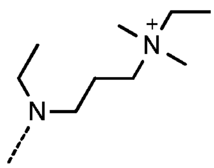 | H |

(continued)

| Compound | $R_1$ | $R_2$ | $R_4$ | $R_7$ |
|---|---|---|---|---|
| 18 | $-CH_3$ | phenyl | 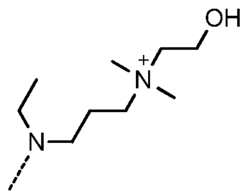 | H |
| 19 | $-CH_3$ | phenyl | 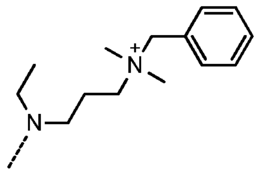 | H |
| 20 | $-CH_3$ | phenyl | 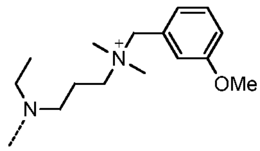 | H |
| 21 | $-CH_3$ | phenyl | 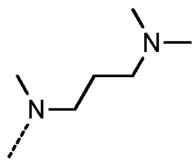 | H |
| 23 | $-CH_3$ | phenyl | 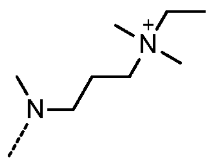 | H |
| 25 | $-CH_3$ | phenyl | 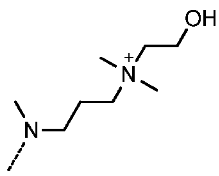 | H |
| 26 | $-CH_3$ | phenyl | 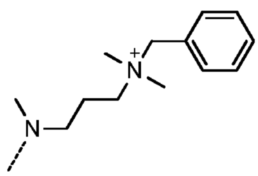 | H |
| 27 | $-CH_3$ | phenyl | 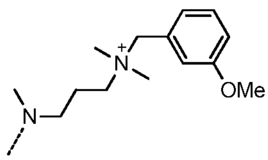 | H |
| 30 | $-CH_3$ | phenyl | 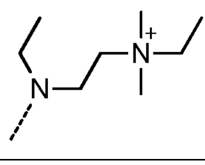 | H |

(continued)

| Compound | $R_1$ | $R_2$ | $R_4$ | $R_7$ |
|---|---|---|---|---|
| 31 | $-CH_3$ | phenyl | 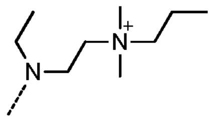 | H |
| 32 | $-CH_3$ | phenyl | 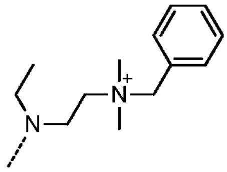 | H |
| 33 | $-CH_3$ | phenyl | 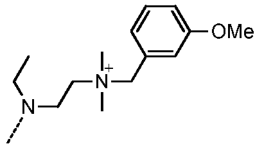 | H |
| 34 | $-CH_3$ | phenyl | 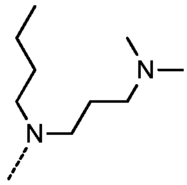 | $-OCH_3$ |
| 35 | $-CH_3$ | phenyl | 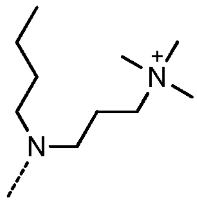 | $-OCH_3$ |
| 36 | $-CH_3$ | phenyl | 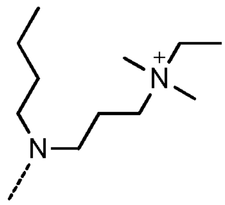 | $-OCH_3$ |
| 37 | $-CH_3$ | phenyl | 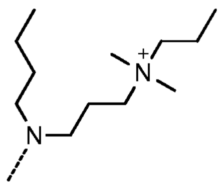 | $-OCH_3$ |
| 38 | $-CH_3$ | phenyl | 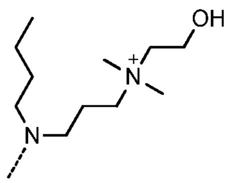 | $-OCH_3$ |

(continued)

| Compound | $R_1$ | $R_2$ | $R_4$ | $R_7$ |
|---|---|---|---|---|
| 39 | $-CH_3$ | phenyl | 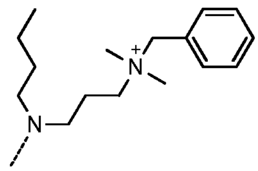 | $-OCH_3$ |
| 40 | $-CH_3$ | phenyl | 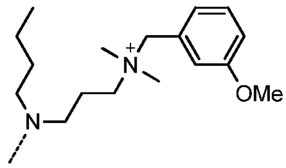 | $-OCH_3$ |
| 41 | $-CH_3$ | phenyl | 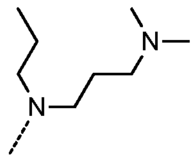 | $-OCH_3$ |
| 42 | $-CH_3$ | phenyl | 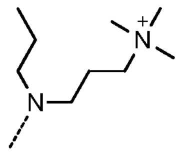 | $-OCH_3$ |
| 43 | $-CH_3$ | phenyl | 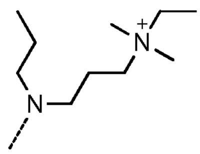 | $-OCH_3$ |
| 44 | $-CH_3$ | phenyl | 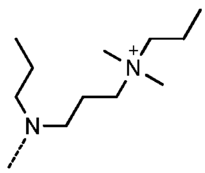 | $-OCH_3$ |
| 45 | $-CH_3$ | phenyl | 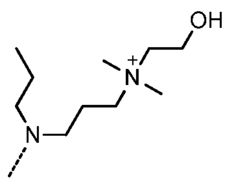 | $-OCH_3$ |
| 46 | $-CH_3$ | phenyl | 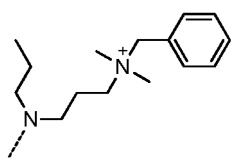 | $-OCH_3$ |
| 47 | $-CH_3$ | phenyl | 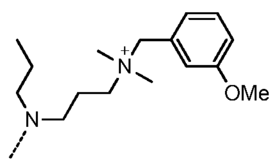 | $-OCH_3$ |

(continued)

| Compound | $R_1$ | $R_2$ | $R_4$ | $R_7$ |
|---|---|---|---|---|
| 48 | $-CH_3$ | phenyl | 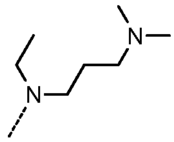 | $-OCH_3$ |
| 49 | $-CH_3$ | phenyl | 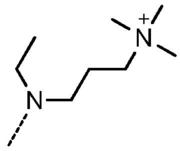 | $-OCH_3$ |
| 50 | $-CH_3$ | phenyl | 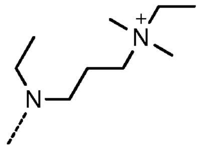 | $-OCH_3$ |
| 51 | $-CH_3$ | phenyl | 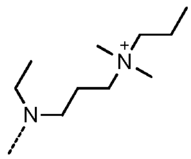 | $-OCH_3$ |
| 52 | $-CH_3$ | phenyl | 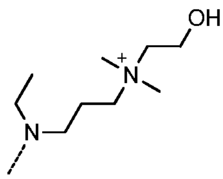 | $-OCH_3$ |
| 53 | $-CH_3$ | phenyl | 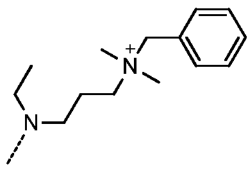 | $-OCH_3$ |
| 54 | $-CH_3$ | phenyl | 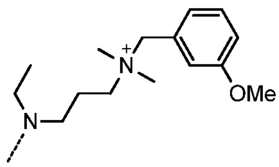 | $-OCH_3$ |
| 55 | $-CH_3$ | phenyl | 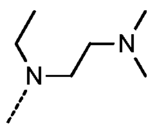 | $-OCH_3$ |
| 56 | $-CH_3$ | phenyl | 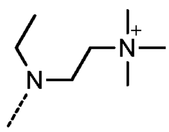 | $-OCH_3$ |

(continued)

| Compound | $R_1$ | $R_2$ | $R_4$ | $R_7$ |
|---|---|---|---|---|
| 57 | $-CH_3$ | phenyl | 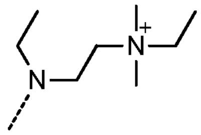 | $-OCH_3$ |
| 58 | $-CH_3$ | phenyl | 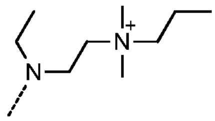 | $-OCH_3$ |
| 59 | $-CH_3$ | phenyl | 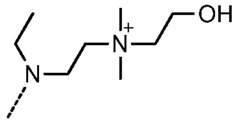 | $-OCH_3$ |
| 60 | $-CH_3$ | phenyl | 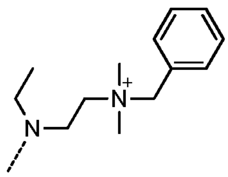 | $-OCH_3$ |
| 61 | $-CH_3$ | phenyl | 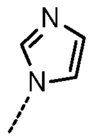 | H |
| 62 | $-CH_3$ | phenyl | 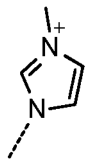 | H |
| 63 | $-CH_3$ | phenyl | 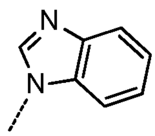 | H |
| 64 | $-CH_3$ | phenyl | $-CH_2CH_2CH_2N(CH_3)_2$ | H |
| 65 | $-CH_3$ | phenyl | $-CH_2CH_2CH_2N^+(CH_3)_3$ | H |

## Claims

**1.** A method of detecting in a sample single-stranded RNA (ssRNA) having 25 or fewer nucleotides, comprising:

(a) combining a sample suspected of containing ssRNA, wherein the ssRNA comprises 25 or fewer nucleotides, with a substituted, unsymmetrical cyanine dye compound of Formula II:

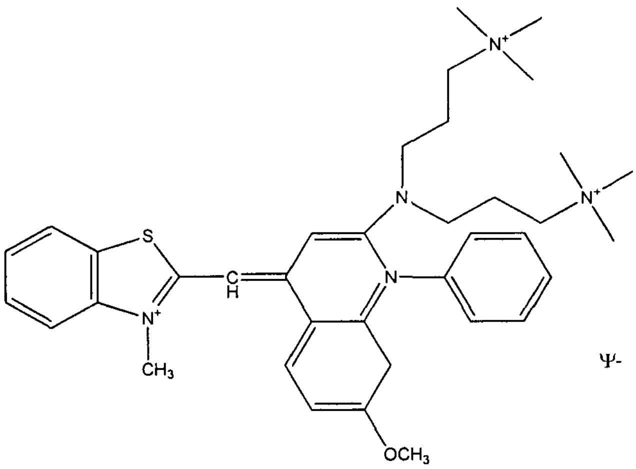

(II)

or

a dye compound selected from the group consisting of Compound 4, Compound 5, Compound 6, Compound 7, Compound 8, Compound 9, Compound 10, Compound 11, Compound 12, Compound 13, Compound 14, Compound 15, Compound 16, Compound 17, Compound 18, Compound 19, Compound 20, Compound 21, Compound 23, Compound 25, Compound 26, Compound 27, Compound 30, Compound 31, Compound 32, Compound 33, Compound 34, Compound 35, Compound 36, Compound 37, Compound 38, Compound 39, Compound 40, Compound 41, Compound 42, Compound 43, Compound 44, Compound 45, Compound 46, Compound 47, Compound 48, Compound 49, Compound 50, Compound 51, Compound 52, Compound 53, Compound 54, Compound 55, Compound 56, Compound 57, Compound 58, Compound 59 and Compound 60 having the following formula:

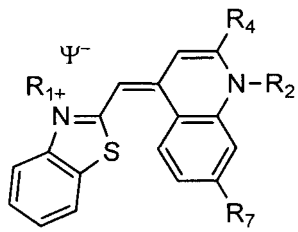

| Compound | $R_1$ | $R_2$ | $R_4$ | $R_7$ |
|---|---|---|---|---|
| 4 | $-CH_3$ | phenyl | 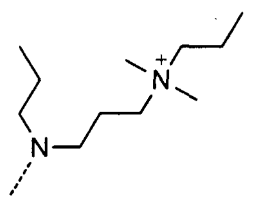 | H |
| 5 | $-CH_3$ | phenyl | 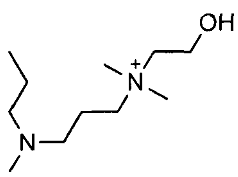 | H |

(continued)

| Compound | $R_1$ | $R_2$ | $R_4$ | $R_7$ |
|---|---|---|---|---|
| 6 | $-CH_3$ | phenyl | 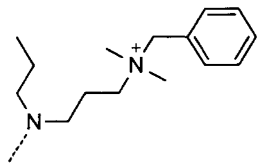 | H |
| 7 | $-CH_3$ | phenyl | 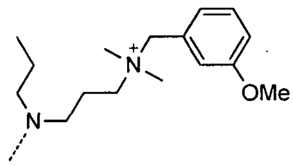 | H |
| 8 | $-CH_3$ | phenyl | 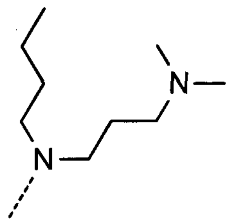 | H |
| 9 | $-CH_3$ | phenyl | 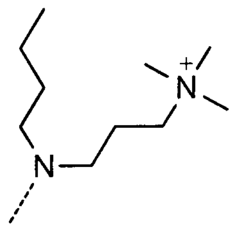 | H |
| 10 | $-CH_3$ | phenyl | 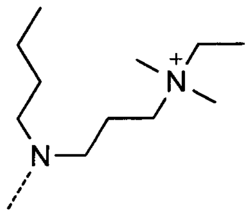 | H |
| 11 | $-CH_3$ | phenyl | 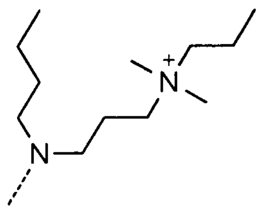 | H |
| 12 | $-CH_3$ | phenyl | 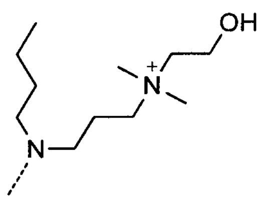 | H |

(continued)

| Compound | $R_1$ | $R_2$ | $R_4$ | $R_7$ |
|---|---|---|---|---|
| 13 | $-CH_3$ | phenyl | 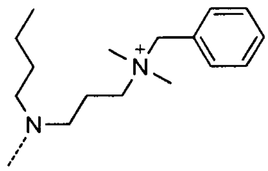 | H |
| 14 | $-CH_3$ | phenyl | 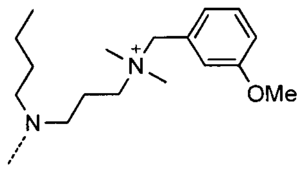 | H |
| 15 | $-CH_3$ | phenyl | 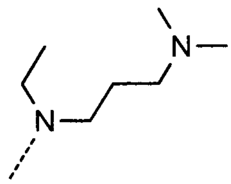 | H |
| 16 | $-CH_3$ | phenyl | 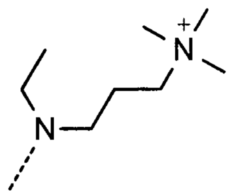 | H |
| 17 | $-CH_3$ | phenyl | 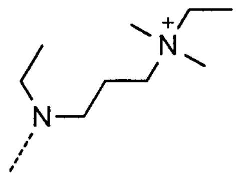 | H |
| 18 | $-CH_3$ | phenyl | 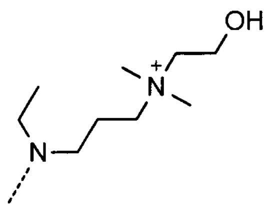 | H |
| 19 | $-CH_3$ | phenyl | 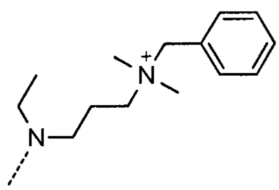 | H |
| 20 | $-CH_3$ | phenyl | 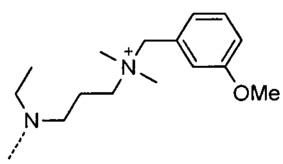 | H |

(continued)

| Compound | $R_1$ | $R_2$ | $R_4$ | $R_7$ |
|---|---|---|---|---|
| 21 | $-CH_3$ | phenyl | 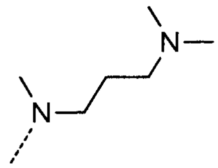 | H |
| 23 | $-CH_3$ | phenyl | 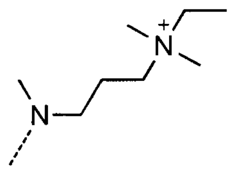 | H |
| 25 | $-CH_3$ | phenyl | 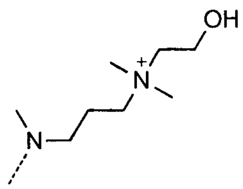 | H |
| 26 | $-CH_3$ | phenyl | 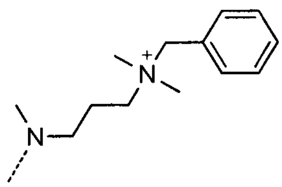 | H |
| 27 | $-CH_3$ | phenyl | 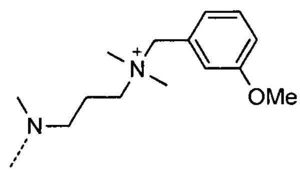 | H |
| 30 | $-CH_3$ | phenyl | 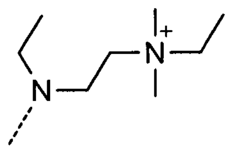 | H |
| 31 | $-CH_3$ | phenyl | 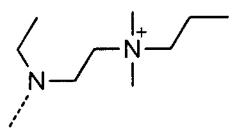 | H |
| 32 | $-CH_3$ | phenyl | 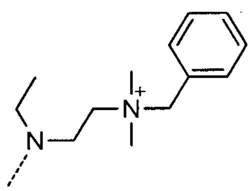 | H |
| 33 | $-CH_3$ | phenyl | 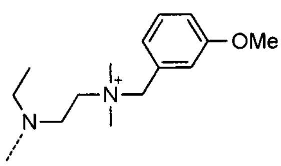 | H |

(continued)

| Compound | $R_1$ | $R_2$ | $R_4$ | $R_7$ |
|---|---|---|---|---|
| 34 | $-CH_3$ | phenyl | 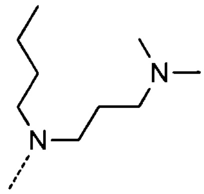 | $-OCH_3$ |
| 35 | $-CH_3$ | phenyl | 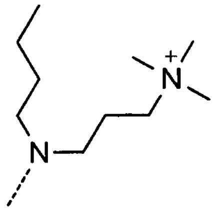 | $-OCH_3$ |
| 36 | $-CH_3$ | phenyl | 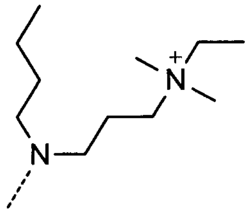 | $-OCH_3$ |
| 37 | $-CH_3$ | phenyl | 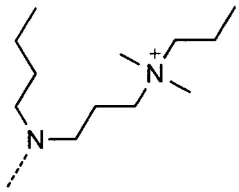 | $-OCH_3$ |
| 38 | $-CH_3$ | phenyl | 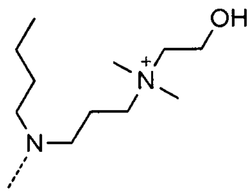 | $-OCH_3$ |
| 39 | $-CH_3$ | phenyl | 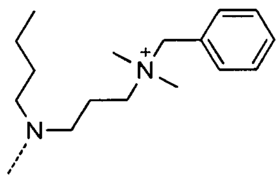 | $-OCH_3$ |
| 40 | $-CH_3$ | phenyl | 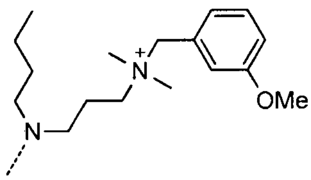 | $-OCH_3$ |
| 41 | $-CH_3$ | phenyl | 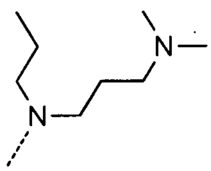 | $-OCH_3$ |

(continued)

| Compound | $R_1$ | $R_2$ | $R_4$ | $R_7$ |
|---|---|---|---|---|
| 42 | $-CH_3$ | phenyl | 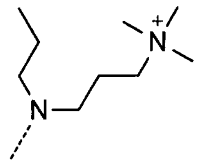 | $-OCH_3$ |
| 43 | $-CH_3$ | phenyl | 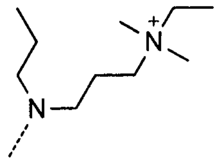 | $-OCH_3$ |
| 44 | $-CH_3$ | phenyl | 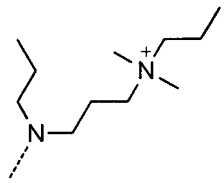 | $-OCH_3$ |
| 45 | $-CH_3$ | phenyl | 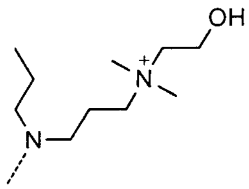 | $-OCH_3$ |
| 46 | $-CH_3$ | phenyl | 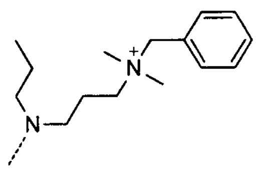 | $-OCH_3$ |
| 47 | $-CH_3$ | phenyl | 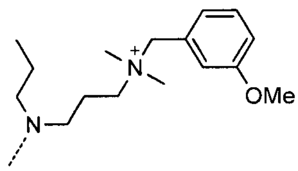 | $-OCH_3$ |
| 48 | $-CH_3$ | phenyl | 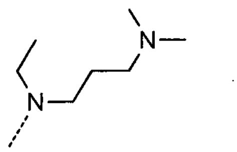 | $-OCH_3$ |
| 49 | $-CH_3$ | phenyl | 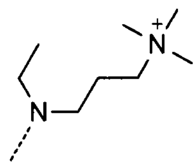 | $-OCH_3$ |

(continued)

| Compound | $R_1$ | $R_2$ | $R_4$ | $R_7$ |
|---|---|---|---|---|
| 50 | $-CH_3$ | phenyl | 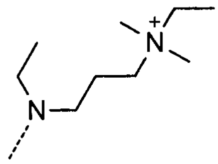 | $-OCH_3$ |
| 51 | $-CH_3$ | phenyl | 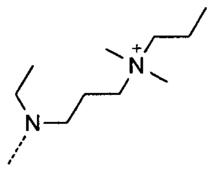 | $-OCH_3$ |
| 52 | $-CH_3$ | phenyl | 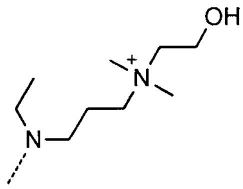 | $-OCH_3$ |
| 53 | $-CH_3$ | phenyl | 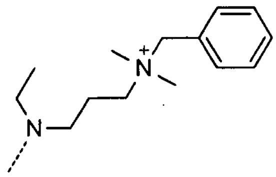 | $-OCH_3$ |
| 54 | $-CH_3$ | phenyl | 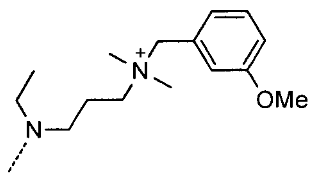 | $-OCH_3$ |
| 55 | $-CH_3$ | phenyl | 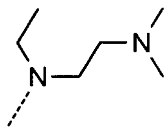 | $-OCH_3$ |
| 56 | $-CH_3$ | phenyl | 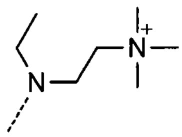 | $-OCH_3$ |
| 57 | $-CH_3$ | phenyl | 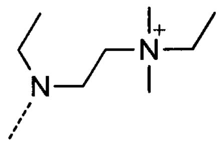 | $-OCH_3$ |
| 58 | $-CH_3$ | phenyl | 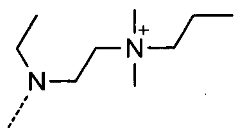 | $-OCH_3$ |

(continued)

| Compound | $R_1$ | $R_2$ | $R_4$ | $R_7$ |
|---|---|---|---|---|
| 59 | $-CH_3$ | phenyl | 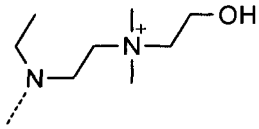 | $-OCH_3$ |
| 60 | $-CH_3$ | phenyl | 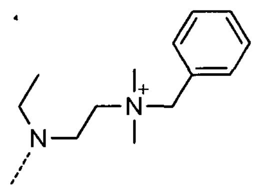 | $-OCH_3$ |

;

(b) incubating the sample for a time sufficient for the dye compound to combine with the ssRNA in the sample, if present, to form a dye-ssRNA complex that emits a detectable fluorescent signal; and

(c) detecting for the emission of the fluorescent signal, wherein the detectable fluorescent signal is indicative of the presence of a dye-ssRNA complex.

**2.** The method of claim 1, wherein the ssRNA comprises 20-25 nucleotides.

**3.** The method of claim 1, wherein the ssRNA comprises 10-20 nucleotides.

**4.** The method of claim 1, wherein the ssRNA is microRNA (miRNA).

**5.** The method of claim 1, wherein the ssRNA comprises a chemical modification to at least one sugar, phosphate, or base group.

**6.** The method of claim 1, wherein the ssRNA comprises a chemical group other than a hydroxyl or phosphate at its 5' termini.

**7.** The method of claim 1, wherein the ssRNA is microRNA (miRNA), small nuclear RNA (snRNA), small nucleolar RNA (snoRNA), tiny non-coding RNA (tncRNA), small modulatory RNA (smRNA), or piwi-interacting RNA (piRNA).

**8.** The method of claim 1, further comprising detecting ssRNA in a concentration of about 5 ng/mL to about 1000 ng/mL.

**9.** The method of claim 1, wherein the fluorescence signal is stable for at least 30 minutes at room temperature.

**10.** A dye compound selected from the group consisting of Compound 4, Compound 5, Compound 6, Compound 7, Compound 8, Compound 9, Compound 10, Compound 11, Compound 12, Compound 13, Compound 14, Compound 15, Compound 16, Compound 17, Compound 18, Compound 19, Compound 20, Compound 23, Compound 25, Compound 26, Compound 27, Compound 30, Compound 31, Compound 32, Compound 33, Compound 34, Compound 35, Compound 36, Compound 37, Compound 38, Compound 39, Compound 40, Compound 43, Compound 44, Compound 45, Compound 46, Compound 47, Compound 48, Compound 49, Compound 50, Compound 51, Compound 52, Compound 53, Compound 54, Compound 55, Compound 56, Compound 57, Compound 58, Compound 59, and Compound 60 having the following formula

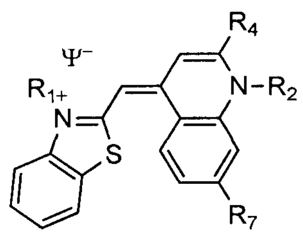

| Compound | $R_1$ | $R_2$ | $R_4$ | $R_7$ |
|---|---|---|---|---|
| 4 | $-CH_3$ | phenyl | 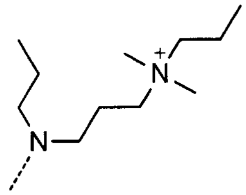 | H |
| 5 | $-CH_3$ | phenyl | 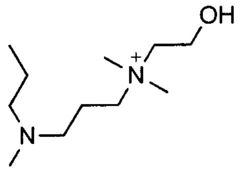 | H |
| 6 | $-CH_3$ | phenyl | 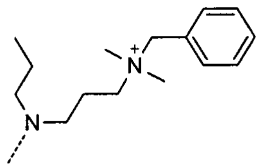 | H |
| 7 | $-CH_3$ | phenyl | 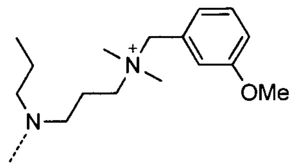 | H |
| 8 | $-CH_3$ | phenyl | 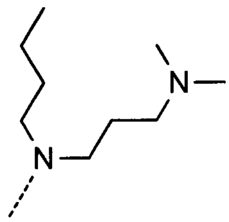 | H |
| 9 | $-CH_3$ | phenyl | 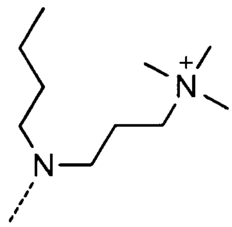 | H |
| 10 | $-CH_3$ | phenyl | 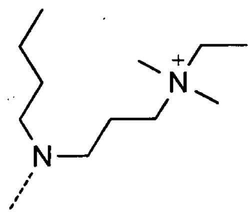 | H |

(continued)

| Compound | $R_1$ | $R_2$ | $R_4$ | $R_7$ |
|---|---|---|---|---|
| 11 | $-CH_3$ | phenyl | 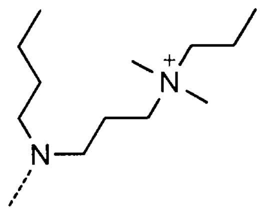 | H |
| 12 | $-CH_3$ | phenyl | 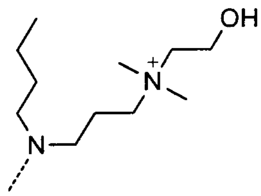 | H |
| 13 | $-CH_3$ | phenyl | 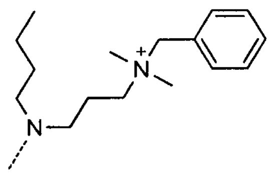 | H |
| 14 | $-CH_3$ | phenyl | 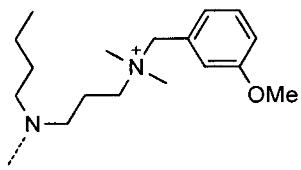 | H |
| 15 | $-CH_3$ | phenyl | 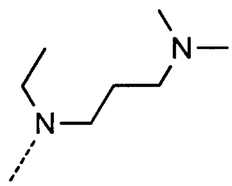 | H |
| 16 | $-CH_3$ | phenyl | 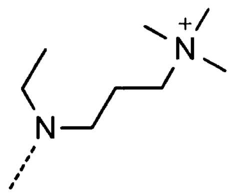 | H |
| 17 | $-CH_3$ | phenyl | 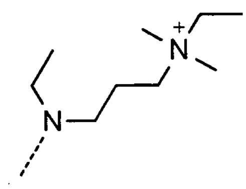 | H |
| 18 | $-CH_3$ | phenyl | 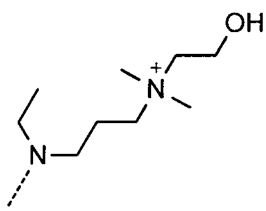 | H |

(continued)

| Compound | $R_1$ | $R_2$ | $R_4$ | $R_7$ |
|---|---|---|---|---|
| 19 | $-CH_3$ | phenyl | 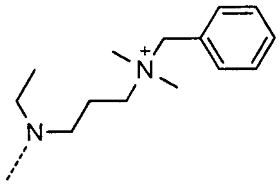 | H |
| 20 | $-CH_3$ | phenyl | 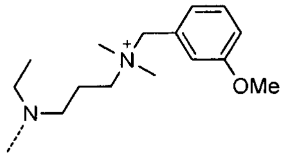 | H |
| 23 | $-CH_3$ | phenyl | 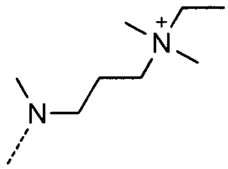 | H |
| 25 | $-CH_3$ | phenyl | 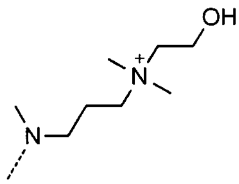 | H |
| 26 | $-CH_3$ | phenyl | 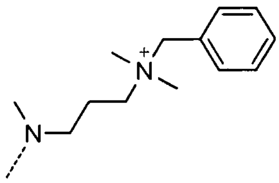 | H |
| 27 | $-CH_3$ | phenyl | 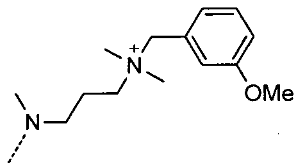 | H |
| 30 | $-CH_3$ | phenyl | 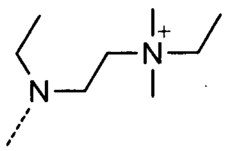 | H |
| 31 | $-CH_3$ | phenyl | 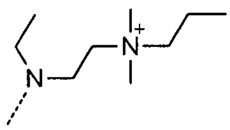 | H |

(continued)

| Compound | $R_1$ | $R_2$ | $R_4$ | $R_7$ |
|---|---|---|---|---|
| 32 | $-CH_3$ | phenyl | 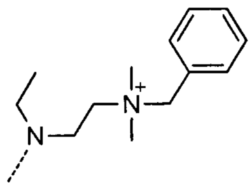 | H |
| 33 | $-CH_3$ | phenyl | 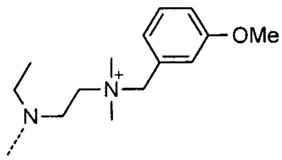 | H |
| 34 | $-CH_3$ | phenyl | 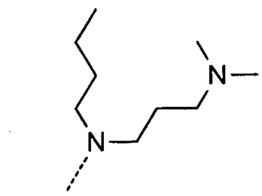 | $-OCH_3$ |
| 35 | $-CH_3$ | phenyl | 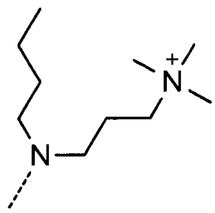 | $-OCH_3$ |
| 36 | $-CH_3$ | phenyl | 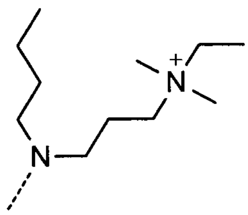 | $-OCH_3$ |
| 37 | $-CH_3$ | phenyl | 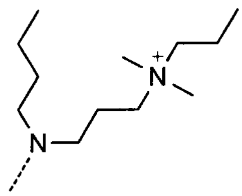 | $-OCH_3$ |
| 38 | $-CH_3$ | phenyl | 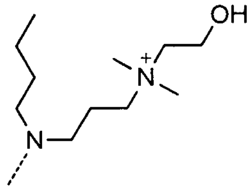 | $-OCH_3$ |

(continued)

| Compound | $R_1$ | $R_2$ | $R_4$ | $R_7$ |
|---|---|---|---|---|
| 39 | $-CH_3$ | phenyl | 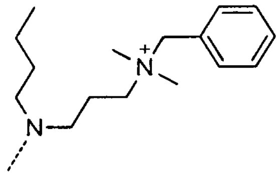 | $-OCH_3$ |
| 40 | $-CH_3$ | phenyl | 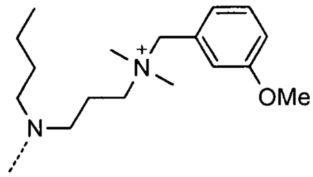 | $-OCH_3$ |
| 43 | $-CH_3$ | phenyl | 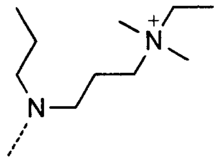 | $-OCH_3$ |
| 44 | $-CH_3$ | phenyl | 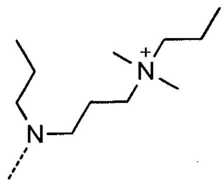 | $-OCH_3$ |
| 45 | $-CH_3$ | phenyl | 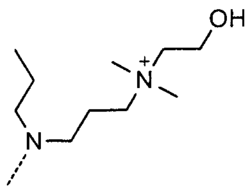 | $-OCH_3$ |
| 46 | $-CH_3$ | phenyl | 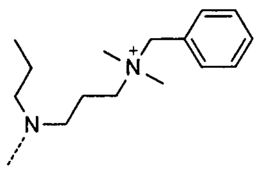 | $-OCH_3$ |
| 47 | $-CH_3$ | phenyl | 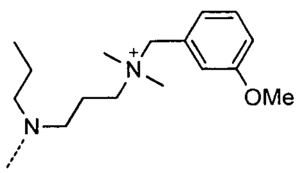 | $-OCH_3$ |
| 48 | $-CH_3$ | phenyl | 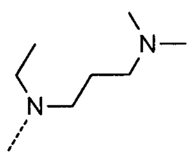 | $-OCH_3$ |

(continued)

| Compound | $R_1$ | $R_2$ | $R_4$ | $R_7$ |
|---|---|---|---|---|
| 49 | $-CH_3$ | phenyl | 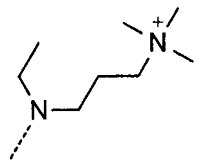 | $-OCH_3$ |
| 50 | $-CH_3$ | phenyl | 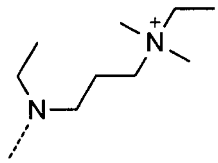 | $-OCH_3$ |
| 51 | $-CH_3$ | phenyl | 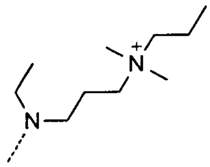 | $-OCH_3$ |
| 52 | $-CH_3$ | phenyl | 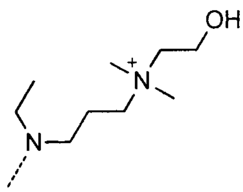 | $-OCH_3$ |
| 53 | $-CH_3$ | phenyl | 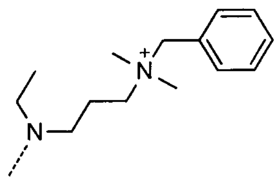 | $-OCH_3$ |
| 54 | $-CH_3$ | phenyl | 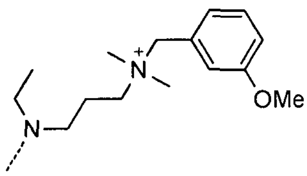 | $-OCH_3$ |
| 55 | $-CH_3$ | phenyl | 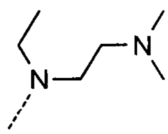 | $-OCH_3$ |
| 56 | $-CH_3$ | phenyl | 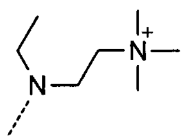 | $-OCH_3$ |
| 57 | $-CH_3$ | phenyl | 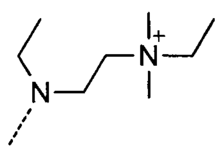 | $-OCH_3$ |

(continued)

| Compound | $R_1$ | $R_2$ | $R_4$ | $R_7$ |
|---|---|---|---|---|
| 58 | $-CH_3$ | phenyl | 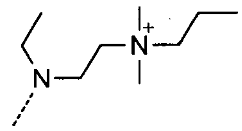 | $-OCH_3$ |
| 59 | $-CH_3$ | phenyl | 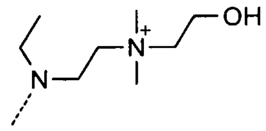 | $-OCH_3$ |
| 60 | $-CH_3$ | phenyl | 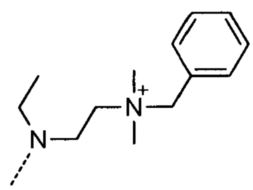 | $-OCH_3$ |

**11.** A kit for detecting the presence of single-stranded RNA (ssRNA) in a sample, comprising: the dye compound of claim 10, and instructions for using the dye in the method of claim 1.

**Patentansprüche**

**1.** Verfahren zum Nachweisen von einsträngiger RNA (ssRNA) mit 25 oder weniger Nukleotiden in einer Probe, Folgendes umfassend:

(a) Kombinieren einer Probe, von der angenommen wird, dass sie ssRNA enthält, wobei die ssRNA 25 oder weniger Nukleotide umfasst, mit einer substituierten unsymmetrischen Cyaninfarbstoffverbindung der Formel II:

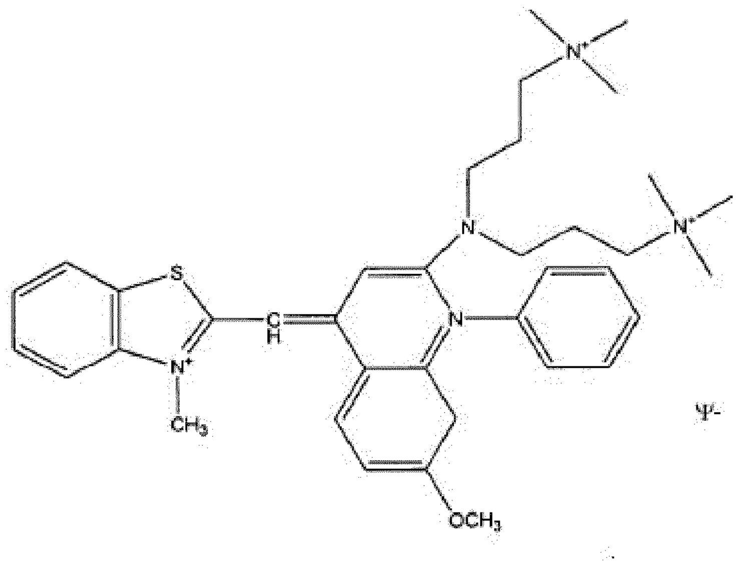

(II)

oder

einer Farbstoffverbindung, ausgewählt aus der Gruppe bestehend aus Verbindung 4, Verbindung 5, Verbindung 6, Verbindung 7, Verbindung 8, Verbindung 9, Verbindung 10, Verbindung 11, Verbindung 12, Verbindung 13, Verbindung 14, Verbindung 15, Verbindung 16, Verbindung 17, Verbindung 18, Verbindung 19, Verbindung 20, Verbindung 21, Verbindung 23, Verbindung 25, Verbindung 26, Verbindung 27, Verbindung 30, Verbindung 31, Verbindung 32, Verbindung 33, Verbindung 34, Verbindung 35, Verbindung 36, Verbindung 37, Verbindung

38, Verbindung 39, Verbindung 40, Verbindung 41, Verbindung 42, Verbindung 43, Verbindung 44, Verbindung 45, Verbindung 46, Verbindung 47, Verbindung 48, Verbindung 49, Verbindung 50, Verbindung 51, Verbindung 52, Verbindung 53, Verbindung 54, Verbindung 55, Verbindung 56, Verbindung 57, Verbindung 58, Verbindung 59 und Verbindung 60 mit der folgenden Formel:

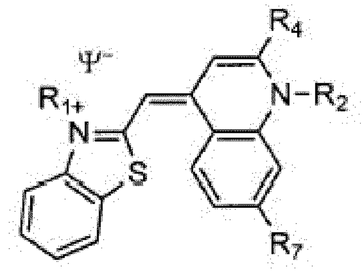

| Verbindung | $R_1$ | $R_2$ | $R_4$ | $R_7$ |
|---|---|---|---|---|
| 4 | $-CH_3$ | Phenyl | 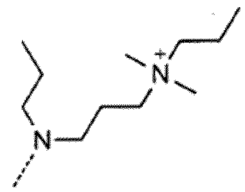 | H |
| 5 | $-CH_3$ | Phenyl | 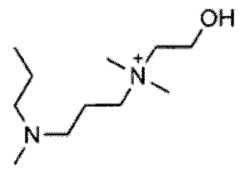 | H |
| 6 | $-CH_3$ | Phenyl | 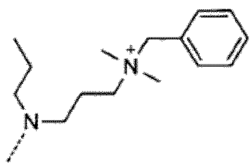 | H |
| 7 | $-CH_3$ | Phenyl | 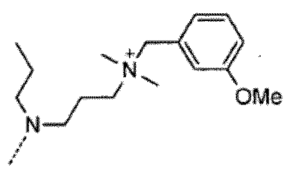 | H |
| 8 | $-CH_3$ | Phenyl | 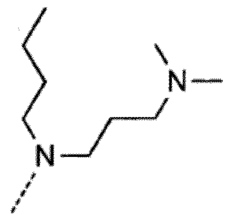 | H |

(fortgesetzt)

| Verbindung | $R_1$ | $R_2$ | $R_4$ | $R_7$ |
|---|---|---|---|---|
| 9 | $-CH_3$ | Phenyl | 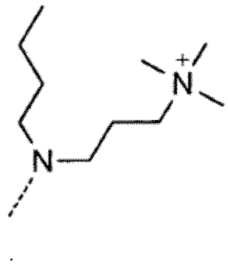 | H |
| 10 | $-CH_3$ | Phenyl | 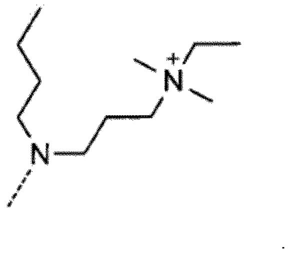 | H |
| 11 | $-CH_3$ | Phenyl | 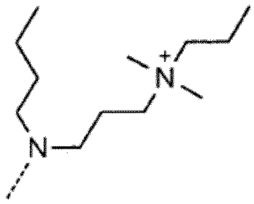 | H |
| 12 | $-CH_3$ | Phenyl | 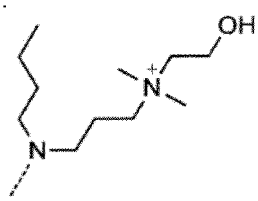 | H |
| 13 | $-CH_3$ | Phenyl | 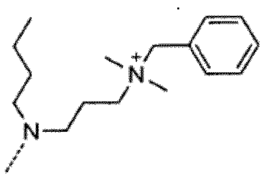 | H |
| 14 | $-CH_3$ | Phenyl | 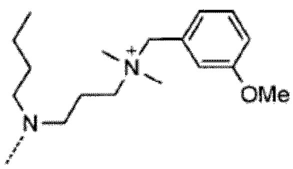 | H |
| 15 | $-CH_3$ | Phenyl | 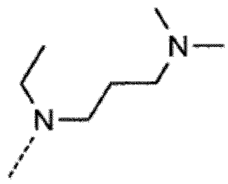 | H |

(fortgesetzt)

| Verbindung | $R_1$ | $R_2$ | $R_4$ | $R_7$ |
|---|---|---|---|---|
| 16 | $-CH_3$ | Phenyl | 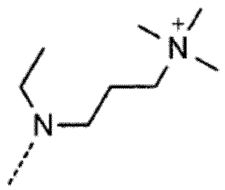 | H |
| 17 | $-CH_3$ | Phenyl | 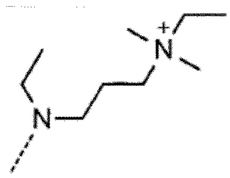 | H |
| 18 | $-CH_3$ | Phenyl | 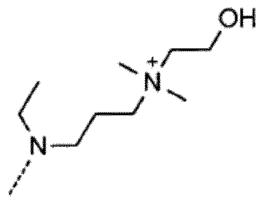 | H |
| 19 | $-CH_3$ | Phenyl | 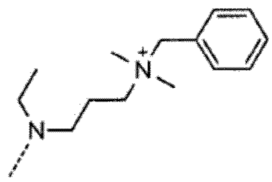 | H |
| 20 | $-CH_3$ | Phenyl | 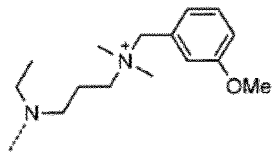 | H |
| 21 | $-CH_3$ | Phenyl | 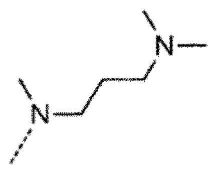 | H |
| 23 | $-CH_3$ | Phenyl | 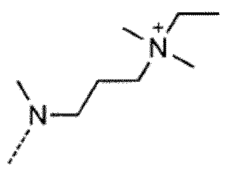 | H |
| 25 | $-CH_3$ | Phenyl | 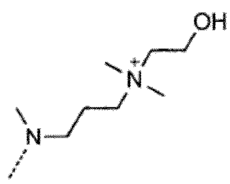 | H |

(fortgesetzt)

| Verbindung | $R_1$ | $R_2$ | $R_4$ | $R_7$ |
|---|---|---|---|---|
| 26 | $-CH_3$ | Phenyl | 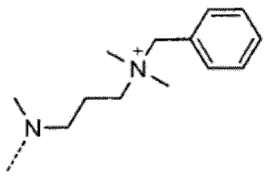 | H |
| 27 | $-CH_3$ | Phenyl | 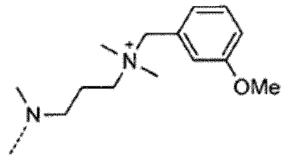 | H |
| 30 | $-CH_3$ | Phenyl | 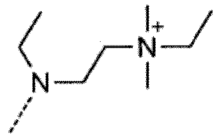 | H |
| 31 | $-CH_3$ | Phenyl | 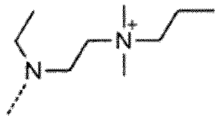 | H |
| 32 | $-CH_3$ | Phenyl | 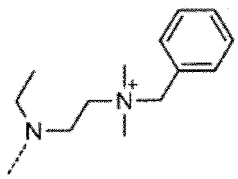 | H |
| 33 | $-CH_3$ | Phenyl | 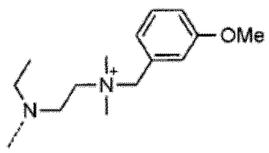 | H |
| 34 | $-CH_3$ | Phenyl | 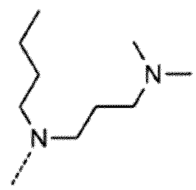 | $-OCH_3$ |
| 35 | $-CH_3$ | Phenyl | 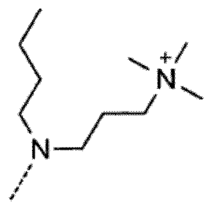 | $-OCH_3$ |

(fortgesetzt)

| Verbindung | $R_1$ | $R_2$ | $R_4$ | $R_7$ |
|---|---|---|---|---|
| 36 | $-CH_3$ | Phenyl | 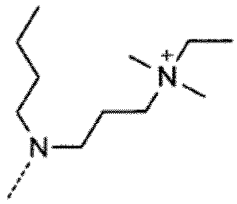 | $-OCH_3$ |
| 37 | $-CH_3$ | Phenyl | 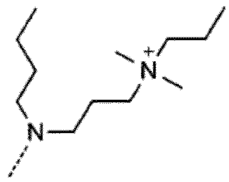 | $-OCH_3$ |
| 38 | $-CH_3$ | Phenyl | 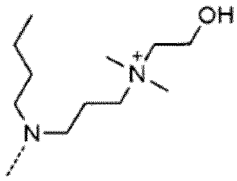 | $-OCH_3$ |
| 39 | $-CH_3$ | Phenyl | 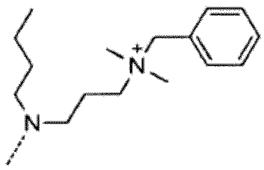 | $-OCH_3$ |
| 40 | $-CH_3$ | Phenyl | 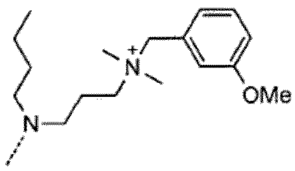 | $-OCH_3$ |
| 41 | $-CH_3$ | Phenyl | 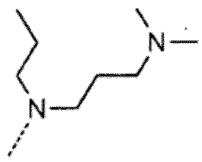 | $-OCH_3$ |
| 42 | $-CH_3$ | Phenyl | 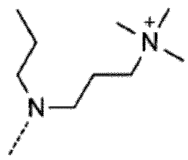 | $-OCH_3$ |
| 43 | $-CH_3$ | Phenyl | 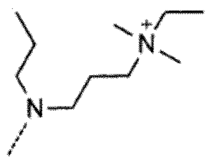 | $-OCH_3$ |

(fortgesetzt)

| Verbindung | $R_1$ | $R_2$ | $R_4$ | $R_7$ |
|---|---|---|---|---|
| 44 | $-CH_3$ | Phenyl | 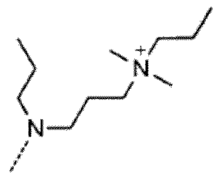 | $-OCH_3$ |
| 45 | $-CH_3$ | Phenyl | 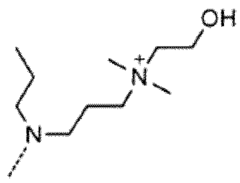 | $-OCH_3$ |
| 46 | $-CH_3$ | Phenyl | 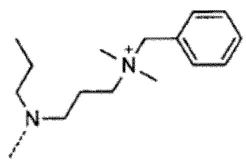 | $-OCH_3$ |
| 47 | $-CH_3$ | Phenyl | 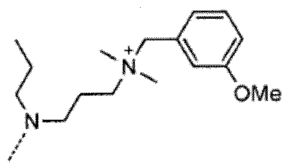 | $-OCH_3$ |
| 48 | $-CH_3$ | Phenyl | 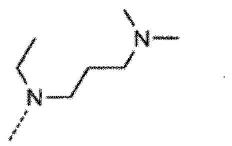 | $-OCH_3$ |
| 49 | $-CH_3$ | Phenyl | 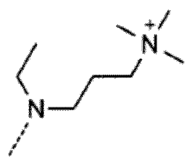 | $-OCH_3$ |
| 50 | $-CH_3$ | Phenyl | 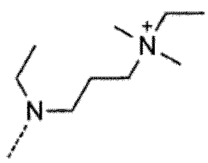 | $-OCH_3$ |
| 51 | $-CH_3$ | Phenyl | 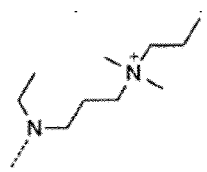 | $-OCH_3$ |
| 52 | $-CH_3$ | Phenyl | 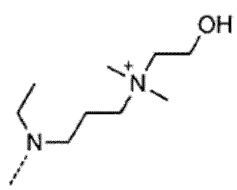 | $-OCH_3$ |

(fortgesetzt)

| Verbindung | $R_1$ | $R_2$ | $R_4$ | $R_7$ |
|---|---|---|---|---|
| 53 | $-CH_3$ | Phenyl | 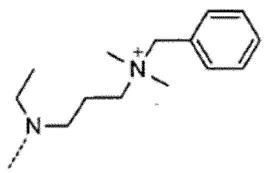 | $-OCH_3$ |
| 54 | $-CH_3$ | Phenyl | 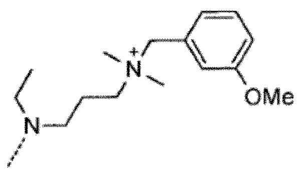 | $-OCH_3$ |
| 55 | $-CH_3$ | Phenyl | 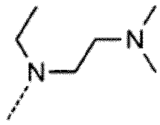 | $-OCH_3$ |
| 56 | $-CH_3$ | Phenyl | 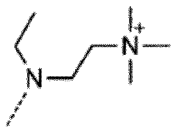 | $-OCH_3$ |
| 57 | $-CH_3$ | Phenyl | 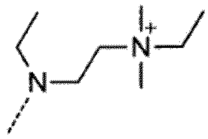 | $-OCH_3$ |
| 58 | $-CH_3$ | Phenyl | 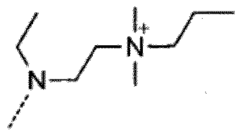 | $-OCH_3$ |
| 59 | $-CH_3$ | Phenyl | 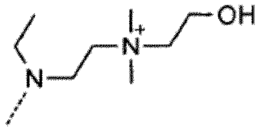 | $-OCH_3$ |
| 60 | $-CH_3$ | Phenyl | 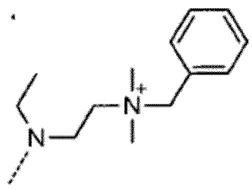 | $-OCH_3$ |

(b) Inkubieren der Probe eine Zeit lang, die ausreichend ist, um die Farbstoffverbindung mit der ssRNA in der Probe zu kombinieren, falls vorhanden, um einen Farbstoff-ssRNA-Komplex auszubilden, der ein nachweisbares fluoreszierendes Signal ausstrahlt; und

(c) Nachweisen der Ausstrahlung des fluoreszierenden Signals, wobei das nachweisbare fluoreszierende Signal hinweisend auf das Vorhandensein eines Farbstoff-ssRNA-Komplexes ist.

**2.** Verfahren nach Anspruch 1, wobei die ssRNA 20-25 Nukleotide umfasst.

**3.** Verfahren nach Anspruch 1, wobei die ssRNA 10-20 Nukleotide umfasst.

4. Verfahren nach Anspruch 1, wobei die ssRNA microRNA (miRNA) ist.

5. Verfahren nach Anspruch 1, wobei die ssRNA eine chemische Modifikation an wenigstens einem Zucker, einem Phosphat oder einer Basengruppe umfasst.

6. Verfahren nach Anspruch 1, wobei die ssRNA eine andere chemische Gruppe als ein Hydroxyl oder ein Phosphat an ihren 5'-Termini umfasst.

7. Verfahren nach Anspruch 1, wobei die ssRNA Folgendes ist: microRNA (miRNA), kleine nukleäre RNA (snRNA), kleine nukleoläre RNA (snoRNA), sehr kleine nichtcodierende RNA (tncRNA), kleine modulatorische RNA (smRNA) oder an PIWI-Proteine bindende RNA (piRNA).

8. Verfahren nach Anspruch 1, ferner das Nachweisen von ssRNA in einer Konzentration von etwa 5 ng/ml bis etwa 1000 ng/ml umfassend.

9. Verfahren nach Anspruch 1, wobei das Fluoreszenzsignal wenigstens 30 Minuten lang bei Zimmertemperatur stabil ist.

10. Farbstoffverbindung, ausgewählt aus der Gruppe bestehend aus Verbindung 4, Verbindung 5, Verbindung 6, Verbindung 7, Verbindung 8, Verbindung 9, Verbindung 10, Verbindung 11, Verbindung 12, Verbindung 13, Verbindung 14, Verbindung 15, Verbindung 16, Verbindung 17, Verbindung 18, Verbindung 19, Verbindung 20, Verbindung 23, Verbindung 25, Verbindung 26, Verbindung 27, Verbindung 30, Verbindung 31, Verbindung 32, Verbindung 33, Verbindung 34, Verbindung 35, Verbindung 36, Verbindung 37, Verbindung 38, Verbindung 39, Verbindung 40, Verbindung 43, Verbindung 44, Verbindung 45, Verbindung 46, Verbindung 47, Verbindung 48, Verbindung 49, Verbindung 50, Verbindung 51, Verbindung 52, Verbindung 53, Verbindung 54, Verbindung 55, Verbindung 56, Verbindung 57, Verbindung 58, Verbindung 59 und Verbindung 60 mit der folgenden Formel:

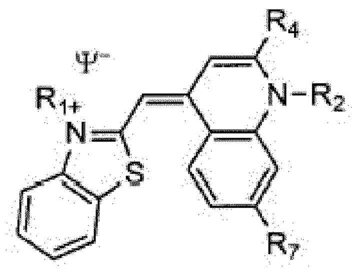

| Verbindung | $R_1$ | $R_2$ | $R_4$ | $R_7$ |
|---|---|---|---|---|
| 4 | $-CH_3$ | Phenyl | 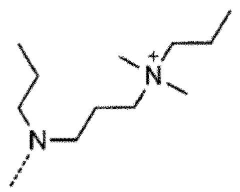 | H |
| 5 | $-CH_3$ | Phenyl | 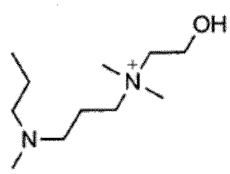 | H |

(fortgesetzt)

| Verbindung | $R_1$ | $R_2$ | $R_4$ | $R_7$ |
|---|---|---|---|---|
| 6 | $-CH_3$ | Phenyl | 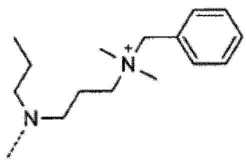 | H |
| 7 | $-CH_3$ | Phenyl | 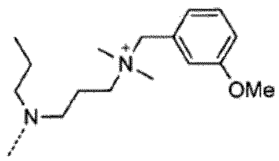 | H |
| 8 | $-CH_3$ | Phenyl | 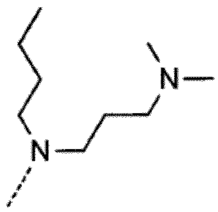 | H |
| 9 | $-CH_3$ | Phenyl | 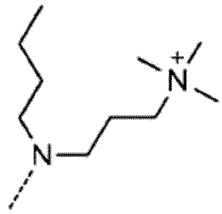 | H |
| 10 | $-CH_3$ | Phenyl | 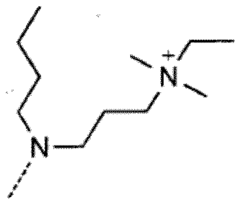 | H |
| 11 | $-CH_3$ | Phenyl | 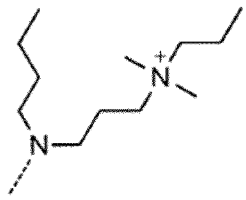 | H |
| 12 | $-CH_3$ | Phenyl | 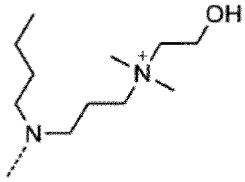 | H |

(fortgesetzt)

| Verbindung | $R_1$ | $R_2$ | $R_4$ | $R_7$ |
|---|---|---|---|---|
| 13 | $-CH_3$ | Phenyl | 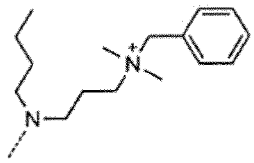 | H |
| 14 | $-CH_3$ | Phenyl | 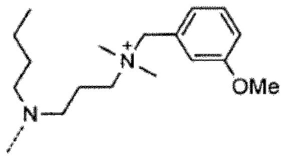 | H |
| 15 | $-CH_3$ | Phenyl | 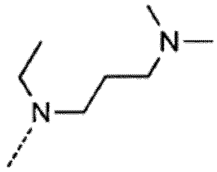 | H |
| 16 | $-CH_3$ | Phenyl | 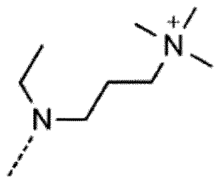 | H |
| 17 | $-CH_3$ | Phenyl | 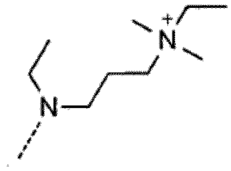 | H |
| 18 | $-CH_3$ | Phenyl | 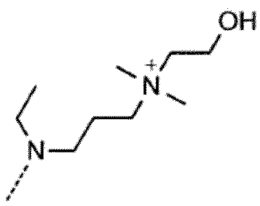 | H |
| 19 | $-CH_3$ | Phenyl | 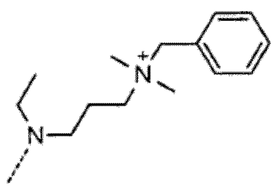 | H |
| 20 | $-CH_3$ | Phenyl | 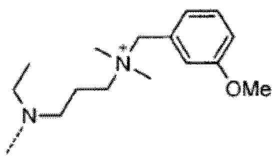 | H |

(fortgesetzt)

| Verbindung | $R_1$ | $R_2$ | $R_4$ | $R_7$ |
|---|---|---|---|---|
| 23 | $-CH_3$ | Phenyl | 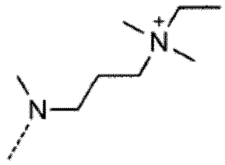 | H |
| 25 | $-CH_3$ | Phenyl | 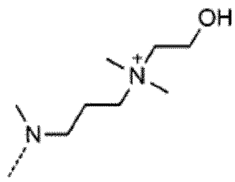 | H |
| 26 | $-CH_3$ | Phenyl | 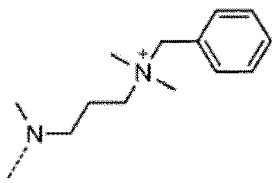 | H |
| 27 | $-CH_3$ | Phenyl | 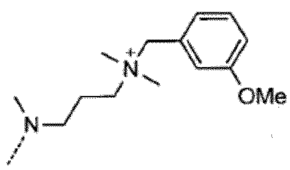 | H |
| 30 | $-CH_3$ | Phenyl | 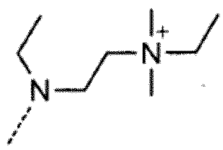 | H |
| 31 | $-CH_3$ | Phenyl | 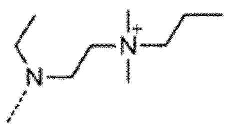 | H |
| 32 | $-CH_3$ | Phenyl | 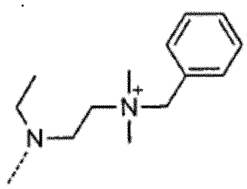 | H |
| 33 | $-CH_3$ | Phenyl | 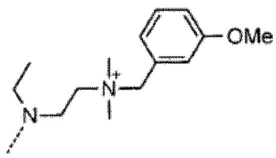 | H |

(fortgesetzt)

| Verbindung | $R_1$ | $R_2$ | $R_4$ | $R_7$ |
|---|---|---|---|---|
| 34 | $-CH_3$ | Phenyl | 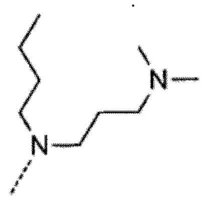 | $-OCH_3$ |
| 35 | $-CH_3$ | Phenyl | 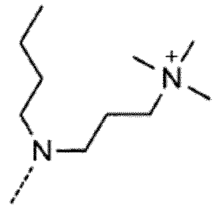 | $-OCH_3$ |
| 36 | $-CH_3$ | Phenyl | 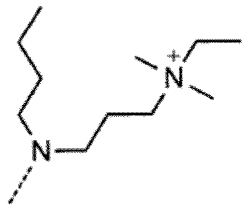 | $-OCH_3$ |
| 37 | $-CH_3$ | Phenyl | 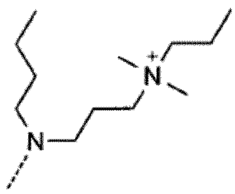 | $-OCH_3$ |
| 38 | $-CH_3$ | Phenyl | 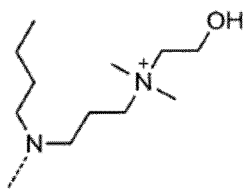 | $-OCH_3$ |
| 39 | $-CH_3$ | Phenyl | 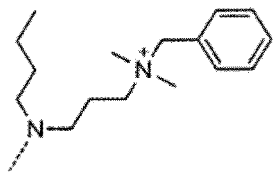 | $-OCH_3$ |
| 40 | $-CH_3$ | Phenyl | 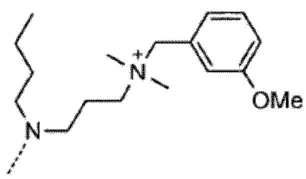 | $-OCH_3$ |

(fortgesetzt)

| Verbindung | $R_1$ | $R_2$ | $R_4$ | $R_7$ |
|---|---|---|---|---|
| 43 | $-CH_3$ | Phenyl | 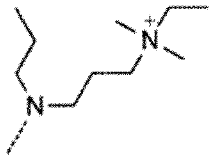 | $-OCH_3$ |
| 44 | $-CH_3$ | Phenyl | 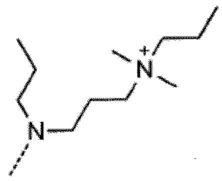 | $-OCH_3$ |
| 45 | $-CH_3$ | Phenyl | 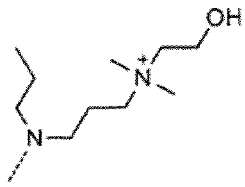 | $-OCH_3$ |
| 46 | $-CH_3$ | Phenyl | 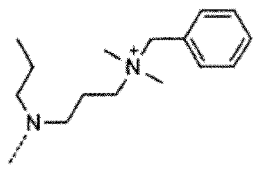 | $-OCH_3$ |
| 47 | $-CH_3$ | Phenyl | 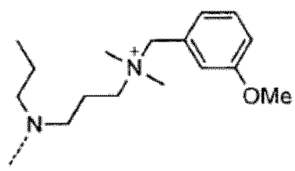 | $-OCH_3$ |
| 48 | $-CH_3$ | Phenyl | 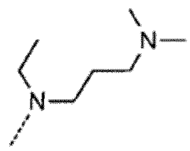 | $-OCH_3$ |
| 49 | $-CH_3$ | Phenyl | 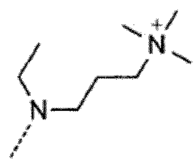 | $-OCH_3$ |
| 50 | $-CH_3$ | Phenyl | 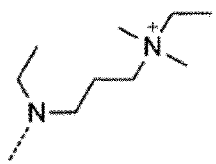 | $-OCH_3$ |

(fortgesetzt)

| Verbindung | $R_1$ | $R_2$ | $R_4$ | $R_7$ |
|---|---|---|---|---|
| 51 | $-CH_3$ | Phenyl | 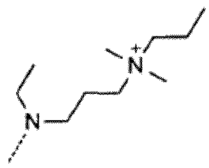 | $-OCH_3$ |
| 52 | $-CH_3$ | Phenyl | 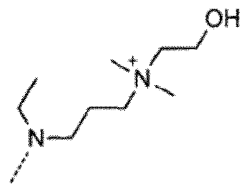 | $-OCH_3$ |
| 53 | $-CH_3$ | Phenyl | 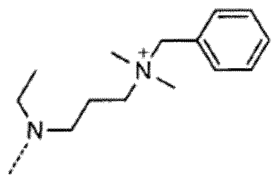 | $-OCH_3$ |
| 54 | $-CH_3$ | Phenyl | 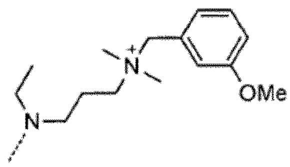 | $-OCH_3$ |
| 55 | $-CH_3$ | Phenyl | 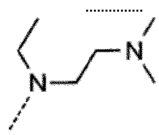 | $-OCH_3$ |
| 56 | $-CH_3$ | Phenyl | 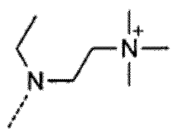 | $-OCH_3$ |
| 57 | $-CH_3$ | Phenyl | 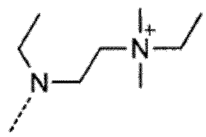 | $-OCH_3$ |
| 58 | $-CH_3$ | Phenyl | 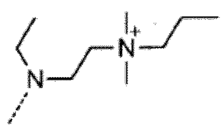 | $-OCH_3$ |
| 59 | $-CH_3$ | Phenyl | 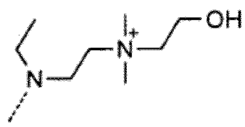 | $-OCH_3$ |

(fortgesetzt)

| Verbindung | $R_1$ | $R_2$ | $R_4$ | $R_7$ |
|---|---|---|---|---|
| 60 | $-CH_3$ | Phenyl | 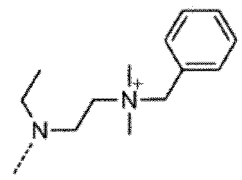 | $-OCH_3$ |

**11.** Satz zum Nachweisen des Vorhandenseins von einzelsträngiger RNA (ssRNA) in einer Probe, Folgendes umfassend: die Farbstoffverbindung nach Anspruch 10 und Anweisungen zur Verwendung des Farbstoffes in dem Verfahren nach Anspruch 1.

## Revendications

**1.** Procédé de détection dans un échantillon d'un ARN simple brin (ARNsb) ayant 25 nucléotides ou moins, comprenant :

(a) la combinaison d'un échantillon susceptible de contenir un ARNsb, l'ARNsb comprenant 25 nucléotides ou moins, avec un composé de colorant de cyanine substitué et non symétrique de la formule II :

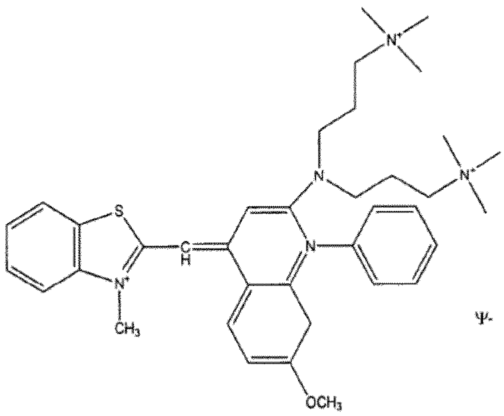

(II)

ou

un composé de colorant sélectionné parmi le groupe constitué du Composé 4, Composé 5, Composé 6, Composé 7, Composé 8, Composé 9, Composé 10, Composé 11, Composé 12, Composé 13, Composé 14, Composé 15, Composé 16, Composé 17, Composé 18, Composé 19, Composé 20, Composé 21, Composé 23, Composé 25, Composé 26, Composé 27, Composé 30, Composé 31, Composé 32, Composé 33, Composé 34, Composé 35, Composé 36, Composé 37, Composé 38, Composé 39, Composé 40, Composé 41, Composé 42, Composé 43, Composé 44, Composé 45, Composé 46, Composé 47, Composé 48, Composé 49, Composé 50, Composé 51, Composé 52, Composé 53, Composé 54, Composé 55, Composé 56, Composé 57, Composé 58, Composé 59 et Composé 60 ayant la formule suivante :

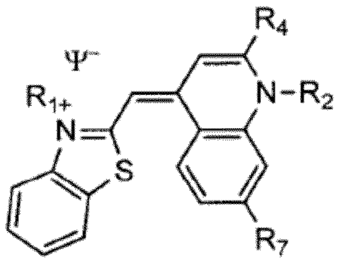

| Composé | $R_1$ | $R_2$ | $R_4$ | $R_7$ |
|---|---|---|---|---|
| 4 | $-CH_3$ | phényle | 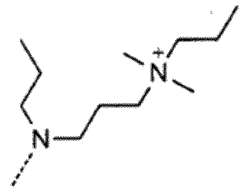 | H |
| 5 | $-CH_3$ | phényle | 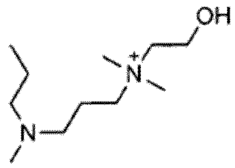 | H |
| 6 | $-CH_3$ | phényle | 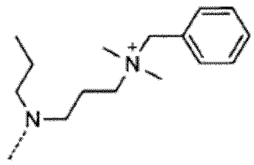 | H |
| 7 | $-CH_3$ | phényle | 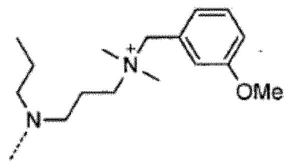 | H |
| 8 | $-CH_3$ | phényle | 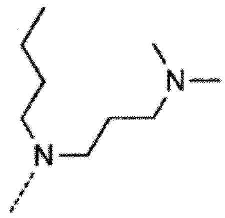 | H |
| 9 | $-CH_3$ | phényle | 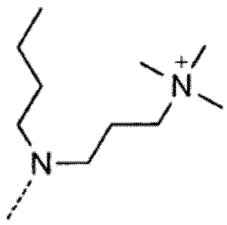 | H |
| 10 | $-CH_3$ | phényle | 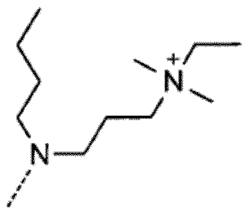 | H |

(suite)

| Composé | $R_1$ | $R_2$ | $R_4$ | $R_7$ |
|---|---|---|---|---|
| 11 | $-CH_3$ | phényle | 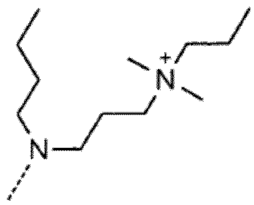 | H |
| 12 | $-CH_3$ | phényle | 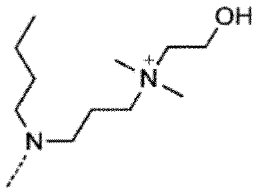 | H |
| 13 | $-CH_3$ | phényle | 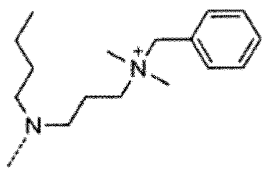 | H |
| 14 | $-CH_3$ | phényle | 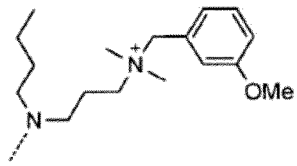 | H |
| 15 | $-CH_3$ | phényle | 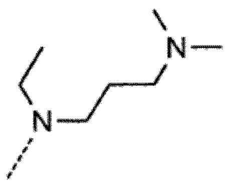 | H |
| 16 | $-CH_3$ | phényle | 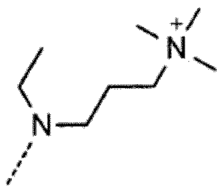 | H |
| 17 | $-CH_3$ | phényle | 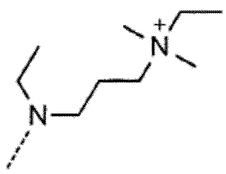 | H |
| 18 | $-CH_3$ | phényle | 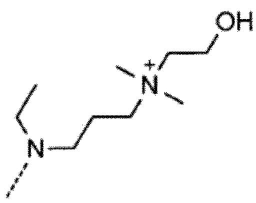 | H |

(suite)

| Composé | $R_1$ | $R_2$ | $R_4$ | $R_7$ |
|---|---|---|---|---|
| 19 | $-CH_3$ | phényle | 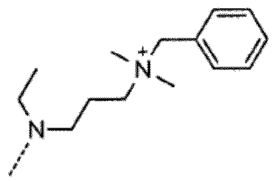 | H |
| 20 | $-CH_3$ | phényle | 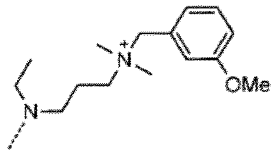 | H |
| 21 | $-CH_3$ | phényle | 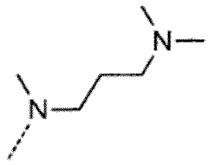 | H |
| 23 | $-CH_3$ | phényle | 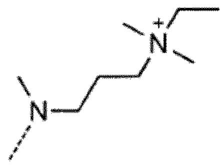 | H |
| 25 | $-CH_3$ | phényle | 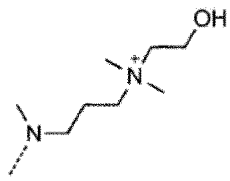 | H |
| 26 | $-CH_3$ | phényle | 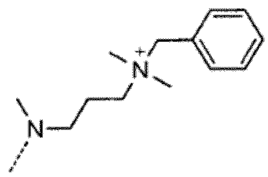 | H |
| 27 | $-CH_3$ | phényle | 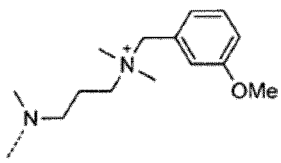 | H |
| 30 | $-CH_3$ | phényle | 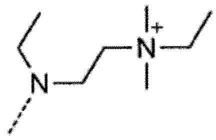 | H |
| 31 | $-CH_3$ | phényle | 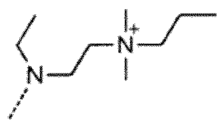 | H |

(suite)

| Composé | $R_1$ | $R_2$ | $R_4$ | $R_7$ |
|---|---|---|---|---|
| 32 | $-CH_3$ | phényle | 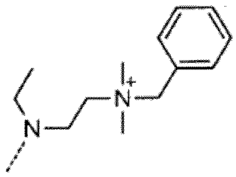 | H |
| 33 | $-CH_3$ | phényle | 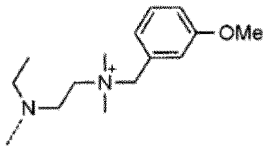 | H |
| 34 | $-CH_3$ | phényle | 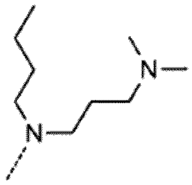 | $-OCH_3$ |
| 35 | $-CH_3$ | phényle | 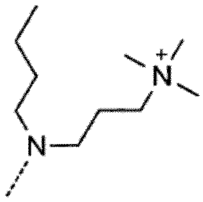 | $-OCH_3$ |
| 36 | $-CH_3$ | phényle | 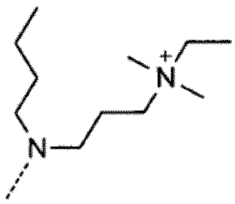 | $-OCH_3$ |
| 37 | $-CH_3$ | phényle | 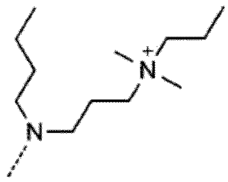 | $-OCH_3$ |
| 38 | $-CH_3$ | phényle | 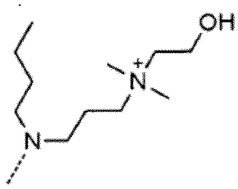 | $-OCH_3$ |
| 39 | $-CH_3$ | phényle | 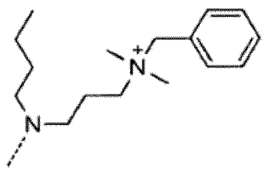 | $-OCH_3$ |

(suite)

| Composé | $R_1$ | $R_2$ | $R_4$ | $R_7$ |
|---|---|---|---|---|
| 40 | $-CH_3$ | phényle | 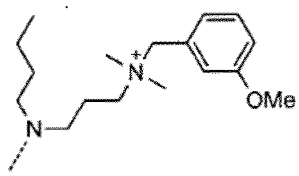 | $-OCH_3$ |
| 41 | $-CH_3$ | phényle | 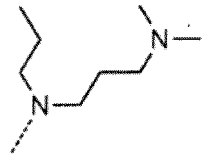 | $-OCH_3$ |
| 42 | $-CH_3$ | phényle | 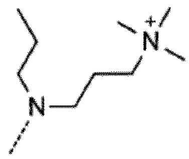 | $-OCH_3$ |
| 43 | $-CH_3$ | phényle | 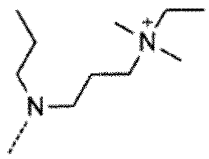 | $-OCH_3$ |
| 44 | $-CH_3$ | phényle | 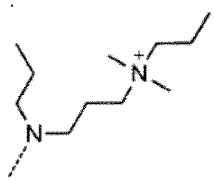 | $-OCH_3$ |
| 45 | $-CH_3$ | phényle | 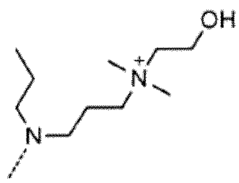 | $-OCH_3$ |
| 46 | $-CH_3$ | phényle | 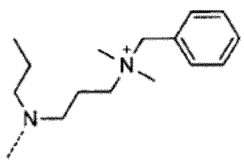 | $-OCH_3$ |
| 47 | $-CH_3$ | phényle | 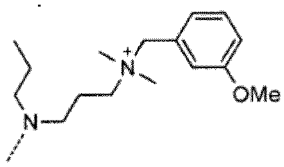 | $-OCH_3$ |
| 48 | $-CH_3$ | phényle | 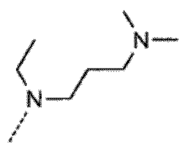 | $-OCH_3$ |

(suite)

| Composé | $R_1$ | $R_2$ | $R_4$ | $R_7$ |
|---|---|---|---|---|
| 49 | $-CH_3$ | phényle | 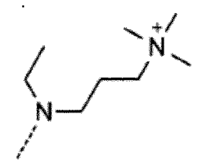 | $-OCH_3$ |
| 50 | $-CH_3$ | phényle | 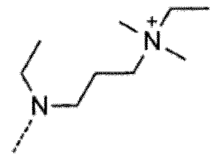 | $-OCH_3$ |
| 51 | $-CH_3$ | phényle | 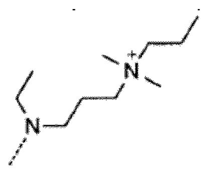 | $-OCH_3$ |
| 52 | $-CH_3$ | phényle | 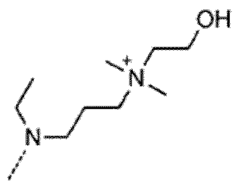 | $-OCH_3$ |
| 53 | $-CH_3$ | phényle | 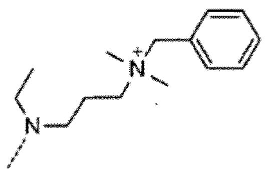 | $-OCH_3$ |
| 54 | $-CH_3$ | phényle | 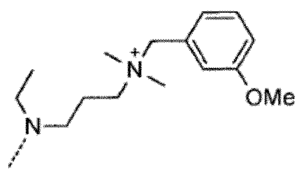 | $-OCH_3$ |
| 55 | $-CH_3$ | phényle | 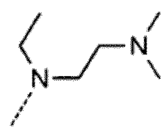 | $-OCH_3$ |
| 56 | $-CH_3$ | phényle | 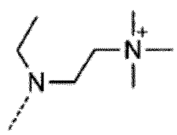 | $-OCH_3$ |
| 57 | $-CH_3$ | phényle | 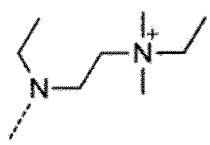 | $-OCH_3$ |

(suite)

| Composé | $R_1$ | $R_2$ | $R_4$ | $R_7$ |
|---|---|---|---|---|
| 58 | $-CH_3$ | phényle | 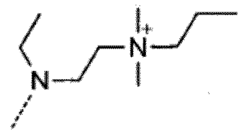 | $-OCH_3$ |
| 59 | $-CH_3$ | phényle | 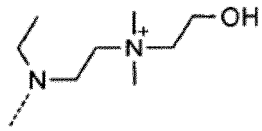 | $-OCH_3$ |
| 60 | $-CH_3$ | phényle | 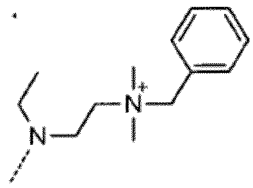 | $-OCH_3$ |

(b) l'incubation de l'échantillon pendant une durée suffisante pour que le composant de colorant soit combiné avec l'ARNsb dans l'échantillon, si présent, pour former un complexe colorant-ARNsb qui émet un signal fluorescent détectable ; et
(c) la détection de l'émission du signal fluorescent, le signal fluorescent détectable étant indicatif de la présence d'un complexe colorant-ARNsb.

**2.** Procédé selon la revendication 1, l'ARNsb comprenant entre 20 et 25 nucléotides.

**3.** Procédé selon la revendication 1, l'ARNsb comprenant entre 10 et 20 nucléotides.

**4.** Procédé selon la revendication 1, l'ARNsb étant un microARN (miARN).

**5.** Procédé selon la revendication 1, l'ARNsb comprenant une modification chimique sur au moins un sucre, un phosphate ou un groupe de base.

**6.** Procédé selon la revendication 1, l'ARNsb comprenant un groupe chimique autre qu'un hydroxyle ou un phosphate au niveau de ses terminaisons 5'.

**7.** Procédé selon la revendication 1, l'ARNsb étant un microARN (miARN), un petit ARN nucléaire (ARNsn), un petit ARN nucléolaire (snoARN), un petit ARN non codant (tncARN), un petit ARN modulateur (smARN) ou un ARN interagissant avec Piwi (piARN).

**8.** Procédé selon la revendication 1, comprenant en outre la détection d'ARNsb dans une concentration d'environ 5 ng/ml à environ 1 000 ng/ml.

**9.** Procédé selon la revendication 1, le signal de fluorescence étant stable pendant au moins 30 minutes à température ambiante.

**10.** Composé de colorant sélectionné parmi le groupe constitué du Composé 4, Composé 5, Composé 6, Composé 7, Composé 8, Composé 9, Composé 10, Composé 11, Composé 12, Composé 13, Composé 14, Composé 15, Composé 16, Composé 17, Composé 18, Composé 19, Composé 20, Composé 23, Composé 25, Composé 26, Composé 27, Composé 30, Composé 31, Composé 32, Composé 33, Composé 34, Composé 35, Composé 36, Composé 37, Composé 38, Composé 39, Composé 40, Composé 43, Composé 44, Composé 45, Composé 46, Composé 47, Composé 48, Composé 49, Composé 50, Composé 51, Composé 52, Composé 53, Composé 54, Composé 55, Composé 56, Composé 57, Composé 58, Composé 59 et Composé 60 ayant la formule suivante :

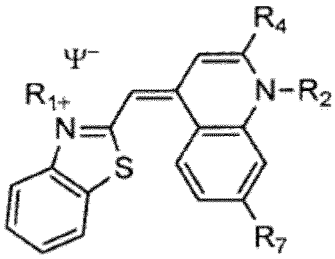

| Composé | $R_1$ | $R_2$ | $R_4$ | $R_7$ |
|---|---|---|---|---|
| 4 | $-CH_3$ | phényle | 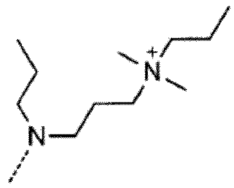 | H |
| 5 | $-CH_3$ | phényle | 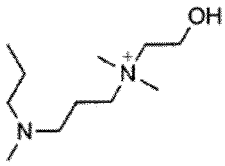 | H |
| 6 | $-CH_3$ | phényle | 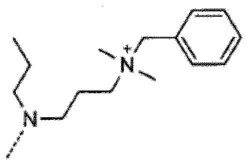 | H |
| 7 | $-CH_3$ | phényle | 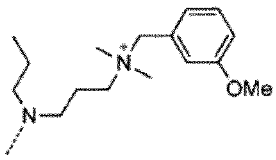 | H |
| 8 | $-CH_3$ | phényle | 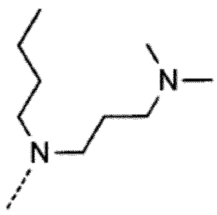 | H |
| 9 | $-CH_3$ | phényle | 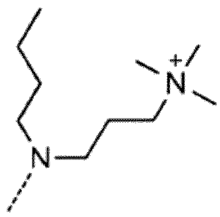 | H |

(suite)

| Composé | $R_1$ | $R_2$ | $R_4$ | $R_7$ |
|---|---|---|---|---|
| 10 | $-CH_3$ | phényle | 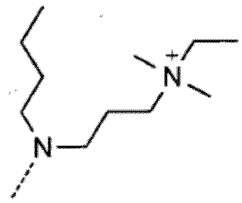 | H |
| 11 | $-CH_3$ | phényle | 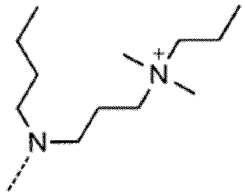 | H |
| 12 | $-CH_3$ | phényle | 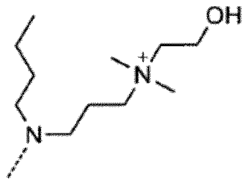 | H |
| 13 | $-CH_3$ | phényle | 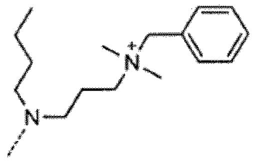 | H |
| 14 | $-CH_3$ | phényle | 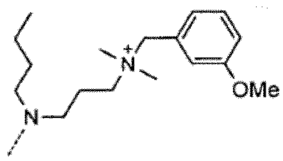 | H |
| 15 | $-CH_3$ | phényle | 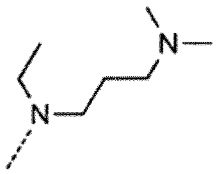 | H |
| 16 | $-CH_3$ | phényle | 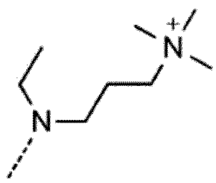 | H |
| 17 | $-CH_3$ | phényle | 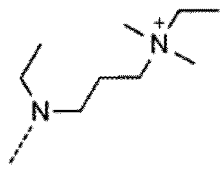 | H |

(suite)

| Composé | $R_1$ | $R_2$ | $R_4$ | $R_7$ |
|---|---|---|---|---|
| 18 | $-CH_3$ | phényle | 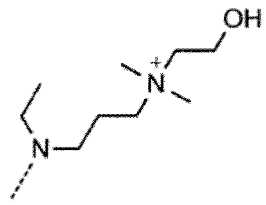 | H |
| 19 | $-CH_3$ | phényle | 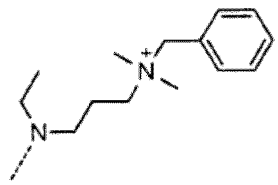 | H |
| 20 | $-CH_3$ | phényle | 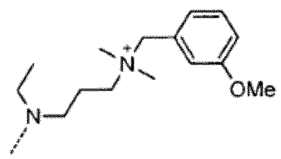 | H |
| 23 | $-CH_3$ | phényle | 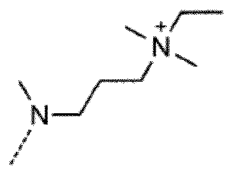 | H |
| 25 | $-CH_3$ | phényle | 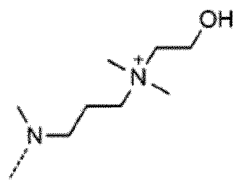 | H |
| 26 | $-CH_3$ | phényle | 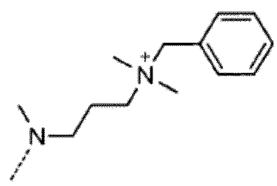 | H |
| 27 | $-CH_3$ | phényle | 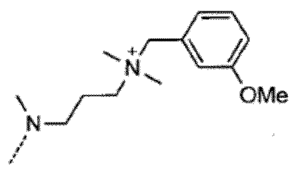 | H |
| 30 | $-CH_3$ | phényle | 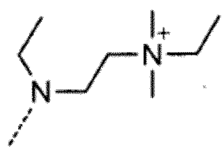 | H |

(suite)

| Composé | $R_1$ | $R_2$ | $R_4$ | $R_7$ |
|---|---|---|---|---|
| 31 | $-CH_3$ | phényle | 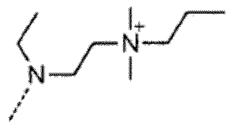 | H |
| 32 | $-CH_3$ | phényle | 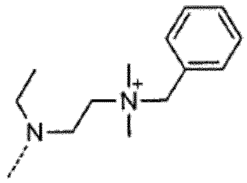 | H |
| 33 | $-CH_3$ | phényle | 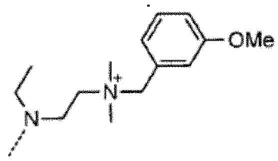 | H |
| 34 | $-CH_3$ | phényle | 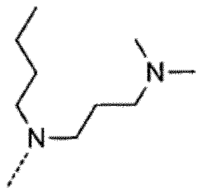 | $-OCH_3$ |
| 35 | $-CH_3$ | phényle | 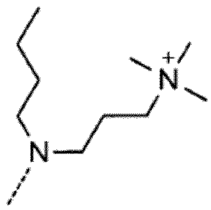 | $-OCH_3$ |
| 36 | $-CH_3$ | phényle | 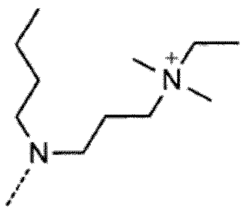 | $-OCH_3$ |
| 37 | $-CH_3$ | phényle | 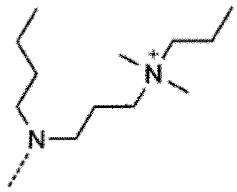 | $-OCH_3$ |

(suite)

| Composé | $R_1$ | $R_2$ | $R_4$ | $R_7$ |
|---|---|---|---|---|
| 38 | $-CH_3$ | phényle | 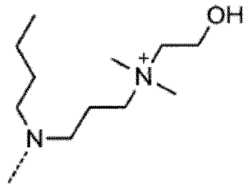 | $-OCH_3$ |
| 39 | $-CH_3$ | phényle | 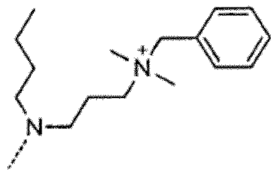 | $-OCH_3$ |
| 40 | $-CH_3$ | phényle | 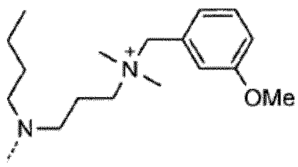 | $-OCH_3$ |
| 43 | $-CH_3$ | phényle | 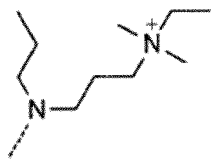 | $-OCH_3$ |
| 44 | $-CH_3$ | phényle | 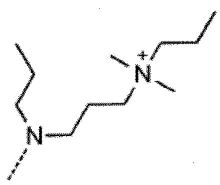 | $-OCH_3$ |
| 45 | $-CH_3$ | phényle | 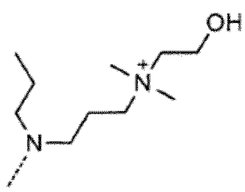 | $-OCH_3$ |
| 46 | $-CH_3$ | phényle | 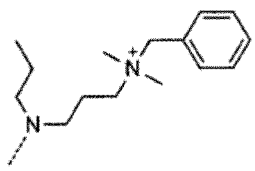 | $-OCH_3$ |
| 47 | $-CH_3$ | phényle | 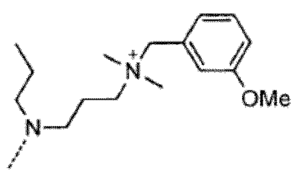 | $-OCH_3$ |

(suite)

| Composé | $R_1$ | $R_2$ | $R_4$ | $R_7$ |
|---|---|---|---|---|
| 48 | $-CH_3$ | phényle | 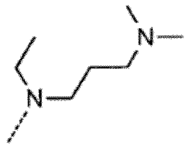 | $-OCH_3$ |
| 49 | $-CH_3$ | phényle | 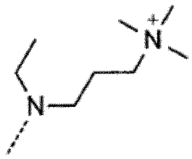 | $-OCH_3$ |
| 50 | $-CH_3$ | phényle | 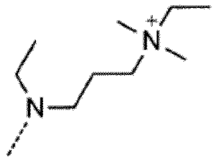 | $-OCH_3$ |
| 51 | $-CH_3$ | phényle | 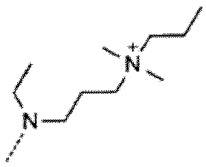 | $-OCH_3$ |
| 52 | $-CH_3$ | phényle | 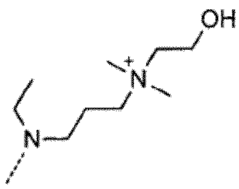 | $-OCH_3$ |
| 53 | $-CH_3$ | phényle | 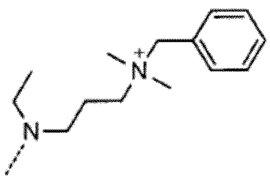 | $-OCH_3$ |
| 54 | $-CH_3$ | phényle | 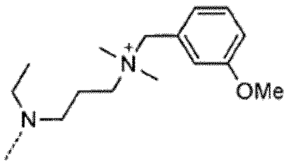 | $-OCH_3$ |
| 55 | $-CH_3$ | phényle | 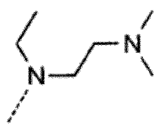 | $-OCH_3$ |
| 56 | $-CH_3$ | phényle | 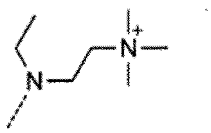 | $-OCH_3$ |

(suite)

| Composé | $R_1$ | $R_2$ | $R_4$ | $R_7$ |
|---|---|---|---|---|
| 57 | $-CH_3$ | phényle | 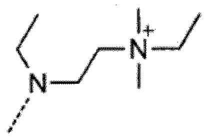 | $-OCH_3$ |
| 58 | $-CH_3$ | phényle | 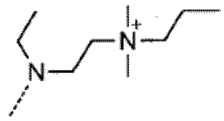 | $-OCH_3$ |
| 59 | $-CH_3$ | phényle | 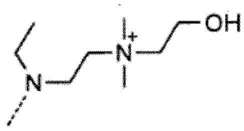 | $-OCH_3$ |
| 60 | $-CH_3$ | phényle | 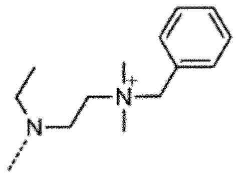 | $-OCH_3$ |

**11.** Kit de détection de la présence d'ARN simple brin (ARNsb) dans un échantillon, comprenant : le composé de colorant selon la revendication 10 et des instructions pour utiliser le colorant dans le procédé selon la revendication 1.

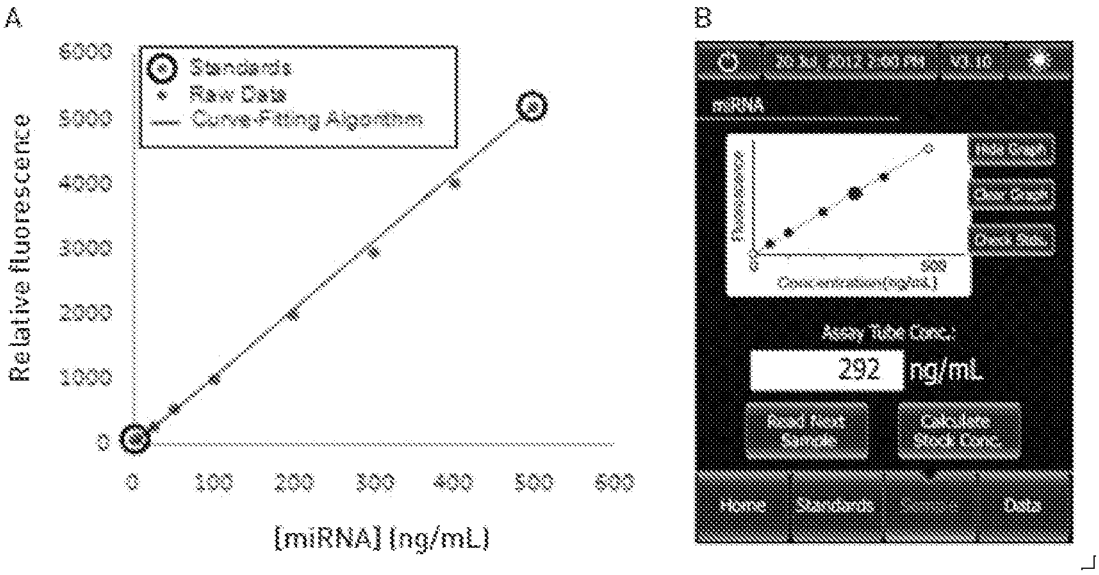
A
B
6000
5000
4000
3000
2000
1000
0
Standards
Raw Data
Curve-Fitting Algorithm
Relative fluorescence
0
100
200
300
400
500
600
[miRNA] (ng/mL)
miRNA
Assay Tube Conc.:
292
ng/mL
Home
Standards
Data

**Figure 1**

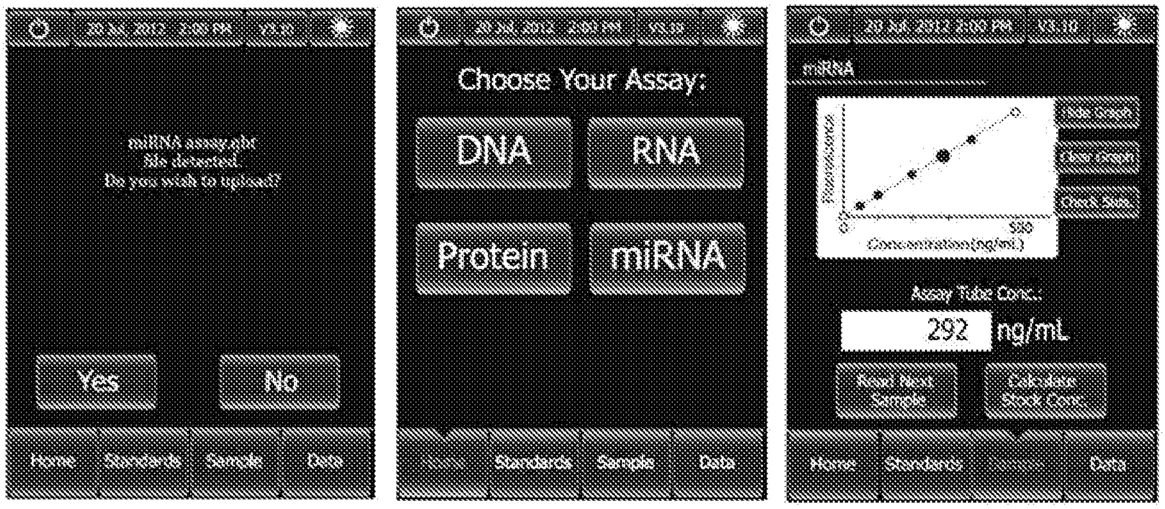
Yes
No
Home
Standards
Sample
Data
Choose Your Assay:
DNA
RNA
Protein
miRNA
Standards
Sample
Data
miRNA
Concentration (ng/mL)
Assay Tube Conc.:
292
ng/mL
Read Next Sample
Calculate Stock Conc.
Home
Standards
Data

Figure 2

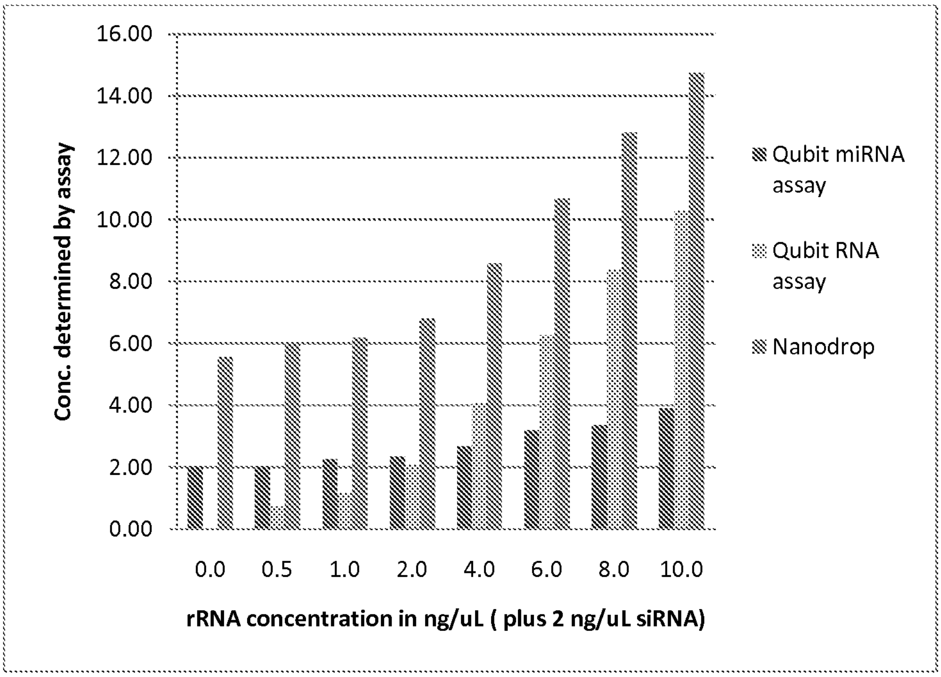
16.00
14.00
12.00
10.00
8.00
6.00
4.00
2.00
0.00
Conc. determined by assay
0.0
0.5
1.0
2.0
4.0
6.0
8.0
10.0
rRNA concentration in ng/uL ( plus 2 ng/uL siRNA)
Qubit miRNA assay
Qubit RNA assay
Nanodrop

Figure 3

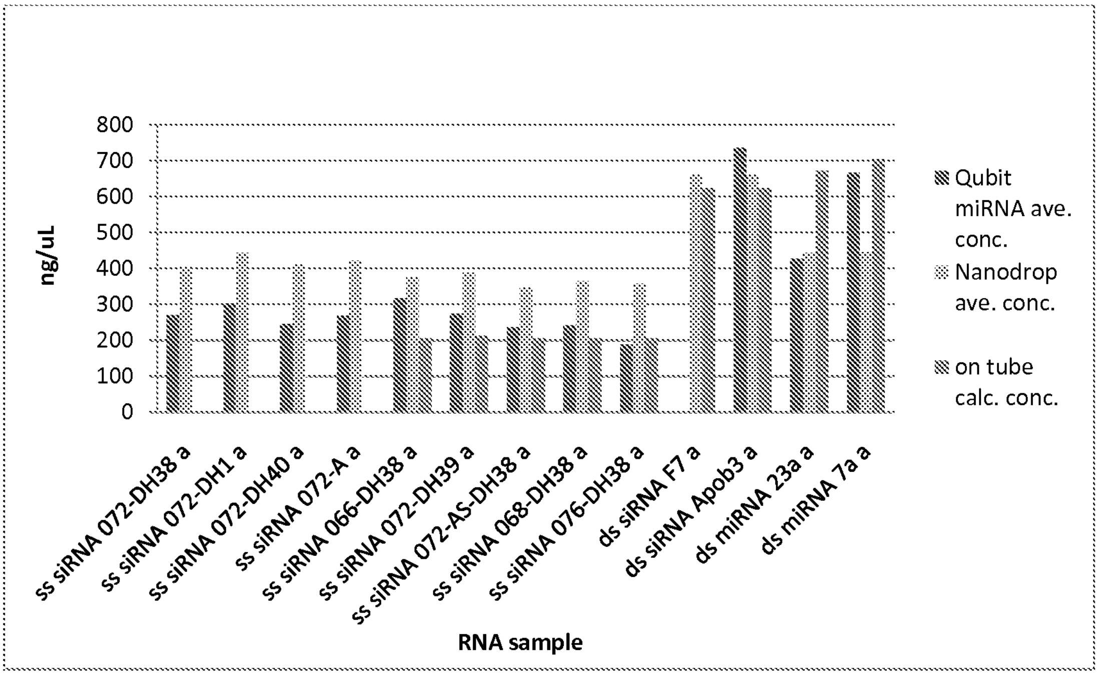

800
700
600
500
400
300
200
100
0
ng/uL
Qubit miRNA ave. conc.
Nanodrop ave. conc.
on tube calc. conc.
ss siRNA 072-DH38 a
ss siRNA 072-DH1 a
ss siRNA 072-DH40 a
ss siRNA 072-A a
ss siRNA 066-DH38 a
ss siRNA 072-DH39 a
ss siRNA 072-AS-DH38 a
ss siRNA 068-DH38 a
ss siRNA 076-DH38 a
ds siRNA F7 a
ds siRNA Apob3 a
ds miRNA 23a a
ds miRNA 7a a
RNA sample


Figure 4

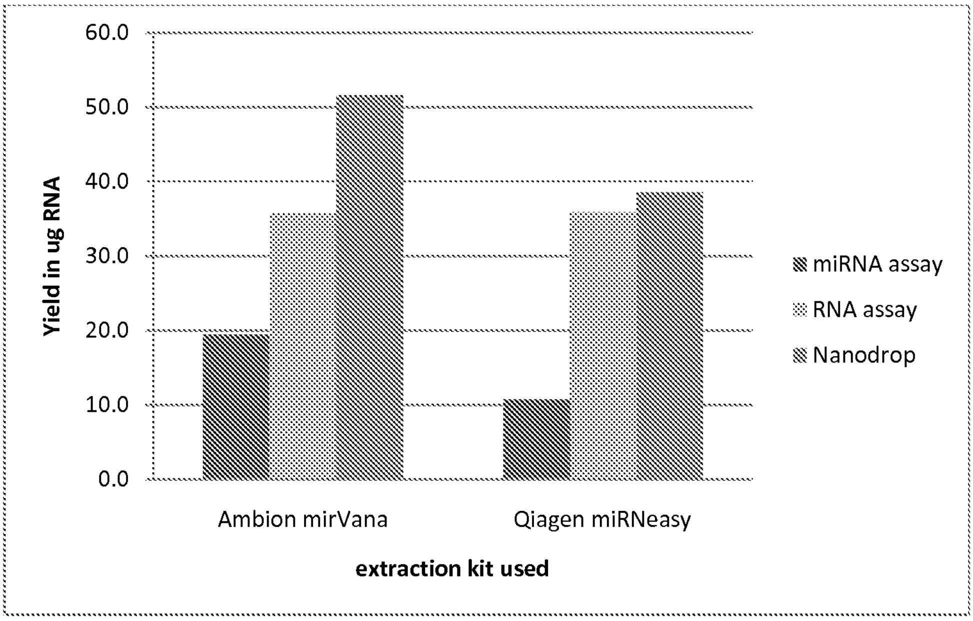
60.0
50.0
40.0
30.0
20.0
10.0
0.0
Yield in ug RNA
Ambion mirVana
Qiagen miRNeasy
extraction kit used
miRNA assay
RNA assay
Nanodrop

**Figure 5**

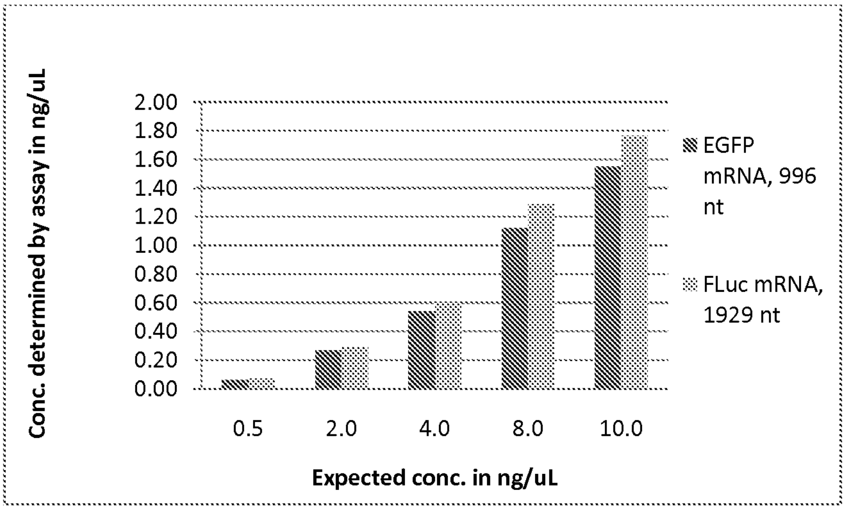


**Figure 6A**

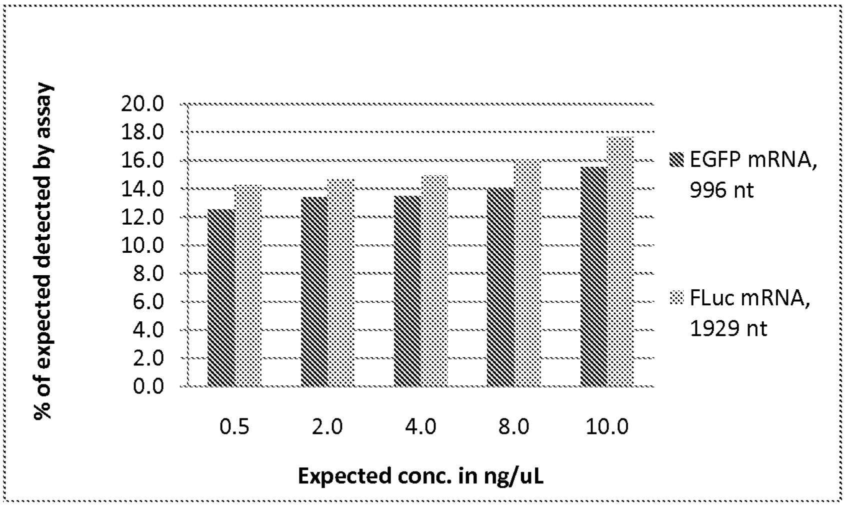


**Figure 6B**

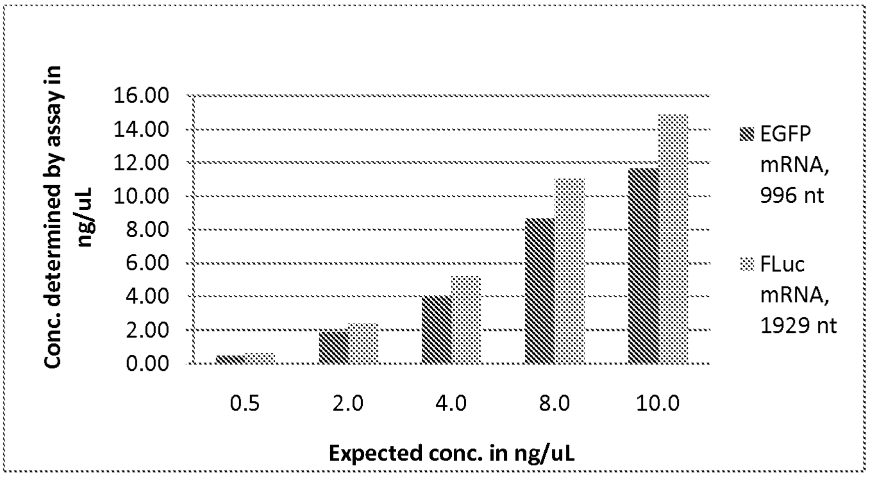


**Figure 6C**

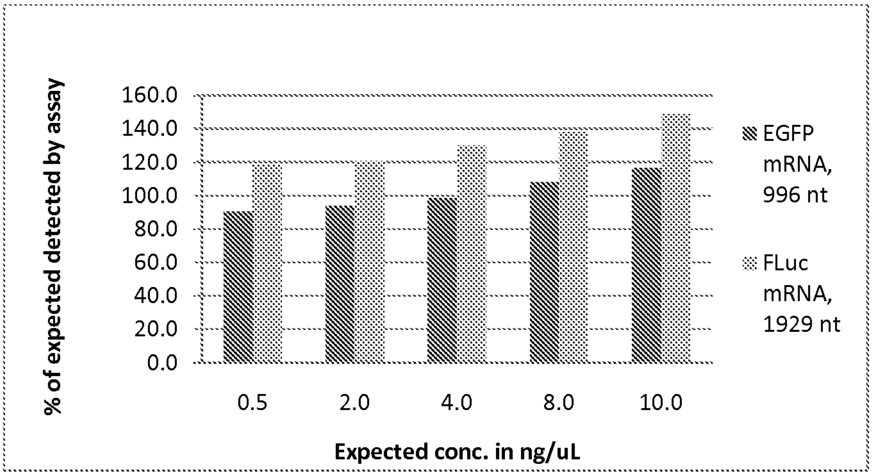


**Figure 6D**

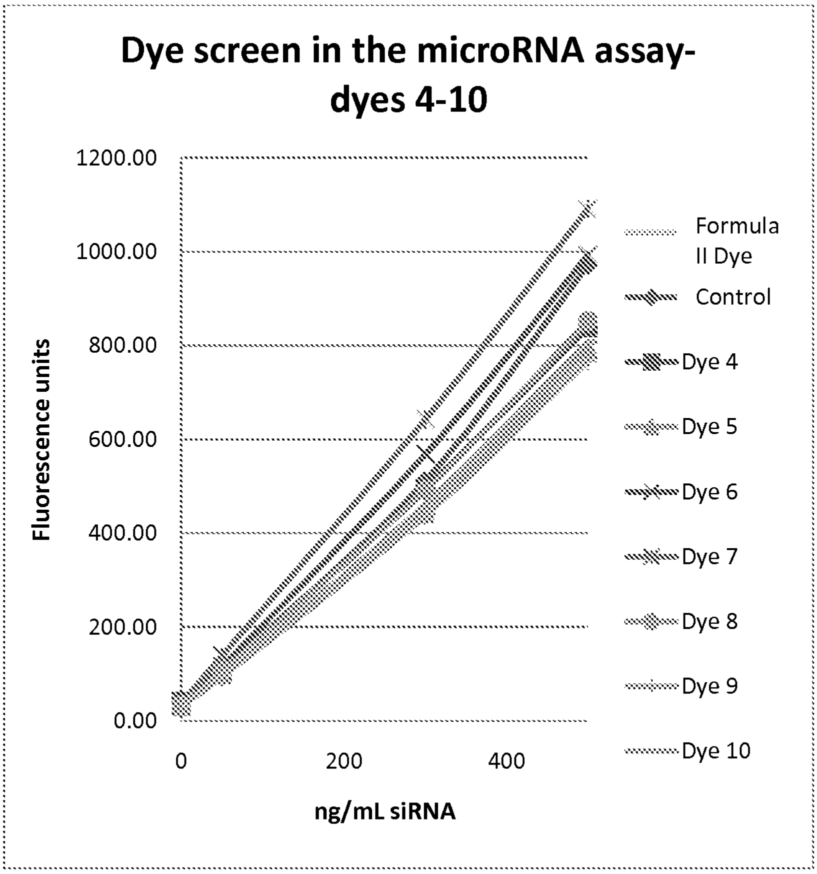
Dye screen in the microRNA assay-
dyes 4-10
1200.00
1000.00
800.00
600.00
400.00
200.00
0.00
Fluorescence units
0
200
400
ng/mL siRNA
Formula II Dye
Control
Dye 4
Dye 5
Dye 6
Dye 7
Dye 8
Dye 9
Dye 10

**Figure 7A**

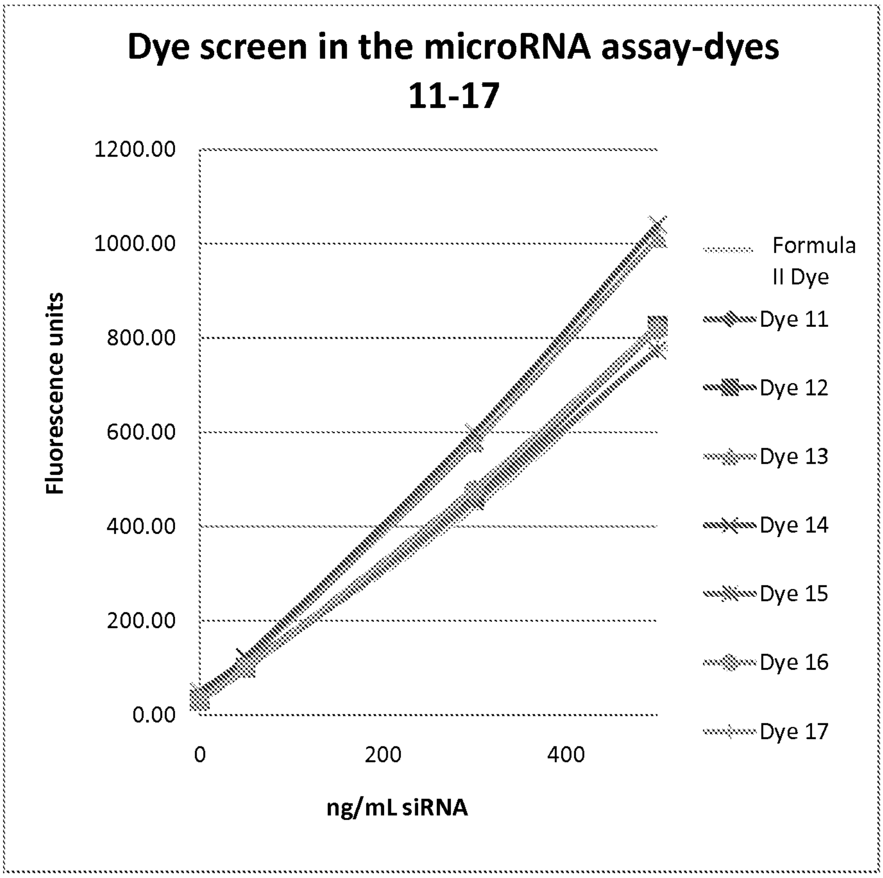

Dye screen in the microRNA assay-dyes 11-17
1200.00
1000.00
800.00
600.00
400.00
200.00
0.00
Fluorescence units
0
200
400
ng/mL siRNA
Formula II Dye
Dye 11
Dye 12
Dye 13
Dye 14
Dye 15
Dye 16
Dye 17


Figure 7B

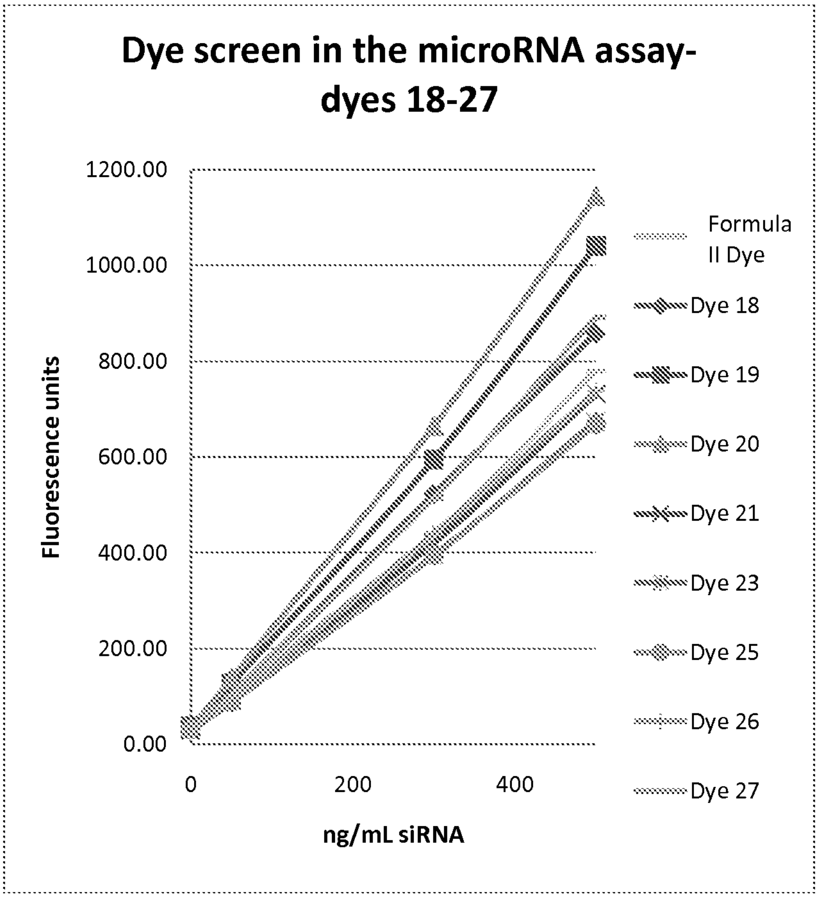
Dye screen in the microRNA assay-
dyes 18-27
Fluorescence units
1200.00
1000.00
800.00
600.00
400.00
200.00
0.00
0
200
400
ng/mL siRNA
Formula II Dye
Dye 18
Dye 19
Dye 20
Dye 21
Dye 23
Dye 25
Dye 26
Dye 27

Figure 7C

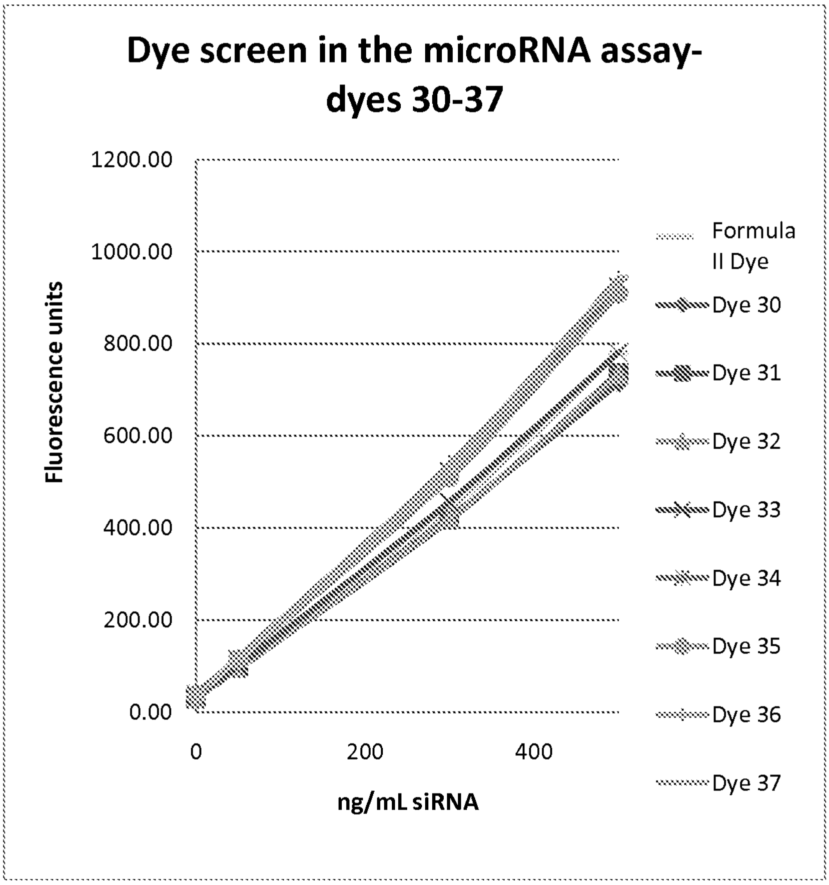
Dye screen in the microRNA assay-
dyes 30-37
1200.00
1000.00
800.00
600.00
400.00
200.00
0.00
Fluorescence units
0
200
400
ng/mL siRNA
Formula II Dye
Dye 30
Dye 31
Dye 32
Dye 33
Dye 34
Dye 35
Dye 36
Dye 37

**Figure 7D**

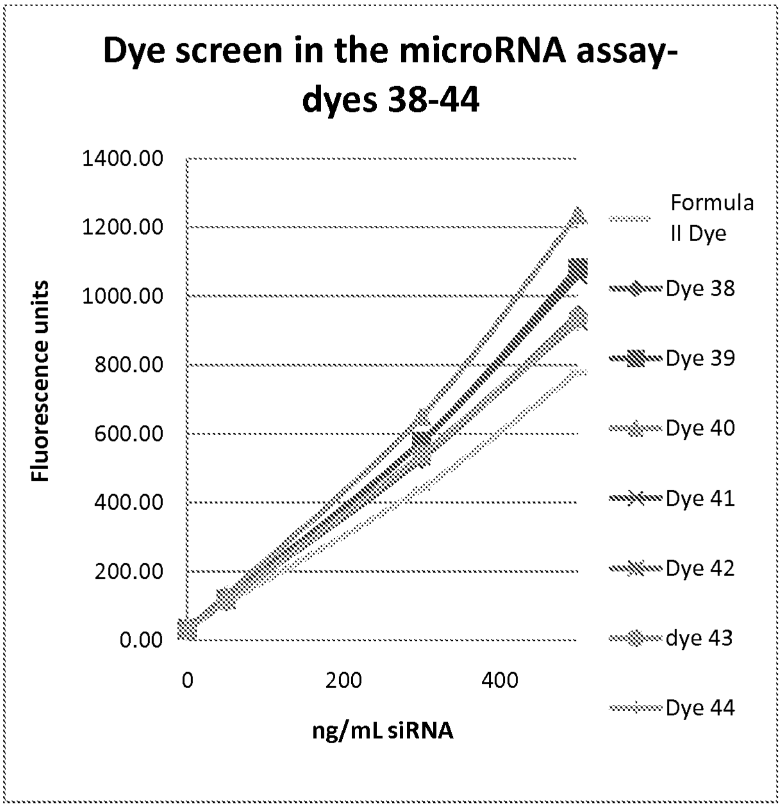
Dye screen in the microRNA assay-dyes 38-44
1400.00
1200.00
1000.00
800.00
600.00
400.00
200.00
0.00
Fluorescence units
0
200
400
ng/mL siRNA
Formula II Dye
Dye 38
Dye 39
Dye 40
Dye 41
Dye 42
dye 43
Dye 44

Figure 7E

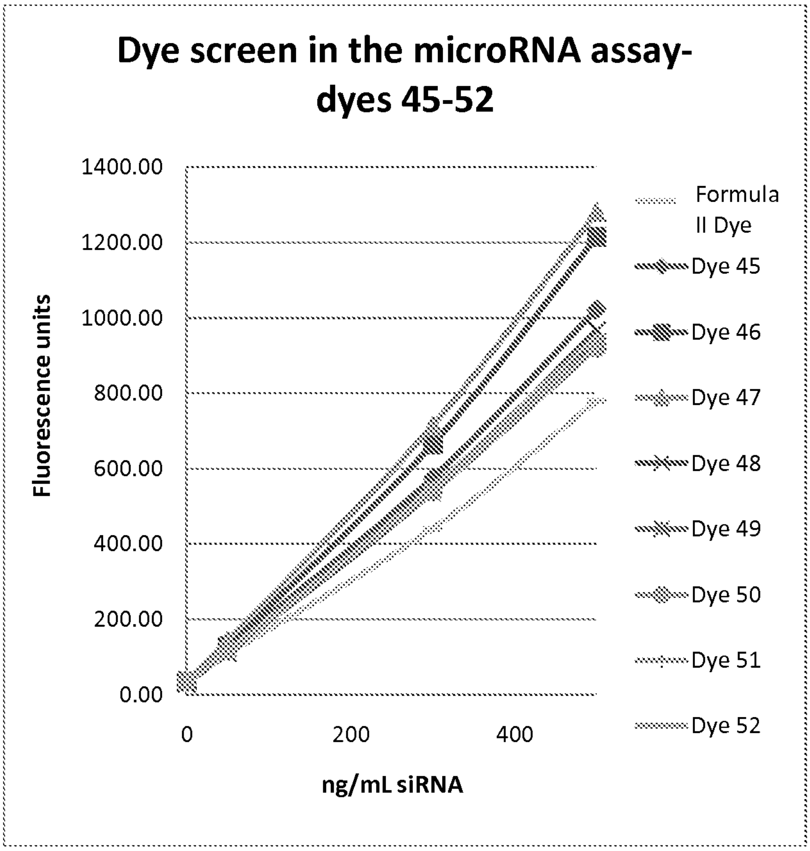
Dye screen in the microRNA assay-dyes 45-52
Fluorescence units
1400.00
1200.00
1000.00
800.00
600.00
400.00
200.00
0.00
0
200
400
ng/mL siRNA
Formula II Dye
Dye 45
Dye 46
Dye 47
Dye 48
Dye 49
Dye 50
Dye 51
Dye 52

**Figure 7F**

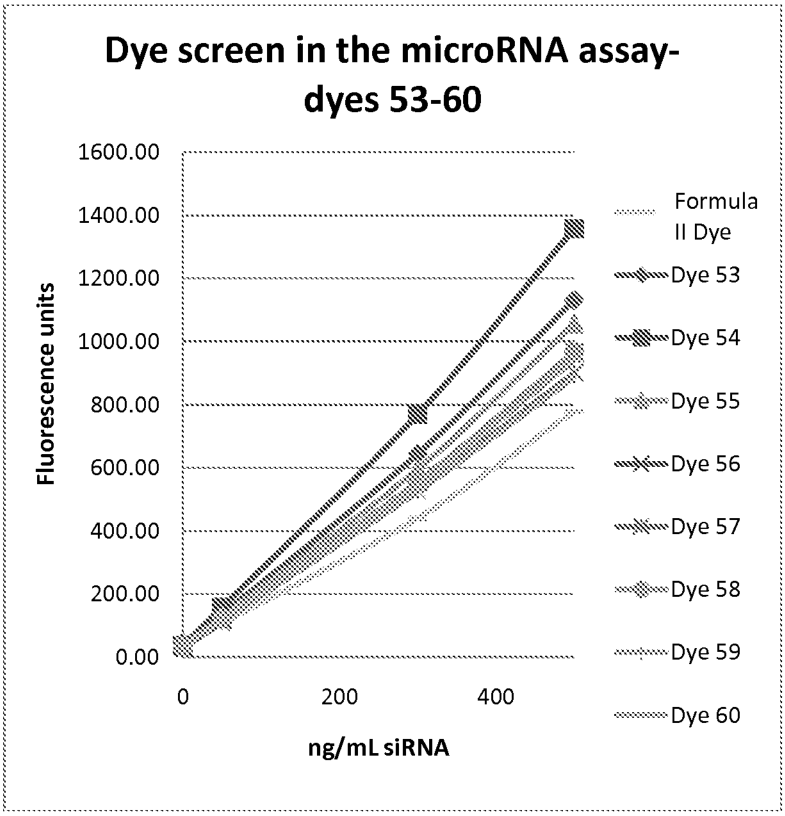
Dye screen in the microRNA assay-
dyes 53-60
Fluorescence units
1600.00
1400.00
1200.00
1000.00
800.00
600.00
400.00
200.00
0.00
0
200
400
ng/mL siRNA
Formula II Dye
Dye 53
Dye 54
Dye 55
Dye 56
Dye 57
Dye 58
Dye 59
Dye 60

**Figure 7G**

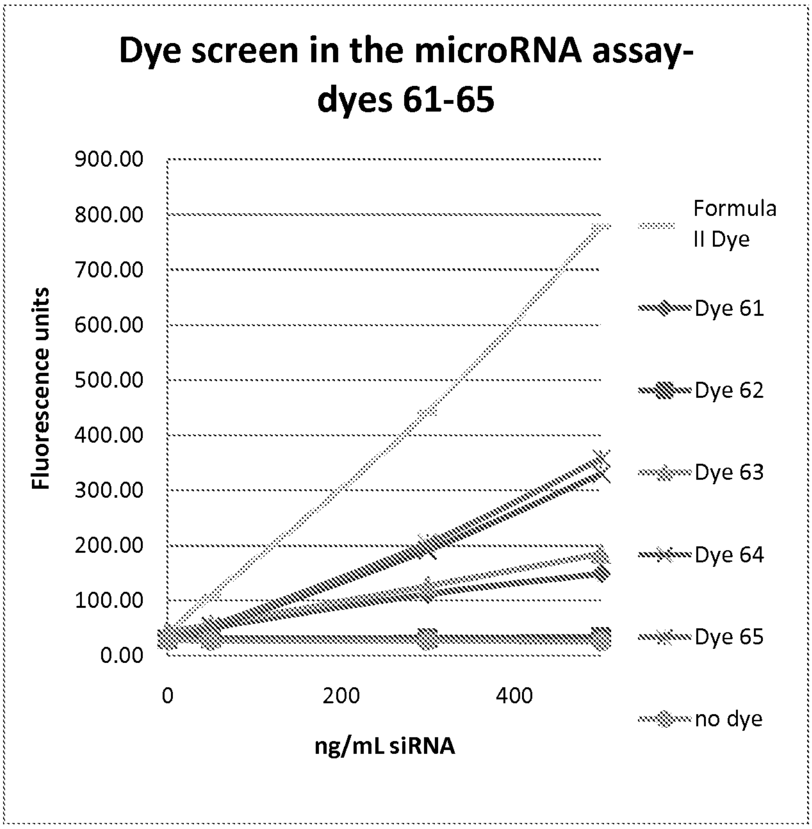
Dye screen in the microRNA assay-
dyes 61-65
900.00
800.00
700.00
600.00
500.00
400.00
300.00
200.00
100.00
0.00
Fluorescence units
0
200
400
ng/mL siRNA
Formula II Dye
Dye 61
Dye 62
Dye 63
Dye 64
Dye 65
no dye

Figure 7H

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0066664 A
- WO 9613552 A
- WO 2005033342 A
- WO 2005056689 A
- US 5658751 A


### Non-patent literature cited in the description


- VOGEL'S ENCYCLOPEDIA OF PRACTICAL ORGANIC CHEMISTRY. Longman Scientific and Technical Ltd, 1991, 809-816
- **HELLER.** *Acc. Chem. Res.,* 1990, vol. 23, 128